(19) Europäisches Patentamt
European Patent Office
Office européen des brevets

(11) Numéro de publication : **0 447 285 B1**

(12) **FASCICULE DE BREVET EUROPEEN**

(45) Date de publication du fascicule du brevet : **12.05.93 Bulletin 93/19**

(21) Numéro de dépôt : **91400526.9**

(22) Date de dépôt : **27.02.91**

(51) Int. Cl.$^5$ : **C07C 233/17,** C07C 233/73, C07C 233/60, C07C 233/22, C07C 237/08, C07D 207/12, C07D 295/15

# (54) Nouveaux dérivés à structure naphtalénique, leur procédé de préparation et les compositions pharmaceutiques qui les contiennent.

(30) Priorité : **27.02.90 FR 9002393**

(43) Date de publication de la demande : **18.09.91 Bulletin 91/38**

(45) Mention de la délivrance du brevet : **12.05.93 Bulletin 93/19**

(84) Etats contractants désignés : **AT BE CH DE DK ES FR GB GR IT LI LU NL SE**

(56) Documents cités :
**US-A- 3 689 557**
**US-A- 4 169 108**
**JOURNAL OF THE CHEMICAL SOCIETY, April 1965, pages 3007-3008; A.J. BIRCH et al.: "Steroid hormones. Part XIII 13-aza and 13-aza-D-homo-analogues of equilenin methyl ester"**
**JOURNAL OF HETEROCYCLIC CHEMISTRY, vol. 9, no. 4, août 1972, pages 805-811; K.-Y. ZEE-CHENG et al.: "Synthesis of 8,9-dialkoxy-substituted tetrahydrobenz[h]isoquinolines"**

(56) Documents cités :
**JOURNAL OF HETEROCYCLIC CHEMISTRY, vol. 14, no. 5, août 1977, pages 905-908; C.S. MENON et al.: "Synthesis of dimethoxy-benz[9]isoquinolines"**

(73) Titulaire : **ADIR ET COMPAGNIE**
**1 rue Carle Hébert**
**F-92415 Courbevoie Cédex (FR)**

(72) Inventeur : **Andrieux, Jean**
**29 avenue Gabriel Péri**
**F-92160 Antony (FR)**
Inventeur : **Houssin, Raymond**
**73 rue de l'Egalité**
**F-59700 Marcq en Baroeul (FR)**
Inventeur : **Yous, Said, Laboratoire de Chimie Synthèse**
**3 rue du Professeur Laquesse**
**F-59045 Lille Cédex (FR)**
Inventeur : **Guardiola, Béatrice**
**6 rue Edouard Nortier**
**F-92200 Neuilly sur Seine (FR)**
Inventeur : **Lesieur, Daniel**
**20 rue de Verdun**
**F-59147 Gondecourt (FR)**

EP 0 447 285 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés d'alkoxy-1-(2-acylamino éthyl) naphtalènes, leur procédé de préparation et les compositions pharmaceutiques qui les contiennent.

Un certain nombre de (2-amino éthyl) naphtalènes ont été décrits antérieurement. La demande de brevet JP 50089352 décrit des dérivés naphtaléniques à activité analgésique antiinflammatoire antipyrétique. La demande de brevet JP 61282348 décrit des (arylalkyl amino alkyl) naphtalènes en tant qu'agents FONGICIDES. Le brevet US 4327022 décrit des amino alkyl dialkoxy naphtalènes en tant que CARDIOTONIQUES. La demande de brevet EP 149588 décrit des (hydroxy amino alkyl) méthoxy naphtalènes comme inhibiteurs de la lipoxygénase et donc utiles dans le traitement de l'asthme, de l'inflammation et du psoriasis. La demande de brevet FR 70.44709 décrit des 1-phényl-2-[2-(napht-1-yl)éthylamino]éthanols en tant qu'anti-spasmodiques et vasodilatateurs.

La demanderesse a présentement découvert que les nouveaux dérivés des alkoxy 1-(2-acylamino éthyl) naphtalènes possédaient d'intéressantes propriétés pharmacologiques sur le système nerveux central, notamment anxiolytiques, antipsychotiques, analgésiques, sur l'ovulation, la circulation cérébrale l'immunomodulation et se démarquent nettement des dérivés amino alkyl naphtalènes déjà décrits.

Plus particulièrement, l'invention concerne les dérivés de formule générale (I) :

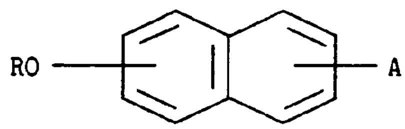

(I)

dans laquelle :

A représente un groupement

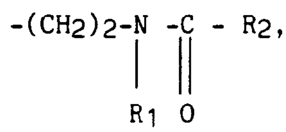

R représente un groupement alkyle inférieur linéaire ou ramifié,

$R_1$ représente un atome d'hydrogène ou un groupement alkyle inférieur, linéaire ou ramifié,

$R_2$ représente

. un atome d'hydrogène,

. un groupement alkyle inférieur linéaire ou ramifié ou cycloalkyle éventuellement substitué par un atome d'halogène,

. un groupement aryle ou hétéroaryle ou arylalkyle inférieur ou aryle substitué ou hétéroaryle substitué ou arylalkyle substitué, étant entendu que par groupement hétéroaryle on entend un groupement insaturé, mono ou bicyclique comprenant de 1 à 3 hétéroatomes choisi parmi azote, oxygène ou soufre chaque cycle comprenant 4 ou 5 sommets, et que par groupement aryle on entend phényle ou naphtyle,

. un groupement imidazolyle éventuellement réduit et/ou substitué par un groupement oxo,

. un groupement de formule :

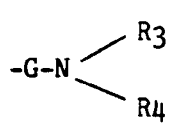

G représentant un groupement alkyle inférieur linéaire ou ramifié,

$R_3$ et $R_4$ identiques ou différents représentent chacun un groupement alkyle inférieur ou un atome d'hydrogène ou un groupement phényle ou phénylalkyle inférieur, ou $R_3$ et $R_4$ forment avec l'atome d'azote auquel ils sont attachés un système hétérocyclique, aromatique ou non, mono ou bicyclique chaque cycle ayant cinq ou six sommets comprenant éventuellement un autre hétéroatome et étant éventuellement substitué par un ou plusieurs groupements alkyle inférieur ou oxo, aryle ou arylalkyle inférieur, ou aryle substitué ou arylalkyle inférieur substitué,
étant entendu que le terme substitué affectant les groupements aryle et arylalkyle, et hétéroaryle dans la définition de $R_2$, $R_3$, $R_4$ signifie que ces groupements sont substitués par un ou plusieurs radicaux choisis parmi alkyle inférieur, alkoxy inférieur, trifluorométhyle ou atome d'halogène,

ou bien $R_1$ forme avec $R_2$ et le groupement N-CO un système hétérocyclique de formule :

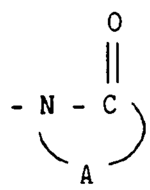

avec A étant un radical alkyle linéaire ou ramifié comprenant de 2 à 8 atomes de carbone,
leurs isomères, épimères, diastéréroisomères ainsi que, le cas échéant, leurs sels d'addition à un acide pharmaceutiquement acceptable, étant entendu que alkyle inférieur et alkoxy inférieur signifient des groupements comprenant de 1 à 6 atomes de carbone et que cycloalkyle signifie des groupements comprenant de 3 à 8 atomes de carbone.

Parmi les acides pharmaceutiquement acceptables que l'on peut, le cas échéant, ajouter aux composés de formule (I) pour obtenir un sel, on peut citer, à titre non limitatif, les acides chlorhydrique, sulfurique, tartrique, maléique, fumarique, oxalique, méthane-sulfonique, camphorique etc...

La présente invention a également pour objet le procédé de préparation des composés de formule (I) caractérisé en ce que l'on utilise comme matière première un dérivé de formule (II) :

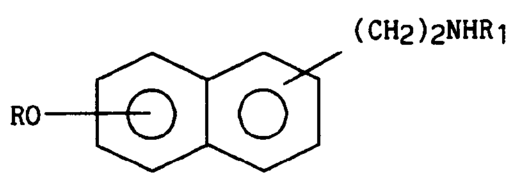

(II)

dans laquelle R et $R_1$ ont la même signification que dans la formule (I), que l'on traite :

- ou bien par un dérivé de formule (III) :

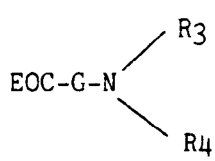

(III)

dans laquelle E signifie un groupe partant choisi parmi hydroxyle, alkoxy inférieur ou halogène, G, $R_3$ et $R_4$ ayant la même signification que dans la formule (I),
éventuellement en présence d'un agent alcalin,
pour conduire à un dérivé de formule (I/A), cas particulier des dérivés de formule (I) :

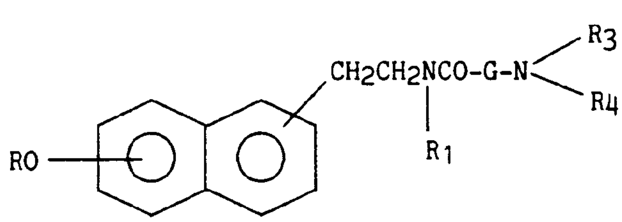

(I/A)

dans laquelle R, $R_1$, $R_3$, $R_4$ et G ont la même définition que précédemment, $R_2$ signifiant ici un groupement :

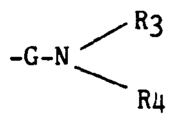

que l'on purifie, si on le désire, par des techniques classiques telles que chromatographie, cristallisation et que l'on salifie, si on le désire par un acide pharmaceutiquement acceptable,

- ou bien par un chlorure d'acide de formule (IV) :

$Cl\text{-}CO\text{-}R'_2$ **(IV)**

ou par l'anhydride d'acide correspondant,
$R'_2$ signifiant ici :

- un groupement alkyle inférieur linéaire ou ramifié ou cycloalkyle éventuellement substitué par un atome d'halogène,
- un groupement aryle ou hétéroaryle ou arylalkyle inférieur éventuellement substitué par un ou plusieurs atomes d'halogène ou groupements choisis parmi alkyle inférieur, alkoxy inférieur, ou trifluorométhyle,
- un groupement imidazolyle éventuellement réduit et/ou substitué par un groupement oxo,

pour conduire à un dérivé de formule (I/B) :

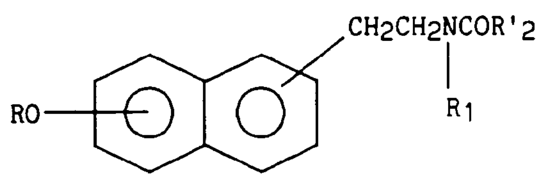

(I/B)

cas particulier des dérivés de formule (I) dans laquelle R, $R_1$ et $R'_2$ ont la même définition que précédemment, que l'on purifie si nécessaire par des techniques classiques telles que chromatographie et/ou cristallisation, dérivé de formule (I/B) qui dans le cas où $R'_2$ représente un groupement alkyle inférieur, linéaire ou ramifié, substitué par un atome d'halogène, peut-être soumis, si on le désire à l'action d'une amine de formule (V) :

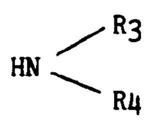

(V)

dans laquelle $R_3$ et $R_4$ ont la même définition que précédemment, en excès ou en présence d'une amine tertiaire ou d'un sel de métal alcalin pour conduire à un dérivé de formule (I/A) tel que précédemment défini, qui si on le désire est purifié par une technique classique telle que chromatographie et/ou cristallisation, et/ou salifié par un acide pharmaceutiquement acceptable,
dérivé de formule (I/B) qui lorsque $R'_2$ représente un substituant alkyle linéaire ou ramifié comprenant au moins deux atomes de carbone et substitué par un atome d'halogène, et lorsque, simultanément $R_1$ représente un atome d'hydrogène, peut être soumis si on le désire, à l'action d'une base forte et préférentiellement un alcoolate de métal alcalin pour conduire à un dérivé de formule (I/C) :

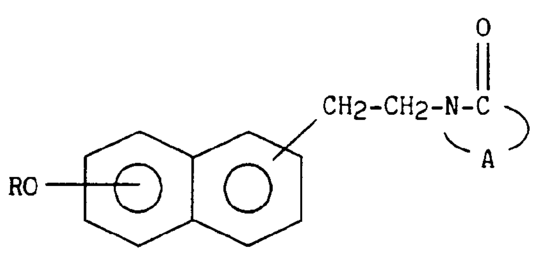

(I/C)

dans laquelle R a la même signification que précédemment et A représente un groupement alkyle linéaire ou

ramifié comprenant de 2 à 8 atomes de carbone, cas particulier des dérivés de formule (I) pour lesquels $R_1$ et $R_2$ forment avec NCO un système monocyclique substitué par un groupement oxo, et éventuellement substitué par un ou plusieurs groupements alkyles inférieurs,
que l'on purifie, si on le désire, par une technique choisie parmi cristallisation ou chromatographie.

Les composés de formule (I) possèdent des propriétés pharmacologiques intéressantes.

L'étude pharmacologique des dérivés de l'invention a en effet montré qu'ils étaient peu toxiques, doués d'une très haute affinité sélective pour les récepteurs de la mélatonine et d'importantes activités sur le système nerveux central et en particulier, on a relevé des propriétés sédatives, anxiolytiques, antipsychotiques, analgésiques ainsi que sur la microcirculation qui permettent d'établir que les produits de l'invention sont utiles dans le traitement du stress, des troubles du sommeil, de l'anxiété, les dépressions saisonnières, les insomnies et fatigues dues aux décalages horaires, schizophrénies, attaque de panique, mélancolie, la régulation de l'appétit, l'insomnie, les troubles psychotiques, l'épilepsie, la maladie de Parkinson, la démence sénile, les divers désordres liés au vieillissement normal ou pathologique, migraine, pertes de mémoire, maladie d'Alzheimer, ainsi que les troubles de la circulation cérébrale.

Dans un autre domaine d'activité, il apparaît que les produits de l'invention possèdent des propriétés d'inhibiteurs de l'ovulation, d'immunomodulateurs et qu'ils sont donc susceptibles d'être utilisés dans le traitement de certains cancers et qu'administrés par voie externe, ils sont utiles dans le traitement du psoriasis, de l'acné, de la seborrhée, protègent la peau et favorisent la pousse des cheveux. Ils peuvent également avoir un usage vétérinaire pour leur propriété sur le pelage.

La présente invention a également pour objet les compositions pharmaceutiques contenant les produits de formule (I) ou le cas échéant un de leurs sels d'addition à un acide pharmaceutiquement acceptable, seuls ou en combinaison avec un ou plusieurs excipients ou véhicules inertes, non toxiques, pharmaceutiquement acceptables.

Parmi les compositions pharmaceutiques selon l'invention, on pourra citer, plus particulièrement celles qui conviennent pour l'administration orale, parentérale, nasale, per/ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, les ampoules buvables, injectables, etc...

La posologie varie selon l'âge et le poids du patient, la voie d'administration, la nature de l'indication thérapeutique, ou des traitements éventuellement associés et s'échelonne entre 0,1 mg et 1 gramme par 24 heures.

Les exemples suivants illustrent l'invention, mais ne la limitent en aucune façon.

Les produits décrits dans les "préparations" ne font pas partie de l'invention. Leur description facilite cependant la réalisation des dérivés de l'invention.

**PREPARATION 1 : 2-(7-METHOXY NAPHT-1-YL)ETHYLAMINE**

**STADE A : (7-METHOXY 1,2,3,4-TETRAHYDRO-1-NAPHTYLIDENE) ACETATE D'ETHYLE**

Mélanger 50 g de 7-méthoxy 1-tétralone, 40 g de bromoacétate d'éthyle et 150 cm³ de benzène par l'intermédiaire d'une ampoule à brome. Ajouter le mélange sur du zinc en aiguilles activé (18,6 g) selon Reformatsky et un cristal d'iode.Chauffer à 60°C et ensuite porter à reflux pendant 45 minutes.
Hydrolyser sur de la glace en présence d'acide chlorhydrique. Extraire au benzène, sécher et porter à ébullition en présence de $P_2O_5$. Filtrer et porter à sec.
Le résidu est utilisé tel quel dans l'étape suivante.
Rendement : 80%

**STADE B : (7-METHOXY NAPHT-1-YL)ACETATE D'ETHYLE**

Mélanger 50 g de 7-méthoxy 1,2,3,4 tétrahydro-1-naphtylidène acétate d'éthyle à 7,35 g de soufre et chauffer à 215°C pendant 10 heures. Refroidir, ajouter 300 cm³ d'acétate d'éthyle, agiter pendant 30 minutes, filtrer. Porter à sec.
Le résidu obtenu est utilisé tel quel pour l'étape de saponification.
Rendement : 70%

**STADE C : ACIDE (7-METHOXY NAPHT-1-YL) ACETIQUE**

Chauffer à reflux pendant 3 heures un mélange du 7-méthoxy napht-1-yl acétate d'éthyle obtenu préce-

demmment dans 250 $cm^3$ de soude à 20 % dans l'éthanol.
Porter à sec et laver le résidu à l'éther. Précipiter par un courant d'acide chlorhydrique gazeux.
Point de fusion : 155-156 °C
Rendement : 68%

**STADE D : CHLORURE DE L'ACIDE (7-METHOXY NAPHT-1-YL) ACETIQUE**

Solubiliser à chaud l'acide 7-methoxy napht-1-yl acétique obtenu précedemment dans 300 ml de chloroforme. Chauffer à reflux puis ajouter goutte à goutte le chlorure de thionyle. Porter deux heures à reflux et évaporer à sec. On obtient une huile qui cristallise par refroidissement. Le résidu obtenu est utilisé tel quel dans le stade suivant.

**STADE E : (7-METHOXY NAPHT-1-YL) ACETAMIDE**

Le chlorure de l'acide (7-méthoxy napht-1-yl) acétique obtenu précédemment est dissous dans 200 ml d'éther anhydre. Après refroidissement de la solution à l'aide d'un bain de glace-sel, ajouter sous agitation 200 ml d'une solution aqueuse d'ammoniaque concentrée.
Agiter 30 minutes et essorer le précipité formé.
Recristalliser dans l'éthanol.
Rendement : 95%
Point de fusion : 201-202° C

**STADE F : (7-METHOXY NAPHT-1-YL) ACETONITRILE**

Mettre en suspension le (7-méthoxy napht-1-yl) acétamide obtenu au stade E dans 80 ml de THF anhydre. Ajouter la triéthylamine. Refroidir la solution dans un bain de glace puis ajouter goutte à goutte l'anhydride trifluoroacétique sous agitation magnétique.
Laisser agiter pendant une heure à température ambiante. Porter à sec. le résidu est repris par de l'eau. Essorer le précipité formé, sécher et recristalliser dans l'éther isopropylique.
Rendement : 83%
Point de fusion : 82-84° C
Caractéristiques spectrales :
Infrarouge : 2240 $cm^{-1}$ CN

**STADE G : 2-(7-METHOXY NAPHT-1-YL) ETHYLAMINE**

Placer dans un autoclave une solution de (7-méthoxy napht-1-yl) acétonitrile dans de l'éthanol saturé par de l'ammoniac. Ajouter du nickel de Raney et de l'hydrogène sous 300 atmosphères.
Agiter à 60°C pendant une nuit. Filtrer et évaporer le filtrat sous vide. L'huile ainsi obtenue est utilisée telle quelle comme matière première.

**PREPARATION 2 : N[2-(7-METHOXY -NAPHT-1-YL) ETHYL] N METHYL AMINE**

En procédant comme dans la préparation 1, mais en remplaçant au stade E l'ammoniac par la méthylamine et en réduisant directement l'amide ainsi obtenu, on obtient le produit du titre.

**PREPARATION 3 : 2-(6-METHOXY NAPHT-1-YL) ETHYLAMINE**

En procédant comme dans la préparation 1, mais en remplaçant au stade A la 7-méthoxy 1-tétralone par la 6-méthoxy 1-tétralone, on obtient le produit du titre.

**PREPARATION 4 : 2-(5-METHOXY NAPHT-1-YL) ETHYLAMINE**

En procédant comme dans la préparation 1, mais en remplaçant au stade A la 7-méthoxy 1-tétralone par la 5-méthoxy 1-tétralone, on obtient le produit du titre.

**PREPARATION 5 : 2-(7-METHOXY NAPHT-2-YL)ETHYLAMINE**

En procédant comme dans la préparation 1, mais en remplaçant au stade A la 7-méthoxy 1-tétralone par la 7-méthoxy 2-tétralone, on obtient le produit du titre.

**PREPARATION 6 : 2-(6-METHOXY NAPHT-2-YL)ETHYLAMINE**

En procédant comme dans la préparation 1, mais en remplaçant au stade A la 7-méthoxy 1-tétralone par la 6-méthoxy 2-tétralone, on obtient le produit du titre.

**EXEMPLE 1 : N[2-(7-METHOXY NAPHT-1-YL) ETHYL] ACETAMIDE**

Dissoudre 0,01 ml de 2-(7-méthoxy napht-1-yl) éthylamine dans 6 ml de pyridine. Refroidir dans un bain de glace puis sous agitation, ajouter goutte à goutte 0,012 mole de chlorure d'acétyle.
Maintenir l'agitation 30 minutes puis verser le milieu réactionnel sur la glace. Essorer le précipité formé, laver, sécher et recristalliser dans l'éther isopropylique.
Rendement : 92%
Point de fusion : 109-110°C
Caractéristiques spectrales :
Infrarouge : 3240 $cm^{-1}$ νNH
1640 $cm^{-1}$ νCO
Résonance Magnétique Nucléaire 1H Solvant $CDCl_3$:
δ : 1,93 ppm, singulet, 3H, $COCH_3$
δ : 3,96 ppm, singulet, 3H, $OCH_3$

**EXEMPLE 2 : N[2-(7-METHOXY NAPHT-1-YL) ETHYL] PHENYLACETAMIDE**

Dissoudre 0,01 mole de chlorhydrate de 2-(7-méthoxy napht-1-yl)éthylamine (obtenu par dissolution de la 2-(7-méthoxy napht-1-yl éthylamine dans l'éther et en faisant barboter un courant d'acide chlorhydrique gazeux puis en essorant le précipité formé) dans 60 ml d'un mélange eau-chloroforme. Ajouter sous agitation magnétique 0,01 mole de carbonate de potassium.
Refroidir et ajouter goutte à goutte 0,012 mole de chlorure de phénacétyle. Maintenir l'agitation 30 minutes à température ambiante et porter à sec la phase chloroformique. Recristalliser dans l'éther isopropylique.
Rendement : 92%
Point de fusion : 101-102°C
Caractéristiques spectrales :
Infrarouge : 3220 $cm^{-1}$ νNH
1640 $cm^{-1}$ νCO
Résonance Magnétique Nucléaire $^1H$ Solvant $CDCl_3$ :
δ : 3,50 ppm, massif, 2H, $CH_2$-N
δ : 3,93 ppm, singulet, 1H, $OCH_3$

**EXEMPLE 3 : N[2-(7-METHOXY NAPHT-1-YL) ETHYL] ISOBUTYRAMIDE**

En procédant comme dans l'exemple 1, mais en remplaçant le chlorure d'acétyle par le chlorure d'isobutyryle, on obtient le produit du titre.
Rendement : 91%
Point de fusion : 77-78°C
Caractéristiques spectrales :
Infrarouge : 3240 $cm^{-1}$ νNH
1640 $cm^{-1}$ νCO
1620 $cm^{-1}$ νCC
Résonance Magnétique Nucléaire $^1H$ Solvant $CDCl_3$ :
δ : 1,11 ppm, doublet, 6H, $2CH_3$ (isopropyl)
δ : 2,29 ppm, multiplet, 1H, CH (COCH)
δ : 3,98 ppm, singulet, 3H, $OCH_3$

**EXEMPLE 4 : N[2-(7-METHOXY NAPHT-1-YL) ETHYL] PROPIONAMIDE**

En procédant comme dans l'exemple 1, mais en remplaçant le chlorure d'acétyle par le chlorure de propionyle, on obtient le produit du titre.
Point de fusion : 104-104,5°C

**EXEMPLE 5 : N[2-(7-METHOXY NAPHT-1-YL) ETHYL] PENTANAMIDE**

En procédant comme dans l'exemple 1, mais en remplaçant le chlorure d'acétyle par le chlorure de pentanoyle, on obtient le produit du titre.
Solvant de cristallisation : cyclohexane
Point de fusion : 90°C

**EXEMPLE 6 : N[2-(7-METHOXY NAPHT-1-YL) ETHYL] (2-OXO PYRROLIDIN-1-YL) ACETAMIDE**

**OU**

**1-{2[(2-OXOPYRROLIDIN-1-YL) ACETAMIDO] ETHYL}-7-METHOXY NAPHTALENE**

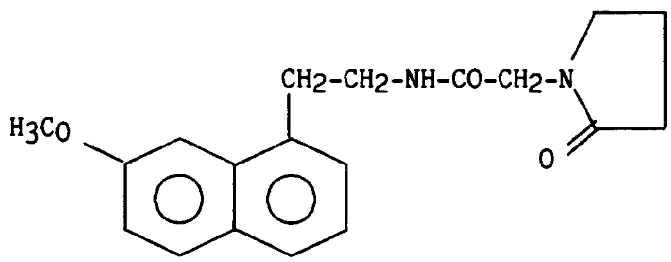

Chauffer sous agitation magnétique à la température de 80°C pendant 3 heures un mélange de 0,02 mole de 2-(7-méthoxy napht-1-yl) éthylamine et de 0,022 mole d'(2-oxo pyrrolidin-1-yl)acétate de méthyle.
Reprendre le milieu réactionnel par de l'eau légèrement acide, essorer le précipité formé.
Recristalliser dans l'éther di-n butylique.
Rendement : 55%
Point de fusion : 125-126°C
Caractéristiques spectrales :

| | |
|---|---|
| Infrarouge : | 3310 $cm^{-1}$ νNH |
| | 3060-2820 $cm^{-1}$ νCH($CH_2CH_2$) |
| | 1690 $cm^{-1}$ νCO (CON) |
| | 1630-1600 cm-1 νCC (aromatiques) |
| | 1030 cm-1 ν$OCH_3$ |

Résonance Magnétique Nucléaire $^1H$ Solvant $CDCl_3$ :
δ : 3,88 ppm, singulet, 2H, $NHCOCH_2$
δ : 4,00 ppm, singulet, 3H, $OCH_3$

**EXEMPLE 7 : N[2-(7-METHOXY NAPHT-1-YL) ETHYL]-4-CHLORO BUTYRAMIDE.**

Dissoudre 0,02 mole de chlorhydrate de 2-(7-méthoxy napht-1-yl) éthylamine dans un mélange eau-chloroforme. Ajouter du carbonate de potassium et laisser agiter pendant 15 minutes dans un bain de glace. Ajouter ensuite goutte à goutte 0,022 mole de chlorure de 4-chloro butyryle.
Maintenir l'agitation à température ambiante pendant 1/2 heure.
Porter à sec la phase chloroformique.
Recristalliser le résidu dans un mélange toluène, cyclohexane (1-1).
Rendement : 93%
Point de fusion : 97-98°C
Caractéristiques spectrales :
Infrarouge : 3320 $cm^{-1}$ νNH

1635 $cm^{-1}$ νCO

**EXEMPLE 8 : N[2-(7-METHOXY NAPHT-1-YL) ETHYL] PYRROLIDIN-2-ONE**

Dissoudre 0,01 mole de sodium dans 50 $cm^3$ d'éthanol. Ajouter sous agitation magnétique le N[2-(7-méthoxy napht-1-yl) éthyl] chloro-4 butyramide obtenu dans l'exemple 7.
Maintenir l'agitation 20 minutes.
Porter à sec. Solubiliser dans 40 $cm^3$ de diméthyl formamide anhydre.
Chauffer à ébullition pendant 7 heures.
Evaporer sous vide et reprendre par l'éther.
Filtrer et porter à sec. Recristalliser dans l'éther de pétrole.
Rendement : 35%
Point de fusion : 60-61°C
Caractéristiques spectrales :
Infrarouge : 3060-2820 $cm^{-1}$ νCH
1670 $cm^{-1}$ νCO

**EXEMPLE 9 : N[2-(7-METHOXY NAPHT-1-YL) ETHYL] 2- BROMO ACETAMIDE**

Même mode opératoire que l'exemple 7 en remplaçant le chlorure de 4-chlorobutyryl par le chlorure de l'acide bromoacétique
Point de fusion : 100-101°C
Rendement : 93 %
Caractéristiques spectrales :
Infrarouge : 3260 $cm^{-1}$ νNH
1635 $cm^{-1}$ νCO
Résonance Magnétique Nucléaire $^1H$ Solvant $CDCl_3$ :
δ : 2,83 ppm, singulet, 2H, ($CH_2Br$)
δ : 3,98 ppm, singulet, 3H, ($OCH_3$)

**EXEMPLE 10 : N[2-(7-METHOXY NAPHT-1-YL) ETHYL] 2-MORPHOLINO ACETAMIDE**

Dissoudre sous agitation magnétique 0,01 mole de morpholine dans 50 $cm^3$ d'acétone. Ajouter 0,012 mole de triéthylamine et 0,01 mole de N-[2-(7-méthoxy napht-1-yl) éthyl]-2-bromo acétamide. Porter à reflux 1 heure sous agitation magnétique. Essorer le précipité formé et évaporer le filtrat.
Reprendre le résidu par de l'eau alcaline Essorer le précipité, laver, sécher et recristalliser dans un mélange Toluène-cyclohexane.
Caractéristiques spectrales :
Infrarouge : 1645 $cm^{-1}$ νCO
Résonance Magnétique Nucléaire $^1H$ Solvant $CDCl_3$ :
δ : 3,98 ppm, singulet, 3H, $OCH_3$
δ : 2,92 ppm, singulet, 2H, ($CO-CH_2$)
Point de fusion : 114-115°C
Rendement : 93 %

**EXEMPLE 11 : N[2-(7-METHOXY NAPHT-1-YL) ETHYL]-2-{4-[(2,3,4-TRIMETHOXYPHENYL)METHYL] PIPERAZIN-1-YL}ACETAMIDE, CHLORHYDRATE**

En procédant comme dans l'exemple précédent, mais en remplaçant la morpholine par la 1-[(2,3,4-triméthoxyphényl)méthyl]pipérazine, on obtient le produit du titre. Le chlorhydrate est obtenu par dissolution du produit dans l'acétone, barbotage d'un courant d'acide chlorhydrique, évaporation et recristallisation dans l'alcool absolu.
Caractéristiques spectrales :
Infrarouge : 1670 $cm^{-1}$ νCO
Résonance Magnétique Nucléaire $^1H$ Solvant $CDCl_3$ :
δ : 3,99 ppm, singulet, 3H, $OCH_3$
Point de fusion : 207-208°C
Rendement : 90 %

**EXEMPLE 12 : N[2-(7-METHOXY NAPHT-1-YL) ETHYL]N METHYLACETAMIDE**

En remplaçant dans l'exemple 1 la 2-(7-méthoxy napht-1-yl)éthylamine par la N-[2(7-méthoxy napht-1-yl)éthyl]N méthylamine, on obtient le produit du titre.
Caractéristiques spectrales :
Infrarouge : 1640 $cm^{-1}$ νCO
Résonance Magnétique Nucléaire $^1H$ Solvant $CDCl_3$ :
δ : 3,98 ppm, singulet, 3H, $OCH_3$

**EXEMPLE 13 : N[2-(7-METHOXY NAPHT-1-YL) ETHYL]BENZAMIDE**

En remplaçant dans l'exemple 2 le chlorure de phénacétyle par le chlorure de benzoyle, on obtient le produit du titre.
Caractéristiques spectrales :
Infrarouge : 1640 $cm^{-1}$ νCO
Résonance Magnétique Nucléaire $^1H$ Solvant $CDCl_3$ :
δ : 3,98 ppm, singulet, 3H, $OCH_3$
Point de fusion : 128-130°C
Rendement : 94 %

**EXEMPLE 14 : N[2-(7-METHOXY NAPHT-1-YL) ETHYL] PARATOLUOYL CARBOXAMIDE**

En remplaçant dans l'exemple 2 le chlorure de phénylacétyle par le chlorure de paratoluoyle, on obtient le produit du titre.
Caractéristiques spectrales :
Infrarouge : 1635 $cm^{-1}$ νCO
Résonance Magnétique Nucléaire $^1H$ Solvant $CDCl_3$ :
δ : 3,95 ppm, singulet, 3H, $OCH_3$

**EXEMPLE 15 : N[2-(7-METHOXY NAPHT-1-YL) ETHYL]4-FLUORO BENZAMIDE**

En remplaçant dans l'exemple 2 le chlorure de phénylacétyle par le chlorure de parafluorobenzoyle, on obtient le produit du titre.
Caractéristiques spectrales :
Infrarouge : 1635 $cm^{-1}$ νCO
Résonance Magnétique Nucléaire $^1H$ Solvant $CDCl_3$ :
δ : 3,95 ppm, singulet, 3H, $OCH_3$

**EXEMPLE 16 : N[2-(7-METHOXY NAPHT-1-YL) ETHYL]-3-TRIFLUOROMETHYL BENZAMIDE**

En remplaçant dans l'exemple 2 le chlorure de phénylacétyle par le chlorure de 3-trifluorométhyl benzoyle, on obtient le produit du titre.
Caractéristiques spectrales :
Infrarouge : 1635 $cm^{-1}$ νCO
Résonance Magnétique Nucléaire $^1H$ Solvant $CDCl_3$ :
δ : 3,95 ppm, singulet, 3H, $OCH_3$

**EXEMPLE 17 : N[2-(7-METHOXY NAPHT-1-YL) ETHYL]-3,5 DICHLORO BENZAMIDE**

En remplaçant dans l'exemple 2 le chlorure de phénacétyle par le chlorure de 3,5-dichloro benzoyle, on obtient le produit du titre.
Rendement : 93 %
Point de fusion : 138°C

**EXEMPLE 18 : N[2-(7-METHOXY NAPHT-1-YL) ETHYL]ISONICOTINAMIDE**

En remplaçant dans l'exemple 2 le chlorure de phénacétyle par le chlorure d'isonicotinoyle, on obtient le produit du titre.

Caractéristiques spectrales :
Infrarouge : 1635 $cm^{-1}$ $\nu CO$
Résonance Magnétique Nucléaire $^1H$ Solvant $CDCl_3$ :
$\delta$ : 3,95 ppm, singulet, 3H, $OCH_3$

**EXEMPLE 19 : N[2-(7-METHOXY NAPHT-1-YL) ETHYL]2-THIOPHENECARBOXAMIDE**

En remplaçant dans l'exemple 2 le chlorure de phénylacétyle par le chlorure de 2-thiophène carbonyle, on obtient le produit du titre.
Caractéristiques spectrales :
Infrarouge : 1635 $cm^{-1}$ $\nu CO$
Résonance Magnétique Nucléaire $^1H$ Solvant $CDCl_3$ :
$\delta$ : 3,95 ppm, singulet, 3H, $OCH_3$

**EXEMPLE 20 : N[2-(7-METHOXY NAPHT-1-YL) ETHYL]-2-QUINOXALINE CARBOXAMIDE**

En remplaçant dans l'exemple 2 le chlorure de phénylacétyle par le chlorure de 2-quinoxaloyle, on obtient le produit du titre.
Caractéristiques spectrales :
Infrarouge : 1635 $cm^{-1}$ $\nu CO$
Résonance Magnétique Nucléaire $^1H$ Solvant $CDCl_3$ :
$\delta$ : 3,95 ppm, singulet, 3H, $OCH_3$

**EXEMPLE 21 : N[2-(7-METHOXY NAPHT-1-YL) ETHYL]INDOL-2-YL CARBOXAMIDE**

En remplaçant dans l'exemple 2 le chlorure de phénacétyle par le chlorure de 2-indolyle, on obtient le produit du titre.
Point de fusion : 198-199°C
Caractéristiques spectrales :
Infrarouge : 3400 $cm^{-1}$ $\nu NH$ (indole)
3300 $cm^{-1}$ $\nu NH$
1640 $cm^{-1}$ $\nu CO$

Résonance Magnétique Nucléaire $^1H$ Solvant $CDCl_3$ :
$\delta$ : 3,98 ppm, singulet, 3H, $OCH_3$

**EXEMPLE 22 : N[2-(7-METHOXY NAPHT-1-YL) ETHYL]2-BENZYLAMINO ACETAMIDE**

En procédant comme dans l'exemple 10 et en remplaçant la morpholine par la benzylamine, on obtient le produit du titre.
Caractéristiques spectrales :
Infrarouge : 1635 $cm^{-1}$ $\nu CO$
Résonance Magnétique Nucléaire $^1H$ Solvant $CDCl_3$ :
$\delta$ : 3,95 ppm, singulet, 3H, $OCH_3$

**EXEMPLE 23 : N[2-(7-METHOXY NAPHT-1-YL) ETHYL]2-(N', N'-DIETHYL) AMINOACETAMIDE**

En procédant comme dans l'exemple 10, mais en remplaçant la morpholine par la N,N diéthylamine, on obtient le produit du titre.
Caractéristiques spectrales :
Infrarouge : 1635 $cm^{-1}$ $\nu CO$
Résonance Magnétique Nucléaire $^1H$ Solvant $CDCl_3$ :
$\delta$ : 3,95 ppm, singulet, 3H, $OCH_3$

**EXEMPLE 24 : N[2-(7-METHOXY NAPHT-1-YL) ETHYL]2-AMINO ACETAMIDE, CHLORHYDRATE**

Dissoudre sous agitation magnétique 0,012 mole d'hexaméthylène tétramine dans 15 $cm^3$ de chloroforme et introduire 0,01 mole de N-[2-(7-méthoxy napht-1-yl) éthyl]-2-bromo- acétamide obtenu dans l'exemple 9 dis-

sous dans 20cm$^3$ de chloroforme. Porter à reflux pendant 100 heures. Essorer, sécher. Introduire le précipité dans une fiole à col rodé. Ajouter 150 cm$^3$ d'alcool et 30 cm$^3$ d'acide chlorhydrique concentré.
Porter deux heures à reflux. Evaporer le solvant. Recristalliser dans l'alcool à 90°C.
Caractéristiques spectrales :
Infrarouge : 1635 $cm^{-1}$ νCO
Résonance Magnétique Nucléaire $^1H$ Solvant $CDCl_3$ :
δ : 3,95 ppm, singulet, 3H, $OCH_3$

**EXEMPLE 25 : N[2-(7-METHOXY NAPHT-1-YL) ETHYL]2-[4-(4-FLUOROPHENYL) PIPERAZIN-1-YL]ACETAMIDE**

En procédant comme dans l'exemple 10, mais en remplaçant la morpholine par la 1-(4-fluoro phényl)pipérazine, on obtient le produit du titre.

**EXEMPLE 26 : N[2-(7-METHOXY-1-NAPHTYL)ETHYL]2-[4-(3-TRIFLUOROMETHYLPHENYL) PIPERAZIN-1-YL]ACETAMIDE**

En procédant comme dans l'exemple 10, mais en remplaçant la morpholine par la 1-(3-trifluorométhyl)pipérazine, on obtient le produit du titre.

**EXEMPLE 27 : N[2-(7-METHOXYNAPHT-1-YL)ETHYL] BUTYRAMIDE**

En procédant comme dans l'exemple 1 mais en remplaçant le chlorure d'acétyle par le chlorure de butyryle , on obtient le produit du titre
Point de fusion : 99° C

**EXEMPLE 28 : N[2-(7-METHOXYNAPHT-1-YL)ETHYL] 4-IMIDAZOLYL ACETAMIDE**

Dissoudre 0,01 mole de chlorhydrate de 2-(7-méthoxy napht-1-yl) éthylamine dans 60 ml d'un mélange eau chloroforme. Ajouter sous agitation magnétique 0,025 mole de carbonate de potassium. Refroidir et ajouter goutte à goutte 0,012 mole de chlorhydrate de chlorure d'acide 4-imidazole acétique. Maintenir l'agitation 30 minutes à température ambiante et évaporer à sec la phase chloroformique. Recristalliser.
Rendement : 67 %
Caractéristiques spectrales :
Infrarouge : 1640 $cm^{-1}$ ν CO
Résonance magnétique nucléaire $^1H$ solvant $CDCl_3$
δ = 3,91 ppm singulet, 1H, $OCH_3$

**EXEMPLE 29 : N[2-(7-METHOXYNAPHT-1-YL)ETHYL] 2-IMIDAZOLINONE-4-CARBOXAMIDE**

En procédant comme dans l'exemple 1 mais en remplaçant le chlorure d'acétyle par le chlorure d'acide 2-imidazolinone-4-carboxylique, on obtient le produit du titre
Rendement : 55 %
Caractéristiques spectrales :
Résonance magnétique nucléaire $^1H$ solvant $CDCl_3$
δ = 3,89 ppm singulet 1H $OCH_3$

**EXEMPLE 30 : N[2-(7-METHOXYNAPHT-1-YL)ETHYL] CYCLOHEXANE CARBOXAMIDE ($R_2$ = cyclohexyle)**

En procédant comme dans l'exemple 1, mais en remplaçant le chlorure d'acétyle par le chlorure d'acide cyclohexane carboxylique, on obtient le produit du titre.
Solvant de recristallisation : cyclohexane
Point de fusion : 105-106°C

**EXEMPLE 31 : N[2-(7-METHOXYNAPHT-1-YL)ETHYL] CYCLOPROPANE CARBOXAMIDE ($R_2$ = cyclopropyle)**

En procédant comme dans l'exemple 1, mais en remplaçant le chlorure d'acétyle par le chlorure d'acide cyclopropyle carboxylique, on obtient le produit du titre.
Solvant de cristallisation : cyclohexane
Point de fusion : 91-92°C

**EXEMPLE 32 : N[2-(7-METHOXY NAPHT-1-YL) ETHYL] IODOACETAMIDE**

En traitant la N[2-(7-méthoxy napht-1-yl) éthyl]2-bromoacétamide obtenu dans l'exemple 9 par l'iodure de potassium, on obtient le produit du titre.
Solvant de recristallisation : éthanol
Point de fusion : 110-112°C

**EXEMPLE 33 : N[2-(7-METHOXY NAPHT-1-YL) ETHYL] FORMAMIDE**

Dans un creuset en porcelaine, placer 0,01 mole de 2-(7-méthoxy napht-1 yl)éthylamine et 0,02 mole d'acide formique. Chauffer à 120° jusqu'à obtention d'un résidu sec. Recristalliser.
Point de fusion : 93°C
Caractéristiques Spectrales :
Résonance Magnétique Nucléaire :
δ : 4,05 ppm, singulet, 3H, $OCH_3$

**EXEMPLE 34 : N[2-(7-METHOXY NAPHT-1-YL) ETHYL] CYCLOBUTANE CARBOXAMIDE**

En procédant comme dans l'exemple 1, mais en remplaçant le chlorure d'acétyle par le chlorure d'acide cyclobutane carboxylique, on obtient le produit du titre.

**EXEMPLE 35 : N[2-(7-METHOXY NAPHT-1-YL) ETHYL] CYCLOPENTANE CARBOXAMIDE**

En procédant comme dans l'exemple 1, mais en remplaçant le chlorure d'acétyle par le chlorure de l'acide cyclopentane carboxylique, on obtient le produit du titre.

En procédant comme dans les exemples précédents, on obtient de même :
**N[2-(7-METHOXY-1-NAPHTYL) ETHYL] 4-BROMO BUTYRAMIDE**
**N[2-(7-METHOXY-1-NAPHTYL) ETHYL] 5-BROMO PENTANAMIDE**
**N[2-(7-METHOXY-1-NAPHTYL) ETHYL] 3-BROMO PROPIONAMIDE**
**N[2-(7-METHOXY-1-NAPHTYL) ETHYL]-3-MORPHOLINO PROPIONAMIDE**
**N[2-(7-METHOXY-1-NAPHTYL) ETHYL]-4-MORPHOLINO BUTYRAMIDE**
**N[2-(7-METHOXY-1-NAPHTYL) ETHYL]-4-{4-[(2,3,4-TRIMETHOXY PHENYL)METHYL]-1-PIPERAZINYL} BUTYRAMIDE**
**N[2-(7-METHOXY-1-NAPHTYL) ETHYL]-3-{4-[2,3,4-TRIMETHOXY PHENYL)METHYL]-1-PIPERAZINYL} PROPIONAMIDE**
**N[2-(7-METHOXY-1-NAPHTYL) ETHYL]PIPERIDINE-2-ONE**
**N[2-(7-METHOXY-1-NAPHTYL) ETHYL]BROMO-2 PROPIONAMIDE**
**N[2-(7-METHOXY-1-NAPHTYL) ETHYL]-2-{4-[(2,3,4-TRIMETHOXY PHENYL)METHYL]-1-PIPERAZINYL} PROPIONAMIDE**

En remplaçant dans les exemples précédents la 2-(7-méthoxy napht-1-yl) éthylamine par la 2-(6-méthoxy napht-1-yl)éthylamine ou par la 2-(5-méthoxy napth-1-yl) éthylamine, on obtient les produits des exemples précédents méthoxylés respectivement en position 6 ou 5 du naphtalène au lieu des dérivés méthoxylés en position 7.

En remplaçant dans les exemples précédents la 2-(7-méthoxy napht-1-yl)éthylamine par la 2-(7-méthoxy napht-2-yl)éthylamine, on obtient :
**N[2-(7-METHOXY NAPHT-2-YL) ETHYL] ACETAMIDE**
**N[2-(7-METHOXY NAPHT-2-YL) ETHYL] PHENYLACETAMIDE**
**N[2-(7-METHOXY NAPHT-2-YL) ETHYL] ISOBUTYRAMIDE**
**N[2-(7-METHOXY NAPHT-2-YL) ETHYL] PROPIONAMIDE**
**N[2-(7-METHOXY NAPHT-2-YL) ETHYL] PENTANAMIDE**

**N[2-(7-METHOXY NAPHT-2-YL) ETHYL] (2-OXO PYRROLIDIN-1-YL)ACETAMIDE**
**N[2-(7-METHOXY NAPHT-2-YL) ETHYL] 4-CHLORO BUTYRAMIDE**
**N[2-(7-METHOXY NAPHT-2-YL) ETHYL] PYRROLIDIN-2-ONE**
**N[2-(7-METHOXY NAPHT-2-YL) ETHYL] 2-BROMOACETAMIDE**
**N[2-(7-METHOXY NAPHT-2-YL) ETHYL] 2-MORPHOLINOACETAMIDE**
**N[2-(7-METHOXY NAPHT-2-YL) ETHYL] 2-{4-[2,3,4-TRIMETHOXY PHENYL) METHYL] PIPERAZIN-1-YL}ACETAMIDE, CHLORHYDRATE**
**N[2-(7-METHOXY NAPHT-2-YL) ETHYL] N METHYL ACETAMIDE**
**N[2-(7-METHOXY NAPHT-2-YL) ETHYL] BENZAMIDE**
**N[2-(7-METHOXY NAPHT-2-YL) ETHYL] PARATOLUOYLCARBOXAMIDE**
**N[2-(7-METHOXY NAPHT-2-YL) ETHYL] 4-FLUOROBENZAMIDE**
**N[2-(7-METHOXY NAPHT-2-YL) ETHYL] 3-TRIFLUOROMETHYLBENZAMIDE**
**N[2-(7-METHOXY NAPHT-2-YL) ETHYL] 3,5-DICHLOROBENZAMIDE**
**N[2-(7-METHOXY NAPHT-2-YL) ETHYL] ISONICOTINAMIDE**
**N[2-(7-METHOXY NAPHT-2-YL) ETHYL] 2-THIOPHENECARBOXAMIDE**
**N[2-(7-METHOXY NAPHT-2-YL) ETHYL] 2-QUINOXALINECARBOXAMIDE**
**N[2-(7-METHOXY NAPHT-2-YL) ETHYL] INDOL-2-YL CARBOXAMIDE**
**N[2-(7-METHOXY NAPHT-2-YL) ETHYL] 2-BENZYLAMINOACETAMIDE**
**N[2-(7-METHOXY NAPHT-2-YL) ETHYL] 2-(N',N' DIETHYL) AMINOACETAMIDE**
**N[2-(7-METHOXY NAPHT-2-YL) ETHYL] 2-AMINOACETAMIDE**
**N[2-(7-METHOXY NAPHT-2-YL) ETHYL] 2-[4-(4-FLUOROPHENYL)PIPERAZIN-1-YL] ACETAMIDE**
**N[2-(7-METHOXY NAPHT-2-YL) ETHYL]2-[4-(3-TRIFLUOROMETHYLPHENYL)PIPERAZIN-1-YL] ACETAMIDE**
**N[2-(7-METHOXY NAPHT-2-YL) ETHYL] BUTYRAMIDE**
**N[2-(7-METHOXY NAPHT-2-YL) ETHYL] 4-IMIDAZOLYLACETAMIDE**
**N[2-(7-METHOXY NAPHT-2-YL) ETHYL] 2-IMIDAZOLINONE 4-CARBOXAMIDE**
**N[2-(7-METHOXY NAPHT-2-YL) ETHYL] CYCLOHEXANE CARBOXAMIDE**
**N[2-(7-METHOXY NAPHT-2-YL) ETHYL] CYCLOPROPANECARBOXAMIDE**
**N[2-(7-METHOXY NAPHT-2-YL) ETHYL] IODOACETAMIDE**
**N[2-(7-METHOXY NAPHT-2-YL) ETHYL] FORMAMIDE**
**N[2-(7-METHOXY NAPHT-2-YL) ETHYL] CYCLOBUTANE CARBOXAMIDE**
**N[2-(7-METHOXY NAPHT-2-YL) ETHYL] CYCLOPENTANE CARBOXAMIDE**

Si dans ces synthèses on remplace la 2-(7-méthoxy napht-2-yl)éthylamine par la 2-(6-méthoxy napht-2-yl)éthylamine, on obtient les isomères des produits précédents pour lesquels le groupement méthoxy est en position 6.

## ETUDE PHARMACOLOGIQUE DES DERIVES DE L'INVENTION

### EXEMPLE A : ETUDE DE LA TOXICITE AIGUE

La toxicité aiguë a été appréciée après administration orale à des lots de 8 souris (26 ±2 grammes). Les animaux ont été observés à intervalles réguliers au cours de la première journée et quotidiennement pendant les deux semaines suivant le traitement. La DL50 entraînant la mort de 50% des animaux, a été évaluée.
La DL50 des produits testés est supérieure à 1000 $mg.kg^{-1}$ pour la plupart des composés étudiés ce qui indique la faible toxicité des composés de l'invention.

### EXEMPLE B : ACTIMETRIE

Les animaux sont placés dans des boites en plexiglass équipées avec des cellules photoélectriques placées dans une atmosphère assombrie. Six animaux sont testés simultanément et le nombre des interruptions des faisceaux photo électriques par chaque animal est enregistré informatiquement pendant une heure.
Les composés à tester sont administrés par voie intrapéritonéale immédiatement avant de placer les animaux dans l'appareil.
Les produits de l'invention diminuent l'activité des animaux.

### EXEMPLE C : TEST DES QUATRE PLAQUES

Les produits de l'invention sont administrés par voie oesophagienne à des lots de dix souris. Un lot reçoit

du sirop de gomme.
30 minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre des passages est enregistré pendant une minute. Après administration, les composés de l'invention augmentent de façon significative le nombre de passages ce qui montre l'activité anxiolytique ces dérivés de l'invention.

**EXEMPLE D : ACTIVITE DES PRODUITS DE L'INVENTION SUR LA MICROCIRCULATION ISCHEMIQUE**

L'étude expérimentale a été réalisée sur les muscles crémasters de rats mâles (Sprague-Dawley) après ligature de l'artère iliaque commune.

Les muscles ont été placés dans une chambre transparente, perfusés par une solution de tampon bicarbonate équilibrée par un mélange gazeux CO2/N2 5/95%. La vélocité des globules rouges et le diamètre des artérioles de premier ou second ordre irriguant le crémaster ont été mesurés, le flux sanguin artériolaire a été calculé. Des informations identiques ont été obtenues pour quatre types de veinules.

On a effectué le même type de mesure simultanément :
- sur le crémaster perfusé normalement,
- sur le crémaster sous ligature, c'est-à-dire le crémaster ischémié 2, 7, 14 et 21 jours après ligature.

Deux groupes d'animaux ont été étudiés :
- un groupe témoin sans traitement,
- un groupe traité per os par un produit de l'invention, à raison de 0,1 mg.kg-1 par jour.

On n'a constaté aucune différence dans la vélocité des globules ni dans le diamètre des vaisseaux dans les muscles crémasters normalement irrigués chez les animaux traités par rapport aux témoins.

Par contre, au niveau du muscle crémaster ischémié, le diamètre moyen des artérioles était amélioré chez les animaux traités par rapport aux témoins. La vélocité des globules rouges était normalisée par un traitement de 21 jours.

En fait, chez les animaux traités, la vélocité des globules rouges et le débit sanguin mesurés 7 jours après la ligature, ne présentent pas de différence significative avec les valeurs obtenues dans le crémaster non ischémié. Ces résultats sont obtenus sans modification de la pression artérielle.

Ces résultats indiquent que le traitement chronique par un composé de l'invention améliore la microcirculation et l'irrigation sanguine des territoires ischémiés.

**EXEMPLE E : STIMULATION DES REPONSES IMMUNITAIRES**

A des groupes de six souris on a administré des globules rouges de moutons. Ces groupes de souris ont ensuite été traités par voie sous cutanée par les composés de l'invention pendant six jours et un groupe témoin a été traité par un placébo. Les souris sont ensuite laissées au repos pendant quatre semaines puis ont ensuite reçu une injection de rappel de globules rouges de mouton sans reçevoir de nouvelles administrations de produit de l'invention. La réponse immunitaire a été évaluée 3 jours après l'injection de rappel. Elle est statistiquement accrue dans le groupe traité par les composés de l'invention.

**EXEMPLE F : INHIBITION DE L'OVULATION**

On utilise des rates femelles adultes avec des cycles réguliers de quatre jours.

Des frottis vaginaux quotidiens ont été réalisés et des rates ont été sélectionnées après qu'elles ont montré au moins deux cycles consécutifs de quatre jours.

Chaque cycle est constitué de deux jours de dioestrus, un jour de proestrus et un jour d'oestrus.

L'après-midi du jour de proestrus, l'hormone lutéinisante est libérée dans le sang par l'hypophyse. Cette hormorne induit l'ovulation qui se traduit par la présence d'oeufs au niveau de l'oviducte le jour de l'oestrus.

Les composés de l'invention sont administrés par voie orale à midi le jour de l'oestrus. Les rates traitées et témoins sont sacrifiées le jour de l'oestrus. Les oviductes sont examinés. On remarque un pourcentage significatif de diminution du nombre des oeufs dans les oviductes de rates traitées.

**EXEMPLE G : MISE EN EVIDENCE DE L'ACTIVITE ANALGESIQUE**

L'activité sur la douleur a été recherchée chez la souris (23-25 g) selon un protocole dérivé de la technique décrite par SIEGMUND (SIEGMUND E.A., R.A. CADMUS & GOLU, J. Pharm. Exp. Ther. 119, 1874, 1954). Les souris, réparties par randomisation en lots de 12 animaux, ont reçu le traitement par voie orale (excipient

pour les témoins) 1 heure avant l'injection intra-péritonéale d'une solution hydroalcoolique de phényl-p-benzoquinone (Sigma) à 0,02%. Les étirements sont dénombrés entre la 5ème et 10ème minute après l'injection.

Il est apparu que certains composés de l'invention possèdent une activité analgésique.

**EXEMPLE H : POTENTIALISATION DU SOMMEIL INDUIT PAR LES BARBITURIQUES**

50 mg.$kg^{-1}$ de pentobarbital sont injectés par voie intrapéritonéale à des souris (22-25 g). On mesure le temps d'apparition et la durée du sommeil. On admet qu'il y a sommeil lorsque les animaux perdent le réflexe de retournement. Les composés à tester sont administrés par voie intrapéritonéale 30 minutes avant l'injection du barbiturique. Certains produits de l'invention augmentent la durée du sommeil induit par le pentobarbital.

**EXEMPLE I : TEST DE BINDING AUX RECEPTEURS DE LA MELATONINE**

Le binding aux récepteurs de la mélatonine des composés de l'invention a été réalisé selon des techniques classiques. Il apparaît que les composés de l'invention se lient de façon très spécifique aux récepteurs de la mélatonine avec une affinité supérieure à la mélatonine elle même. Les plus intéressants ont un K.d de 5,5 x $10^{-13}$ alors que la mélatonine elle même possèdent un K.d de 6,3 x 10-11, ce qui signifie que certains produits de l'invention ont une affinité sélective pour les récepteurs de la mélatonine 100 fois supérieure à la mélatonine elle même.

**EXEMPLE J : ETUDE DE L'ACTIVITE HYPOGLYCEMIANTE**

Des souris mâles KK ont été placées dans des cages à l'âge de huit semaines. Elles sont utilisées pour l'expérience lorsque leur poids est supérieur à 40 grammes à l'âge de 4-5 mois.

Le composé de l'invention est placé en suspension dans du sirop de gomme. Chaque composé testé est administré oralement 18 heures avant le prélèvement sanguin.

Le sang est recueilli par prélèvement au niveau de la veine caudale dans un tube à hématocrite, puis centrifugé. Le plasma est recueilli et le dosage de la glycémie effectué.

Il apparaît que certains composés de l'invention diminuent la glycémie de façon significative.

**EXEMPLE K : COMPOSITION PHARMACEUTIQUE : COMPRIMES**

Comprimés dosés à 50 mg de N-[2-(7-méthoxy-1-naphtyl)éthyl]butyramide

| | |
|---|---|
| N[2-(7-méthoxy-1-naphtyl)éthyl]butyramide | 50 g |
| Amidon de blé | 15 g |
| Amidon de maïs | 15 g |
| Lactose | 15 g |
| Stéarate de magnésium | 2 g |
| Silice | 1 g |
| Hydroxypropyl cellulose | 2 g |

## Revendications

**Revendications pour les Etats contractants suivants : AT, BE, CH, DE, DK, FR, GB, IT, LI, LU, NL, SE**

**1.** Composés de formule générale (I) :

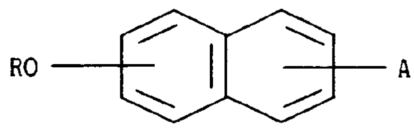

(I)

dans laquelle :

A représente un groupement

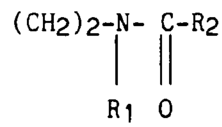

R représente un groupement alkyle inférieur linéaire ou ramifié,

$R_1$ représente un atome d'hydrogène ou un groupement alkyle inférieur, linéaire ou ramifié,

$R_2$ représente

. un atome d'hydrogène,

. un groupement alkyle inférieur linéaire ou ramifié ou cycloalkyle éventuellement substitué par un atome d'halogène,

. un groupement aryle ou hétéroaryle ou arylalkyle inférieur ou aryle substitué ou hétéroaryle substitué ou arylalkyle substitué, étant entendu que par groupement hétéroaryle on entend un groupement insaturé, mono ou bicyclique comprenant de 1 à 3 hétéroatomes choisi parmi azote, oxygène ou soufre chaque cycle comprenant 4 ou 5 sommets, et que par groupement aryle on entend phényle ou naphtyle,

. un groupement imidazolyle éventuellement réduit et/ou substitué par un groupement oxo,

. un groupement de formule :

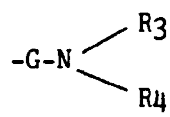

G représentant un groupement alkyle inférieur linéaire ou ramifié,

$R_3$ et $R_4$ identiques ou différents représentent chacun un groupement alkyle inférieur ou un atome d'hydrogène ou un groupement phényle ou phénylalkyle inférieur, ou $R_3$ et $R_4$ forment avec l'atome d'azote auquel ils sont attachés un système hétérocyclique, aromatique ou non, mono ou bicyclique chaque cycle ayant cinq ou six sommets comprenant éventuellement un autre hétéroatome et étant éventuellement substitué par un ou plusieurs groupements alkyle inférieur ou oxo, aryle ou arylalkyle inférieur, ou aryle substitué ou arylalkyle inférieur substitué,

étant entendu que le terme substitué affectant les groupements aryle et arylalkyle ou hétéroaryle dans la définition de $R_2$, $R_3$, $R_4$ signifie que ces groupements sont substitués par un ou plusieurs radicaux choisis parmi alkyle inférieur, alkoxy inférieur, trifluorométhyle ou atome d'halogène,

ou bien $R_1$ forme avec $R_2$ et le groupement N-CO un système hétérocyclique de formule :

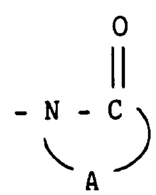

avec A étant un radical alkyle de 2 à 8 atomes de carbone linéaire ou ramifié,

ainsi que, le cas échéant, leurs isomères, épimères, diastéréoisomères et leurs sels d'addition à un acide pharmaceutiquement acceptable,

étant entendu que les termes alkyle inférieur et alkoxy inférieur signifient des groupements comprenant 1 à 6 atomes de carbone et que cycloalkyle signifient des groupements comprenant 3 à 8 atomes de carbone.

**2.** Composés selon la revendication 1 dans laquelle le groupement OR se trouve en position 7, ainsi que, le cas échéant, leurs isomères, épimères, diastéréoisomères et leurs sels d'addition à un acide pharmaceutiquement acceptable.

**3.** Composés selon la revendication 1 dans laquelle $R_1$ représente un atome d'hydrogène ou forme avec $R_2$ et le groupement N-CO le système pyrrolidinone-2, ainsi que, le cas échéant, leurs isomères, épimères, diastéréoisomères et leurs sels d'addition à un acide pharmaceutiquement acceptable.

**4.** Composés selon la revendication 1 dans laquelle $R_2$ représente :

. un groupement alkyle inférieur linéaire ou ramifié ou cycloalkyle éventuellement substitué par un atome d'halogène,

. un groupement aryle ou hétéroaryle mono ou bicyclique ou arylalkyle inférieur éventuellement substitué par un ou plusieurs atomes d'halogène ou groupements choisis parmi alkyle inférieur, alkoxy inférieur, ou trifluorométhyle,

. un groupement de formule :

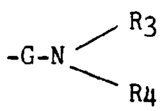

G représentant un groupement alkyle inférieur linéaire ou ramifié,

$R_3$ et $R_4$ forment avec l'atome d'azote auquel ils sont attachés un système hétérocyclique, mono ou bicyclique chaque cycle possédant cinq ou six sommets comprenant éventuellement un autre hétéroatome et éventuellement substitué par un groupement alkyle inférieur ou oxo, aryle ou arylalkyle inférieur, ou aryle substitué ou arylalkyle inférieur substitué étant entendu que le terme substitué affectant les groupements aryle et arylalkyle signifie que ces groupements peuvent être substitués par un ou plusieurs radicaux choisis parmi alkyle inférieur, alkoxy inférieur, trifluorométhyle ou atome d'halogène,

ainsi que, le cas échéant, leurs isomères, épimères, diastéréoisomères et leurs sels d'addition à un acide pharmaceutiquement acceptable.

**5.** Composés selon l'une des revendications 1 ou 2 dans laquelle le groupement OR se trouve en position 7, $R_1$ représente un atome d'hydrogène et $R_2$ représente un groupement alkyle inférieur linéaire ou ramifié ou cycloalkyle éventuellement substitués par un atome d'halogène ainsi que, le cas échéant, leurs isomères, épimères, diastéréoisomères.

**6.** Composés selon la revendication 1 dans laquelle le groupement OR se trouve en position 7, $R_1$ représente un atome d'hydrogène et $R_2$ représente un groupement aryle ou hétéroaryle mono ou bicyclique éventuellement substitué par un ou plusieurs atomes d'halogène ainsi que, le cas échéant, leurs isomères, épimères, diastéréoisomères.

**7.** Composé selon la revendication 1 qui est le N-[2-(7-méthoxy napht-1-yl) éthyl] cyclopropylcarboxamide.

**8.** Composé selon la revendication 1 qui est le N-[2-(7-méthoxy napht-1-yl) éthyl] (2-oxo pyrrolidin-1-yl) acétamide.

**9.** Composé selon la revendication 1 qui est le N-[2-(7-méthoxy napht-1-yl) éthyl] pyrrolidin-2-one.

**10.** Composé selon la revendication 1 qui est le N-[2-(7-méthoxy napht-1-yl) éthyl] acétamide.

**11.** Composé selon la revendication 1 qui est le N-[2-(7-méthoxy napht-1-yl) éthyl] isobutyramide.

**12.** Composé selon la revendication 1 qui est le N-[2-(7-méthoxy napht-1-yl) éthyl] butyramide.

**13.** Composé selon la revendication 1 qui est le N-[2-(7-méthoxy napht-1-yl) éthyl] proprinamide.

**14.** Composé selon la revendication 1 qui est le N-[2-(7-méthoxy napht-1-yl) éthyl] 4-chlorobutyramide.

**15.** Composé selon la revendication 1 qui est le N-[2-(7-méthoxy napht-1-yl) éthyl]-2-morpholinoacétamide.

**16.** Composé selon la revendication 1 qui est le N-[2-(7-méthoxy napht-1-yl) éthyl]-2-{4-[(2,3,4-triméthoxyphényl)méthyl] pipérazin-1-yl} acétamide ainsi que ses sels d'addition à un acide pharmaceutiquement acceptable.

**17.** Composé selon la revendication 1 qui est le N[2-(7-méthoxy napht-1-yl) éthyl] benzamide.

**18.** Composé selon la revendication 1 qui est le N[2-(7-méthoxy napht-1-yl) éthyl]-3,5-dichloro-benzamide.

**19.** Composé selon la revendication 1 qui est le N[2-(7-méthoxy napht-1-yl) éthyl] (indol-2-yl) carboxamide.

**20.** Composé selon la revendication 1 qui est le N[2-(7-méthoxy napht-1-yl) éthyl] pentanamide.

**21.** Composé selon la revendication 1 qui est le N[2-(7-méthoxy napht-1-yl) éthyl]cyclobutane carboxamide.

**22.** Composé selon la revendication 1 qui est le N[2-(7-méthoxy napht-1-yl) éthyl] 2-iodoacétamide.

**23.** Composé selon la revendication 1 qui est le N[2-(7-méthoxy napht-1-yl) éthyl] 2-bromoacétamide.

**24.** Composé selon la revendication 1 qui est le N[2-(7-méthoxy napht-1-yl) éthyl]2-chloroacétamide.

**25.** Procédé de préparation des composés de formule (I) selon la revendication 1, caractérisé en ce que l'on utilise comme matière première un dérivé de formule (II) :

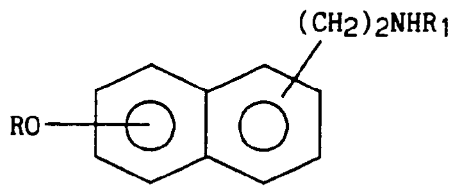

(II)

dans laquelle R et $R_1$ ont la même signification que dans la formule (I), que l'on traite :
- ou bien par un dérivé de formule (III) :

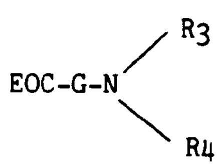

(III)

dans laquelle E signifie un groupe partant choisi parmi hydroxyle, alkoxy inférieur ou halogène, G, $R_3$ et $R_4$ ayant la même signification que dans la formule (I),
éventuellement en présence d'un agent alcalin,
pour conduire à un dérivé de formule (I/A), cas particulier des dérivés de formule (I) :

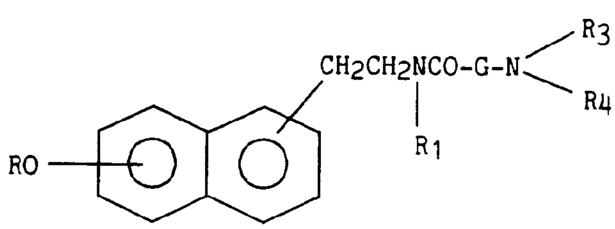

(I/A)

dans laquelle R, $R_1$, $R_3$, $R_4$ et G ont la même définition que précédemment,
$R_2$ signifiant ici un groupement :

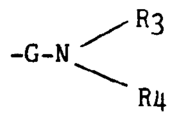

que l'on purifie, si on le désire, par des techniques classiques telles que chromatographie, cristallisation et que l'on salifie, si on le désire par un acide pharmaceutiquement acceptable,
- ou bien par un chlorure d'acide de formule (IV) :

$$Cl\text{-}CO\text{-}R'_2 \qquad \textbf{(IV)}$$

ou par l'anhydride d'acide correspondant,
$R'_2$ signifiant :

- un groupement alkyle inférieur linéaire ou ramifié ou cycloalkyle éventuellement substitué par un atome d'halogène,
- un groupement aryle ou hétéroaryle ou arylalkyle inférieur éventuellement substitué par un ou plusieurs atomes d'halogène ou groupements choisis parmi alkyle inférieur, alkoxy inférieur, ou trifluorométhyle,
- un groupement imidazolyle éventuellement réduit et/ou substitué par un groupement oxo,

pour conduire à un dérivé de formule (I/B) :

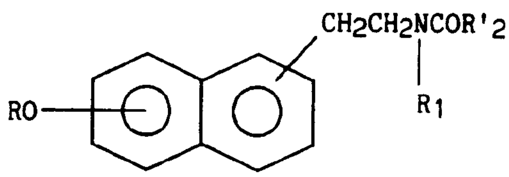

**(I/B)**

dans laquelle R, $R_1$ et $R'_2$ ont la même définition que précédemment,
que l'on purifie si nécessaire par des techniques classiques telles que chromatographie et/ou cristallisation
dérivé de formule (I/B) qui dans le cas où R'2 représente un groupement alkyle inférieur, linéaire ou ramifié, substitué par un atome d'halogène, peut-être soumis, si on le désire à l'action d'une amine de formule (V) :

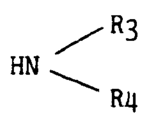

**(V)**

dans laquelle $R_3$ et $R_4$ ont la même définition que précédemment,
en excès ou en présence d'une amine tertiaire ou d'un sel de métal alcalin pour conduire à un dérivé de formule (I/A) tel que précédemment défini, qui si on le désire est purifié par une technique classique telle que chromatographie et/ou cristallisation, et/ou salifié par un acide pharmaceutiquement acceptable,
dérivé de formule (I/B) qui lorsque $R'_2$ représente un substituant alkyle linéaire ou ramifié comprenant au moins deux atomes de carbone et substitué par un atome d'halogène, et lorsque, simultanément $R_1$ représente un atome d'hydrogène, peut être soumis si on le désire, à l'action d'une base forte et préférentiellement un alcoolate de métal alcalin pour conduire à un dérivé de formule (I/C) :

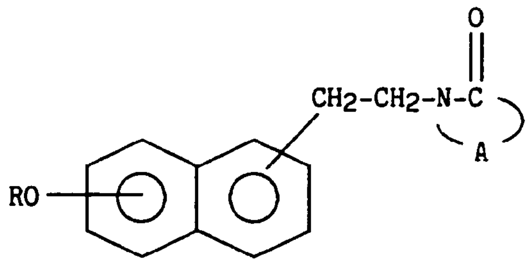

(I/C)

dans laquelle R a la même signification que précédemment et A représente un groupement alkyle linéaire ou ramifié comprenant de 2 à 8 atomes de carbone, cas particulier des dérivés de formule (I) pour lesquels $R_1$ et $R_2$ forment avec NCO un système monocyclique substitué par un groupement oxo, et éventuellement substitué par un ou plusieurs groupements alkyle inférieur,
que l'on purifie, si on le désire, par une technique choisie parmi cristallisation ou chromatographie.

**26.** Procédé de préparation selon la revendication 25 des composés de formule (I/A) :

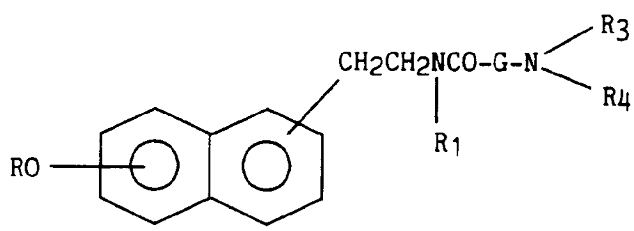

(I/A)

dans laquelle R, $R_1$, $R_3$ $R_4$ et G ont la même signification que dans la formule (I), caractérisé en ce qu'on la traite en dérivé de formule (II) :

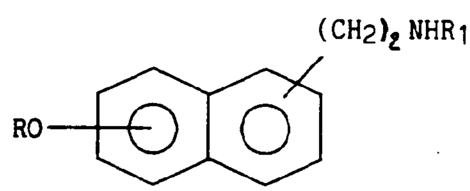

(II)

dans laquelle R et $R_1$ ont la même signification que dans la formule (I), par un dérivé de formule (III) :

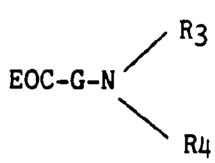

(III)

dans laquelle E signifie un groupe partant choisi parmi hydroxyle, alkoxy inférieur ou halogène, G, $R_3$ et $R_4$ ayant la même signification que dans la formule (I),
éventuellement en présence d'un agent alcalin,
pour conduire à un dérivé de formule (I/A) que l'on purifie, si on le désire, par des techniques classiques telles que chromatographie, cristallisation, dont on sépare le cas échéant les isomères et que l'on salifié, si on le désire, par un acide pharmaceutiquement acceptable.

**27.** Procédé de préparation selon la revendication 25 des composés de formule (I/B) :

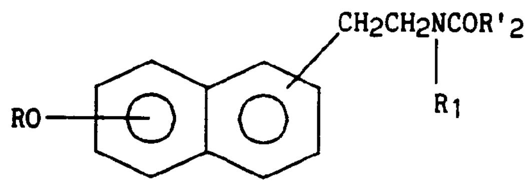

(I/B)

dans laquelle R, $R_1$ ont la même définition que dans la formule (I),
$R_2$ signifiant :

- un groupement alkyle inférieur linéaire ou ramifié ou cycloalkyle éventuellement substitué par un atome d'halogène,
- un groupement aryle ou hétéroaryle ou arylalkyle inférieur éventuellement substitué par un ou plusieurs atomes d'halogène ou groupements choisis parmi alkyle inférieur, alkoxy inférieur, ou trifluorométhyle,

caractérisé en ce que l'on traite un dérivé de formule (II) :

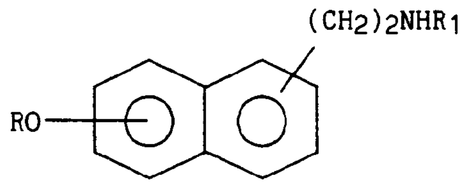

(II)

dans laquelle R et $R_1$ ont la même signification que dans la formule (I),

- par un chlorure d'acide de formule (IV) :

  $Cl\text{-}CO\text{-}R'_2$ **(IV)**

  dans laquelle $R'_2$ a la même définition que précédemment
- ou par l'anhydride d'acide correspondant pour conduire à un dérivé de formule (I/B) que l'on purifie si nécessaire par des techniques classiques telles que chromatographie et/ou cristallisation et dont on sépare les isomères, le cas échéant.

**28.** Procédé de préparation selon la revendication 25 des composés de formule (I/A) :

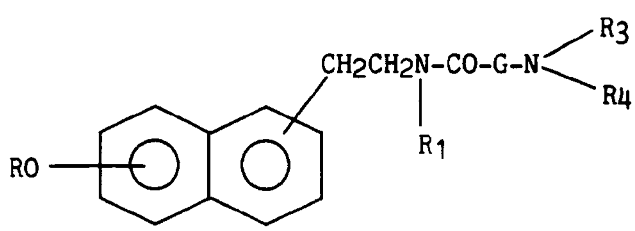

(I/A)

dans laquelle R, $R_1$, $R_3$ et $R_4$ ont la même signification que dans la formule (I), caractérisé en ce que l'on traite un dérivé de formule (II) :

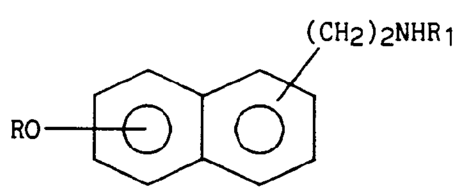

(II)

dans laquelle R et $R_1$ ont la même définition que dans la formule (I),

- par un chlorure d'acide de formule (IV) :

$Cl\text{-}CO\text{-}R'_2$ **(IV)**

- ou par l'anhydride d'acide correspondant, dans laquelle $R'_2$ signifie un groupement alkyle inférieur linéaire ou ramifié substitué par un atome d'halogène,

pour obtenir un dérivé de formule (I/B) :

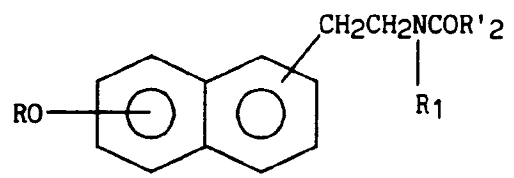

(I/B)

dans laquelle R, $R_1$ et $R'_2$ ont la même définition que précédemment, que l'on purifie si nécessaire par des techniques classiques telles que chromatographie et/ou cristallisation,
que l'on soumet à l'action d'une amine de formule (V) :

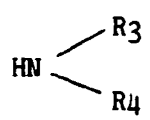

(V)

dans laquelle $R_3$ et $R_4$ ont la même définition que précédemment,
en excès ou en présence d'une amine tertiaire ou d'un sel de métal alcalin pour conduire à un dérivé de formule (I/A) tel que précédemment défini, qui si on le désire est purifié par une technique classique telle que chromatographie et/ou cristallisation, et/ou salifié par un acide pharmaceutiquement acceptable.

**29.** Procédé de préparation selon la revendication 25 des composés de formule (I/C) :

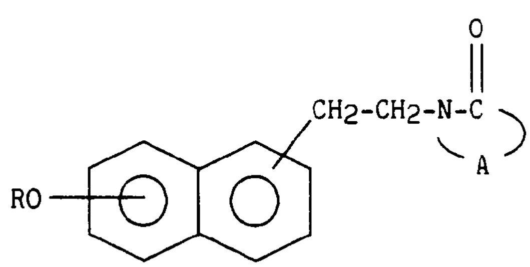

(I/C)

dans laquelle R a la même définition que dans la formule (I) et A représente un groupement alkyle linéaire ou ramifié comprenant de 2 à 8 atomes de carbone,
caractérisé en ce que l'on traite un dérivé de formule (II) :

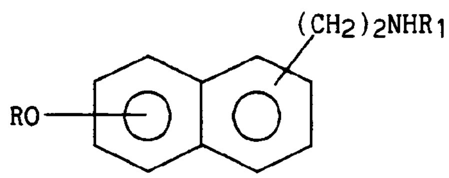

(II)

dans laquelle R a la même définition que dans la formule (I),

- par un chlorure d'acide de formule (IV) :

$Cl\text{-}CO\text{-}R'_2$ **(IV)**

- ou par l'anhydride d'acide correspondant, dans laquelle $R'_2$ signifie un groupement alkyle inférieur linéaire ou ramifié substitué par un atome d'halogène,

pour obtenir un dérivé de formule (I/B) :

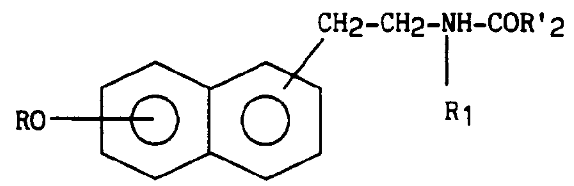

(I/B)

dans laquelle R et $R'_2$ ont la même définition que précédemment,
que l'on purifie si nécessaire par des techniques classiques telles que chromatographie et/ou cristallisation,
que l'on soumet à l'action d'une base forte et préférentiellement un alcoolate de métal alcalin, pour conduire à un dérivé de formule (I/C), que l'on purifie si on le désire par une technique choisie parmi cristallisation ou chromatographie.

**30.** Composition pharmaceutique contenant comme principe actif au moins un composé selon l'une des revendications 1 à 24, en combinaison avec un ou plusieurs excipients ou véhicules inertes non-toxiques, pharmaceutiquement acceptables.

**31.** Composition pharmaceutique selon la revendication 30 utile dans le traitement des troubles du système mélatoninergique et notamment du stress, de l'anxiété, des dépressions saisonnières, des insomnies et fatigues dues aux décalages horaires, schizophrénies, attaque de panique, mélancolie, de la régulation de l'appétit, de l'insomnie, des troubles psychotiques, de l'épilepsie, de la maladie de Parkinson, de la démence sénile, des divers désordres liés au vieillissement normal ou pathologique, pertes de mémoire, maladie d'Alzheimer, ainsi que les troubles de la circulation cérébrale, de certains cancers, du psoriasis, de l'acné, de la séborrhée ou en tant qu'inhibiteur de l'ovulation ou en médecine vétérinaire dans les troubles du pelage.

**Revendications pour l'Etat contractant suivant : ES**

**1.** Procédé de préparation de composés de formule générale (I) :

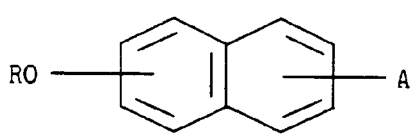

(I)

dans laquelle :

A représente un groupement

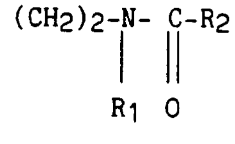

R représente un groupement alkyle inférieur linéaire ou ramifié,

$R_1$ représente un atome d'hydrogène ou un groupement alkyle inférieur, linéaire ou ramifié,

$R_2$ représente

- . un atome d'hydrogène,
- . un groupement alkyle inférieur linéaire ou ramifié ou cycloalkyle éventuellement substitué par un atome d'halogène,
- . un groupement aryle ou hétéroaryle ou arylalkyle inférieur ou aryle substitué ou hétéroaryle substitué ou arylalkyle substitué, étant entendu que par groupement hétéroaryle on entend un groupement insaturé, mono ou bicyclique comprenant de 1 à 3 hétéroatomes choisi parmi azote, oxygène ou soufre chaque cycle comprenant 4 ou 5 sommets, et que par groupement aryle on entend phényle ou naphtyle,

. un groupement imidazolyle éventuellement réduit et/ou substitué par un groupement oxo,

. un groupement de formule :

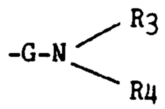

G représentant un groupement alkyle inférieur linéaire ou ramifié,

$R_3$ et $R_4$ identiques ou différents représentent chacun un groupement alkyle inférieur ou un atome d'hydrogène ou un groupement phényle ou phénylalkyle inférieur, ou $R_3$ et $R_4$ forment avec l'atome d'azote auquel ils sont attachés un système hétérocyclique, aromatique ou non, mono ou bicyclique chaque cycle ayant cinq ou six sommets comprenant éventuellement un autre hétéroatome et étant éventuellement substitué par un ou plusieurs groupements alkyle inférieur ou oxo, aryle ou arylalkyle inférieur, ou aryle substitué ou arylalkyle inférieur substitué,

étant entendu que le terme substitué affectant les groupements aryle et arylalkyle ou hétéroaryle dans la définition de $R_2$, $R_3$, $R_4$ signifie que ces groupements sont substitués par un ou plusieurs radicaux choisis parmi alkyle inférieur, alkoxy inférieur, trifluorométhyle ou atome d'halogène,

ou bien $R_1$ forme avec $R_2$ et le groupement N-CO un système hétérocyclique de formule :

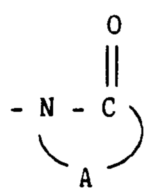

avec A étant un radical alkyle de 2 à 8 atomes de carbone linéaire ou ramifié,

ainsi que, le cas échéant, leurs isomères, épimères, diastéréoisomères et leurs sels d'addition à un acide pharmaceutiquement acceptable,

étant entendu que les termes alkyle inférieur et alkoxy inférieur signifient des groupements comprenant 1 à 6 atomes de carbone et que cycloalkyle signifient des groupements comprenant 3 à 8 atomes de carbone,

caractérisé en ce que l'on utilise comme matière première un dérivé de formule (II) :

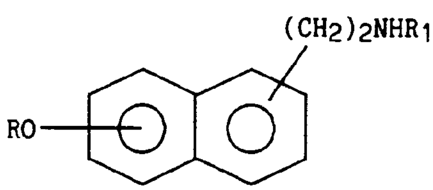

(II)

dans laquelle R et $R_1$ ont la même signification que dans la formule (I), que l'on traite :

- ou bien par un dérivé de formule (III) :

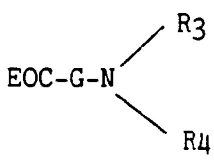

(III)

dans laquelle E signifie un groupe partant choisi parmi hydroxyle, alkoxy inférieur ou halogène, G, $R_3$ et

$R_4$ ayant la même signification que dans la formule (I), éventuellement en présence d'un agent alcalin, pour conduire à un dérivé de formule (I/A), cas particulier des dérivés de formule (I) :

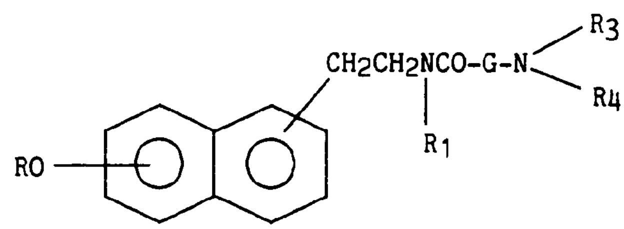

(I/A)

dans laquelle R, $R_1$, $R_3$, $R_4$ et G ont la même définition que précédemment, $R_2$ signifiant ici un groupement :

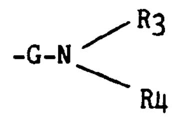

que l'on purifie, si on le désire, par des techniques classiques telles que chromatographie, cristallisation et que l'on salifie, si on le désire par un acide pharmaceutiquement acceptable,
- ou bien par un chlorure d'acide de formule (IV) :

$$Cl\text{-}CO\text{-}R'_2 \qquad \textbf{(IV)}$$

ou par l'anhydride d'acide correspondant,
$R'_2$ signifiant :

- un groupement alkyle inférieur linéaire ou ramifié ou cycloalkyle éventuellement substitué par un atome d'halogène,
- un groupement aryle ou hétéroaryle ou arylalkyle inférieur éventuellement substitué par un ou plusieurs atomes d'halogène ou groupements choisis parmi alkyle inférieur, alkoxy inférieur, ou trifluorométhyle,
- un groupement imidazolyle éventuellement réduit et/ou substitué par un groupement oxo,

pour conduire à un dérivé de formule (I/B) :

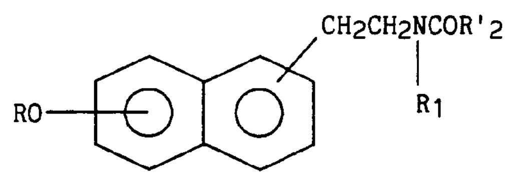

(I/B)

dans laquelle R, $R_1$ et $R'_2$ ont la même définition que précédemment, que l'on purifie si nécessaire par des techniques classiques telles que chromatographie et/ou cristallisation dérivé de formule (I/B) qui dans le cas où $R'_2$ représente un groupement alkyle inférieur, linéaire ou ramifié, substitué par un atome d'halogène, peut-être soumis, si on le désire à l'action d'une amine de formule (V) :

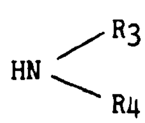

(V)

dans laquelle $R_3$ et $R_4$ ont la même définition que précédemment, en excès ou en présence d'une amine tertiaire ou d'un sel de métal alcalin pour conduire à un dérivé de formule (I/A) tel que précédemment défini, qui si on le désire est purifié par une technique classique telle que chromatographie et/ou cristallisation, et/ou salifié par un acide pharmaceutiquement acceptable,

dérivé de formule (I/B) qui lorsque $R'_2$ représente un substituant alkyle linéaire ou ramifié comprenant au moins deux atomes de carbone et substitué par un atome d'halogène, et lorsque, simultanément $R_1$ représente un atome d'hydrogène, peut être soumis si on le désire, à l'action d'une base forte et préférentiellement un alcoolate de métal alcalin pour conduire à un dérivé de formule (I/C) :

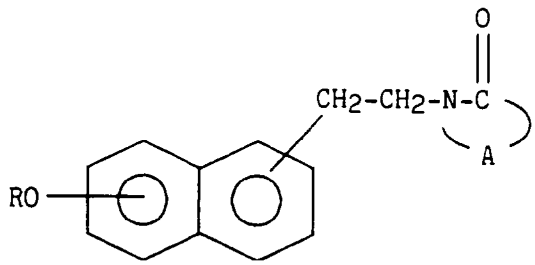

(I/C)

dans laquelle R a la même signification que précédemment et A représente un groupement alkyle linéaire ou ramifié comprenant de 2 à 8 atomes de carbone, cas particulier des dérivés de formule (I) pour lesquels $R_1$ et $R_2$ forment avec NCO un système monocyclique substitué par un groupement oxo, et éventuellement par un ou plusieurs groupements alkyle inférieur,
que l'on purifie, si on le désire, par une technique choisie parmi cristallisation ou chromatographie.

**2.** Procédé de préparation selon la revendication 1 des composés de formule (I/A) :

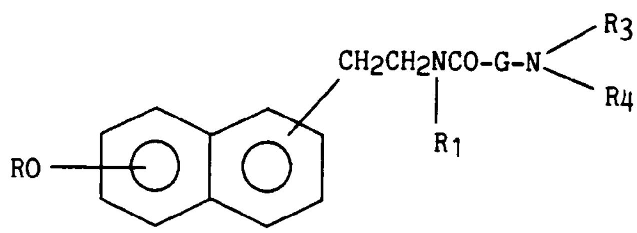

(I/A)

dans laquelle R, $R_1$, $R_3$, $R_4$ et G ont la même signification que dans la formule (I), caractérisé en ce qu'on la traite en dérivé de formule (II) :

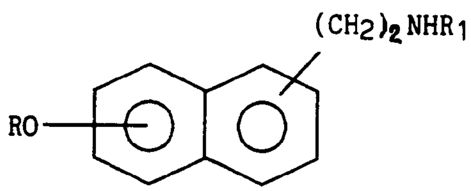

(II)

dans laquelle R et $R_1$ ont la même signification que dans la formule (I), par un dérivé de formule (III) :

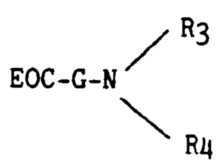

(III)

dans laquelle E signifie un groupe partant choisi parmi hydroxyle, alkoxy inférieur ou halogène, G, $R_3$ et $R_4$ ayant la même signification que dans la formule (I),
éventuellement en présence d'un agent alcalin,
pour conduire à un dérivé de formule (I/A) que l'on purifie, si on le désire, par des techniques classiques

telles que chromatographie, cristallisation, dont on sépare le cas échéant les isomères et que l'on sacrifie, si on le désire, par un acide pharmaceutiquement acceptable.

3. Procédé de préparation selon la revendication 1 des composés de formule (I/B) :

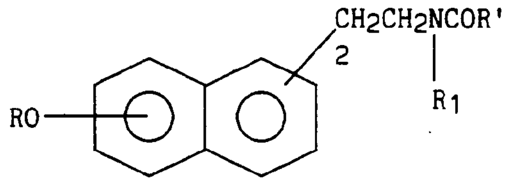

**(I/B)**

dans laquelle R, $R_1$ ont la même définition que dans la formule (I),
$R_2$ signifiant :

- un groupement alkyle inférieur linéaire ou ramifié ou cycloalkyle éventuellement substitué par un atome d'halogène,
- un groupement aryle ou hétéroaryle ou arylalkyle inférieur éventuellement substitué par un ou plusieurs atomes d'halogène ou groupements choisis parmi alkyle inférieur, alkoxy inférieur, ou trifluorométhyle,

caractérisé en ce que l'on traite un dérivé de formule (II) :

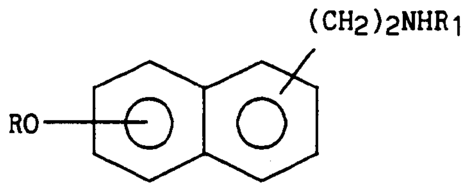

**(II)**

dans laquelle R et $R_1$ ont la même signification que dans la formule (I),

- par un chlorure d'acide de formule (IV) :

  $Cl\text{-}CO\text{-}R'_2$ **(IV)**

  dans laquelle $R'_2$ a la même définition que précédemment
- ou par l'anhydride d'acide correspondant pour conduire à un dérivé de formule (I/B) que l'on purifie si nécessaire par des techniques classiques telles que chromatographie et/ou cristallisation et dont on sépare les isomères, le cas échéant.

4. Procédé de préparation selon la revendication 1 des composés de formule (I/A) :

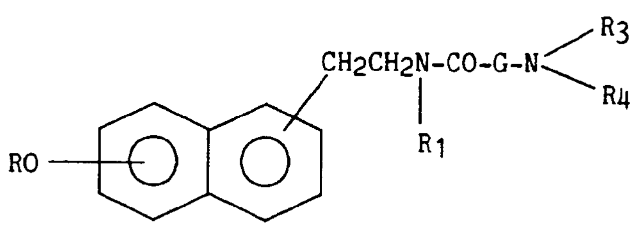

**(I/A)**

dans laquelle R, $R_1$, $R_3$ et $R_4$ ont la même signification que dans la formule (I), caractérisé en ce que l'on traite un dérivé de formule (II) :

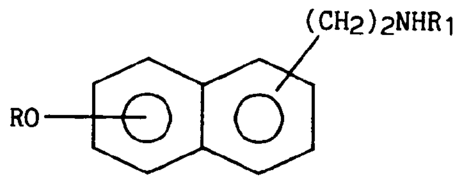

(II)

dans laquelle R et $R_1$ ont la même définition que dans la formule (I),

- par un chlorure d'acide de formule (IV) :

  Cl-CO-R'$_2$ **(IV)**

- ou par l'anhydride d'acide correspondant, dans laquelle R'$_2$ signifie un groupement alkyle inférieur linéaire ou ramifié substitué par un atome d'halogène,

pour obtenir un dérivé de formule (I/B) :

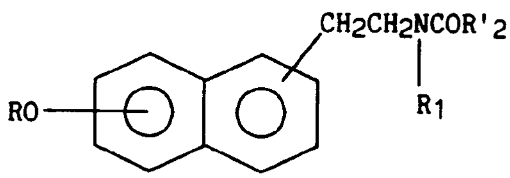

**(I/B)**

dans laquelle R, $R_1$ et R'$_2$ ont la même définition que précédemment, que l'on purifie si nécessaire par des techniques classiques telles que chromatographie et/ou cristallisation,
que l'on soumet à l'action d'une amine de formule (V) :

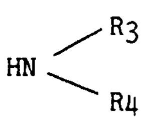

(V)

dans laquelle $R_3$ et $R_4$ ont la même définition que précédemment,
en excès ou en présence d'une amine tertiaire ou d'un sel de métal alcalin pour conduire à un dérivé de formule (I/A) tel que précédemment défini, qui si on le désire est purifié par une technique classique telle que chromatographie et/ou cristallisation, et/ou salifié par un acide pharmaceutiquement acceptable.

**5.** Procédé de préparation selon la revendication 1 des composés de formule (I/C) :

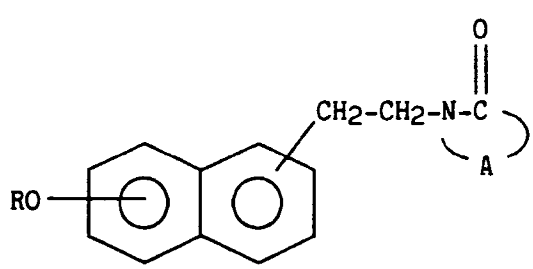

(I/C)

dans laquelle R a la même définition que dans la formule (I) et A représente un groupement alkyle linéaire ou ramifié comprenant de 2 à 8 atomes de carbone,
caractérisé en ce que l'on traite un dérivé de formule (II) :

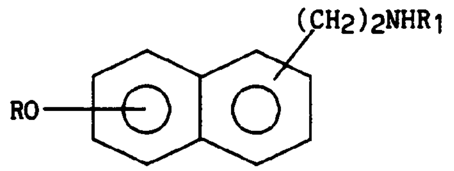

(II)

dans laquelle R a la même définition que dans la formule (I),

- par un chlorure d'acide de formule (IV) :

$$Cl\text{-}CO\text{-}R'_2 \quad \textbf{(IV)}$$

- ou par l'anhydride d'acide correspondant, dans laquelle $R'_2$ signifie un groupement alkyle inférieur linéaire ou ramifié substitué par un atome d'halogène,
  pour obtenir un dérivé de formule (I/B) :

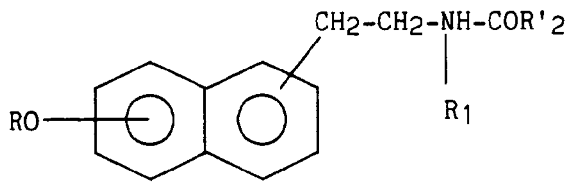

(I/B)

dans laquelle R et $R'_2$ ont la même définition que précédemment,
que l'on purifie si nécessaire par des techniques classiques telles que chromatographie et/ou cristallisation,
que l'on soumet à l'action d'une base forte et préférentiellement un alcoolate de métal alcalin, pour conduire à un dérivé de formule (I/C), que l'on purifie si on le désire par une technique choisie parmi cristallisation ou chromatographie.

**6.** Procédé selon l'une des revendications 1 à 5 de préparation de composés de formule (I) dans laquelle le groupement OR se trouve en position 7, ainsi que, le cas échéant, de leurs isomères, épimères, diastéréoisomères et de leurs sels d'addition à un acide pharmaceutiquement acceptable.

**7.** Procédé selon l'une des revendications 1 à 5 de préparation de composés de formule (I) dans laquelle $R_1$ représente un atome d'hydrogène ou forme avec $R_2$ et le groupement N-CO le système pyrrolidinone-2, ainsi que, le cas échéant, de leurs isomères, épimères, diastéréoisomères et leurs sels d'addition à un acide pharmaceutiquement acceptable.

**8.** Procédé selon la revendication 1 de préparation de dérivés de formule (I) dans laquelle $R_2$ représente :

. un groupement alkyle inférieur linéaire ou ramifié ou cycloalkyle éventuellement substitué par un atome d'halogène,

. un groupement aryle ou hétéroaryle mono ou bicyclique ou arylalkyle inférieur éventuellement substitué par un ou plusieurs atomes d'halogène ou groupements choisis parmi alkyle inférieur, alkoxy inférieur, ou trifluorométhyle,

. un groupement de formule :

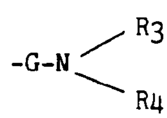

G représentant un groupement alkyle inférieur linéaire ou ramifié,
$R_3$ et $R_4$ forment avec l'atome d'azote auquel ils sont attachés un système hétérocyclique, mono ou bicyclique chaque cycle possédant cinq ou six sommets comprenant éventuellement un autre hétéroatome et éventuellement substitué par un groupement alkyle inférieur ou oxo, aryle ou arylalkyle inférieur, ou aryle substitué ou arylalkyle inférieur substitué étant entendu que le terme substitué affectant les groupements aryle et arylalkyle signifie que ces groupements peuvent être substitués par un ou plusieurs radicaux choisis parmi alkyle inférieur, alkoxy inférieur, trifluorométhyle ou atome d'halogène,

ainsi que, le cas échéant, leurs isomères, épimères, diastéréoisomères et leurs sels d'addition à un acide pharmaceutiquement acceptable.

**9.** Procédé selon l'une des revendications 1 à 6 de préparation de composés de formule (I) dans laquelle le groupement OR se trouve en position 7, $R_1$ représente un atome d'hydrogène et $R_2$ représente un groupement alkyle inférieur linéaire ou ramifié ou cycloalkyle éventuellement substitués par un atome d'halogène ainsi que, le cas échéant, leurs isomères, épimères, diastéréoisomères.

**10.** Procédé selon la revendication 1 de préparation de composés de formule (I) dans laquelle le groupement OR se trouve en position 7, $R_1$ représente un atome d'hydrogène et $R_2$ représente un groupement aryle ou hétéroaryle mono ou bicyclique éventuellement substitué par un ou plusieurs atomes d'halogène ainsi que, le cas échéant, leurs isomères, épimères, diastéréoisomères.

**11.** Procédé de préparation selon la revendication 1 du composé de formule (I) qui est le N-[2-(7-méthoxy napht-1-yl) éthyl] cyclopropylcarboxamide.

**12.** Procédé de préparation selon la revendication 1 du composé de formule (I) qui est le N-[2-(7-méthoxy napht-1-yl) éthyl] (2-oxo pyrrolidin-1-yl) acétamide.

**13.** Procédé de préparation selon la revendication 1 du composé de formule (I) qui est le N-[2-(7-méthoxy napht-1-yl) éthyl] pyrrolidin-2-one.

**14.** Procédé de préparation selon la revendication 1 du composé de formule (I) qui est le N-[2-(7-méthoxy napht-1-yl) éthyl] acétamide.

**15.** Procédé de préparation selon la revendication 1 du composé de formule (I) qui est le N-[2-(7-méthoxy napht-1-yl) éthyl] isobutyramide.

**16.** Procédé de préparation selon la revendication 1 du composé de formule (I) qui est le N-[2-(7-méthoxy napht-1-yl) éthyl] butyramide.

**17.** Procédé de préparation selon la revendication 1 du composé de formule (I) qui est le N-[2-(7-méthoxy napht-1-yl) éthyl] propionamide.

**18.** Procédé de préparation selon la revendication 1 du composé de formule (I) qui est qui est le N-[2-(7-méthoxy napht-1-yl) éthyl] 4-chlorobutyramide.

**19.** Procédé de préparation selon la revendication 1 du composé de formule (I) qui est le N-[2-(7-méthoxy napht-1-yl) éthyl]-2-morpholinoacétamide.

**20.** Procédé de préparation selon la revendication 1 du composé de formule (I) qui est le N-[2-(7-méthoxy napht-1-yl) éthyl]-2-{4-[(2,3,4-triméthoxyphényl)méthyl] pipérazin-1-yl} acétamide ainsi que ses sels d'addition à un acide pharmaceutiquement acceptable.

**21.** Procédé de préparation selon la revendication 1 du composé de formule (I) qui est le N-[2-(7-méthoxy napht-1-yl) éthyl] benzamide.

**22.** Procédé de préparation selon la revendication 1 du composé de formule (I) qui est le N-[2-(7-méthoxy napht-1-yl) éthyl]-3,5-dichloro-benzamide.

**23.** Procédé de préparation selon la revendication 1 du composé de formule (I) qui est le N-[2-(7-méthoxy napht-1-yl) éthyl] (indol-2-yl) carboxamide.

**24.** Procédé de préparation selon la revendication 1 du composé de formule (I) qui est le N[2-(7-méthoxy napht-1-yl) éthyl] pentanamide.

**25.** Procédé de préparation selon la revendication 1 du composé de formule (I) qui est le N[2-(7-méthoxy napht-1-yl) éthyl]cyclobutane carboxamide.

**26.** Procédé de préparation selon la revendication 1 du composé de formule (I) qui est le N[2-(7-méthoxy napht-1-yl) éthyl] 2-iodoacétamide.

**27.** Procédé de préparation selon la revendication 1 du composé de formule (I) qui est le N[2-(7-méthoxy napht-1-yl) éthyl] 2-bromoacétamide.

**28.** Procédé de préparation selon la revendication 1 du composé de formule (I) qui est le N[2-(7-méthoxy napht-1-yl) éthyl]2-chloroacétamide.

**29.** Procédé de préparation de compositions pharmaceutiques contenant comme principe actif au moins un composé selon l'une des revendications 11 à 28, en combinaison avec un ou plusieurs excipients ou véhicules inertes non-toxiques, pharmaceutiquement acceptables.

**30.** Procédé de préparation de composition pharmaceutique selon la revendication 29 utile dans le traitement des troubles du système mélatoninergique et notamment du stress, de l'anxiété, des dépressions saisonnières, des insomnies et fatigues dues aux décalages horaires, schizophrénies, attaque de panique, mélancolie, de la régulation de l'appétit, de l'insomnie, des troubles psychotiques, de l'épilepsie, de la maladie de Parkinson, de la démence sénile, des divers désordres liés au vieillissement normal ou pathologique, pertes de mémoire, maladie d'Alzheimer, ainsi que les troubles de la circulation cérébrale, de certains cancers, du psoriasis, de l'acné, de la séborrhée ou en tant qu'inhibiteur de l'ovulation ou en médecine vétérinaire dans les troubles du pelage.

**Revendications pour l'Etat contractant suivant : GR**

**1.** Procédé de préparation de composés de formule générale (I) :

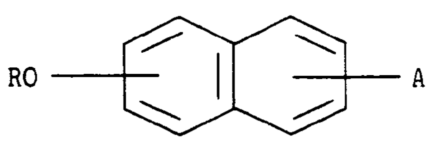

(I)

dans laquelle :

A représente un groupement

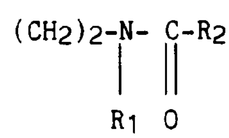

R représente un groupement alkyle inférieur linéaire ou ramifié,

$R_1$ représente un atome d'hydrogène ou un groupement alkyle inférieur, linéaire ou ramifié,

$R_2$ représente

. un atome d'hydrogène,

. un groupement alkyle inférieur linéaire ou ramifié ou cycloalkyle éventuellement substitué par un atome d'halogène,

. un groupement aryle ou hétéroaryle ou arylalkyle inférieur ou aryle substitué ou hétéroaryle substitué ou arylalkyle substitué, étant entendu que par groupement hétéroaryle on entend un groupement insaturé, mono ou bicyclique comprenant de 1 à 3 hétéroatomes choisi parmi azote, oxygène ou soufre chaque cycle comprenant 4 ou 5 sommets, et que par groupement aryle on entend phényle ou naphtyle,

. un groupement imidazolyle éventuellement réduit et/ou substitué par un groupement oxo,

. un groupement de formule :

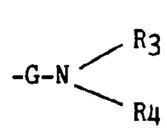

G représentant un groupement alkyle inférieur linéaire ou ramifié,

$R_3$ et $R_4$ identiques ou différents représentent chacun un groupement alkyle inférieur ou un atome d'hydrogène ou un groupement phényle ou phénylalkyle inférieur, ou $R_3$ et $R_4$ forment avec l'atome d'azote auquel ils sont attachés un système hétérocyclique, aromatique ou non, mono ou bicyclique chaque cycle ayant cinq ou six sommets comprenant éventuellement un autre hétéroatome et étant éventuellement substitué par un ou plusieurs groupements alkyle inférieur ou oxo, aryle ou arylalkyle inférieur, ou aryle substitué ou arylalkyle inférieur substitué,

étant entendu que le terme substitué affectant les groupements aryle et arylalkyle ou hétéroaryle dans la définition de $R_2$, $R_3$, $R_4$ signifie que ces groupements sont substitués par un ou plusieurs radicaux choisis parmi alkyle inférieur, alkoxy inférieur, trifluorométhyle ou atome d'halogène,

ou bien $R_1$ forme avec $R_2$ et le groupement N-CO un système hétérocyclique de formule :

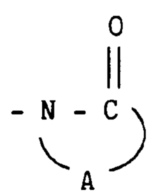

avec A étant un radical alkyle de 2 à 8 atomes de carbone linéaire ou ramifié,

ainsi que, le cas échéant, leurs isomères, épimères, diastéréoisomères et leurs sels d'addition à un acide pharmaceutiquement acceptable,

étant entendu que les termes alkyle inférieur et alkoxy inférieur signifient des groupements comprenant 1 à 6 atomes de carbone et que cycloalkyle signifient des groupements comprenant 3 à 8 atomes de carbone,

caractérisé en ce que l'on utilise comme matière première un dérivé de formule (II) :

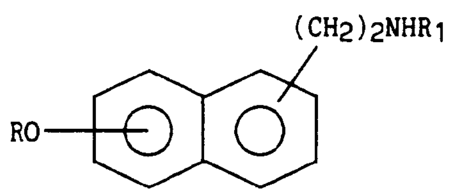

**(II)**

dans laquelle R et $R_1$ ont la même signification que dans la formule (I), que l'on traite :

- ou bien par un dérivé de formule (III) :

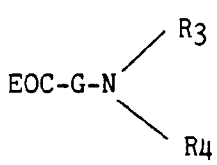

**(III)**

dans laquelle E signifie un groupe partant choisi parmi hydroxyle, alkoxy inférieur ou halogène, G, $R_3$ et $R_4$ ayant la même signification que dans la formule (I),

éventuellement en présence d'un agent alcalin,

pour conduire à un dérivé de formule (I/A), cas particulier des dérivés de formule (I) :

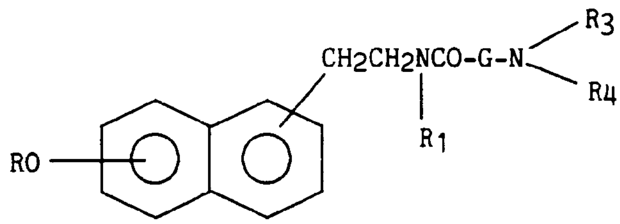

(I/A)

dans laquelle R, $R_1$, $R_3$, $R_4$ et G ont la même définition que précédemment,
$R_2$ signifiant ici un groupement :

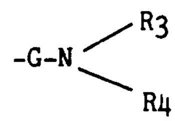

que l'on purifie, si on le désire, par des techniques classiques telles que chromatographie, cristallisation et que l'on salifie, si on le désire par un acide pharmaceutiquement acceptable,
- ou bien par un chlorure d'acide de formule (IV) :

$$Cl\text{-}CO\text{-}R'_2 \qquad \textbf{(IV)}$$

ou par l'anhydride d'acide correspondant,
$R'_2$ signifiant :

- un groupement alkyle inférieur linéaire ou ramifié ou cycloalkyle éventuellement substitué par un atome d'halogène,
- un groupement aryle ou hétéroaryle ou arylalkyle inférieur éventuellement substitué par un ou plusieurs atomes d'halogène ou groupements choisis parmi alkyle inférieur, alkoxy inférieur, ou trifluorométhyle,
- un groupement imidazolyle éventuellement réduit et/ou substitué par un groupement oxo,

pour conduire à un dérivé de formule (I/B) :

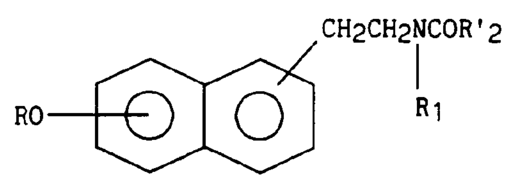

(I/B)

dans laquelle R, $R_1$ et $R'_2$ ont la même définition que précédemment,
que l'on purifie si nécessaire par des techniques classiques telles que chromatographie et/ou cristallisation
dérivé de formule (I/B) qui dans le cas où R'2 représente un groupement alkyle inférieur, linéaire ou ramifié, substitué par un atome d'halogène, peut-être soumis, si on le désire à l'action d'une amine de formule (V) :

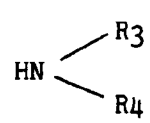

(V)

dans laquelle $R_3$ et $R_4$ ont la même définition que précédemment,
en excès ou en présence d'une amine tertiaire ou d'un sel de métal alcalin pour conduire à un dérivé de formule (I/A) tel que précédemment défini, qui si on le désire est purifié par une technique classique telle que chromatographie et/ou cristallisation, et/ou salifié par un acide pharmaceutiquement acceptable,
dérivé de formule (I/B) qui lorsque $R'_2$ représente un substituant alkyle linéaire ou ramifié comprenant au moins deux atomes de carbone et substitué par un atome d'halogène, et lorsque, simultanément $R_1$ représente un atome d'hydrogène, peut être soumis si on le désire, à l'action d'une base forte et préférentiellement un alcoolate de métal alcalin pour conduire à un dérivé de formule (I/C) :

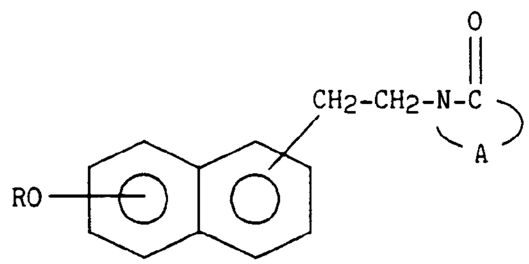

(I/C)

dans laquelle R a la même signification que précédemment et A représente un groupement alkyle linéaire ou ramifié comprenant de 2 à 8 atomes de carbone, cas particulier des dérivés de formule (I) pour lesquels $R_1$ et $R_2$ forment avec NCO un système monocyclique substitué par un groupement oxo, et éventuellement par un ou plusieurs groupements alkyle inférieur,
que l'on purifie, si on le désire, par une technique choisie parmi cristallisation ou chromatographie.

**2.** Procédé de préparation selon la revendication 1 des composés de formule (I/A) :

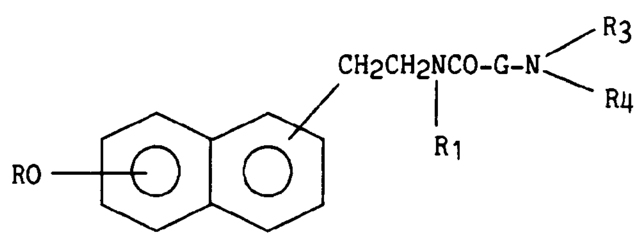

(I/A)

dans laquelle R, $R_1$, $R_3$, $R_4$ et G ont la même signification que dans la formule (I), caractérisé en ce qu'on la traite en dérivé de formule (II) :

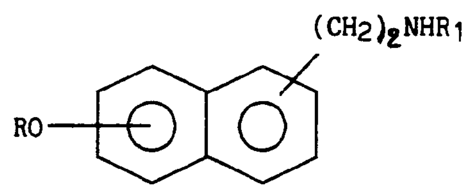

(II)

dans laquelle R et $R_1$ ont la même signification que dans la formule (I), par un dérivé de formule (III) :

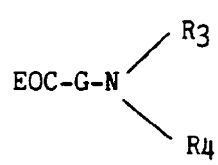

(III)

dans laquelle E signifie un groupe partant choisi parmi hydroxyle, alkoxy inférieur ou halogène, G, $R_3$ et $R_4$ ayant la même signification que dans la formule (I),
éventuellement en présence d'un agent alcalin,
pour conduire à un dérivé de formule (I/A) que l'on purifie, si on le désire, par des techniques classiques telles que chromatographie, cristallisation, dont on sépare le cas échéant les isomères et que l'on sacrifie, si on le désire, par un acide pharmaceutiquement acceptable.

**3.** Procédé de préparation selon la revendication 1 des composés de formule (I/B) :

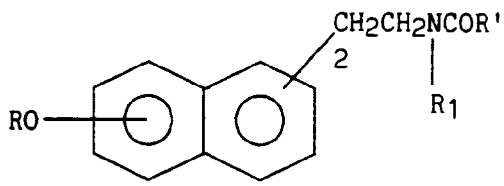

(I/B)

dans laquelle R, $R_1$ ont la même définition que dans la formule (I),
$R_2$ signifiant :

- un groupement alkyle inférieur linéaire ou ramifié ou cycloalkyle éventuellement substitué par un atome d'halogène,
- un groupement aryle ou hétéroaryle ou arylalkyle inférieur éventuellement substitué par un ou plusieurs atomes d'halogène ou groupements choisis parmi alkyle inférieur, alkoxy inférieur, ou trifluorométhyle,

caractérisé en ce que l'on traite un dérivé de formule (II) :

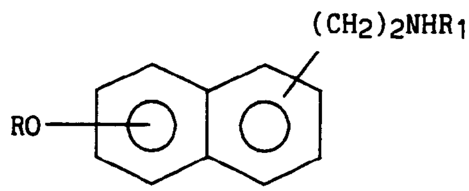

(II)

dans laquelle R et $R_1$ ont la même signification que dans la formule (I),

- par un chlorure d'acide de formule (IV) :

$$Cl\text{-}CO\text{-}R'_2 \qquad \textbf{(IV)}$$

  dans laquelle $R'_2$ a la même définition que précédemment
- ou par l'anhydride d'acide correspondant pour conduire à un dérivé de formule (I/B) que l'on purifie si nécessaire par des techniques classiques telles que chromatographie et/ou cristallisation et dont on sépare les isomères, le cas échéant.

**4.** Procédé de préparation selon la revendication 1 des composés de formule (I/A) :

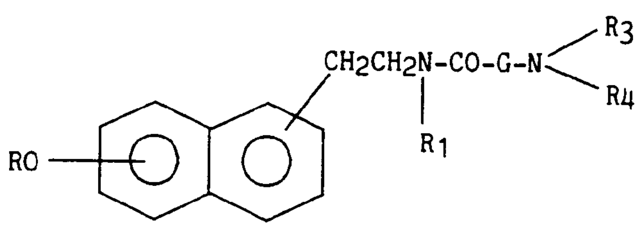

(I/A)

dans laquelle R, $R_1$, $R_3$ et $R_4$ ont la même signification que dans la formule (I), caractérisé en ce que l'on traite un dérivé de formule (II) :

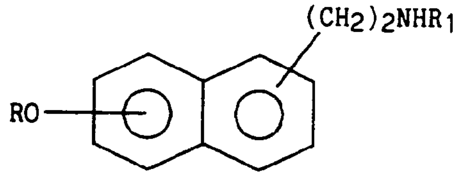

(II)

dans laquelle R et $R_1$ ont la même définition que dans la formule (I),

- par un chlorure d'acide de formule (IV) :

Cl-CO-R'$_2$ **(IV)**

- ou par l'anhydride d'acide correspondant, dans laquelle R'$_2$ signifie un groupement alkyle inférieur linéaire ou ramifié substitué par un atome d'halogène,

pour obtenir un dérivé de formule (I/B) :

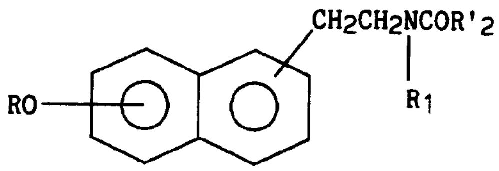

**(I/B)**

dans laquelle R, R$_1$ et R'$_2$ ont la même définition que précédemment, que l'on purifie si nécessaire par des techniques classiques telles que chromatographie et/ou cristallisation, que l'on soumet à l'action d'une amine de formule (V) :

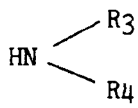

**(V)**

dans laquelle R$_3$ et R$_4$ ont la même définition que précédemment, en excès ou en présence d'une amine tertiaire ou d'un sel de métal alcalin pour conduire à un dérivé de formule (I/A) tel que précédemment défini, qui si on le désire est purifié par une technique classique telle que chromatographie et/ou cristallisation, et/ou salifié par un acide pharmaceutiquement acceptable.

**5.** Procédé de préparation selon la revendication 1 des composés de formule (I/C) :

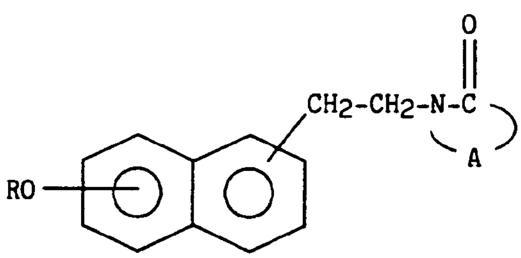

**(I/C)**

dans laquelle R a la même définition que dans la formule (I) et A représente un groupement alkyle linéaire ou ramifié comprenant de 2 à 8 atomes de carbone, caractérisé en ce que l'on traite un dérivé de formule (II) :

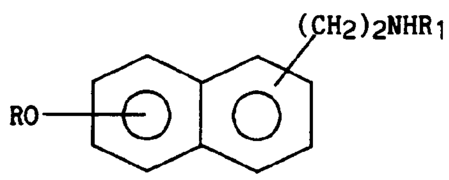

**(II)**

dans laquelle R a la même définition que dans la formule (I),

- par un chlorure d'acide de formule (IV) :

Cl-CO-R'$_2$ **(IV)**

- ou par l'anhydride d'acide correspondant, dans laquelle R'$_2$ signifie un groupement alkyle inférieur linéaire ou ramifié substitué par un atome d'halogène,

pour obtenir un dérivé de formule (I/B) :

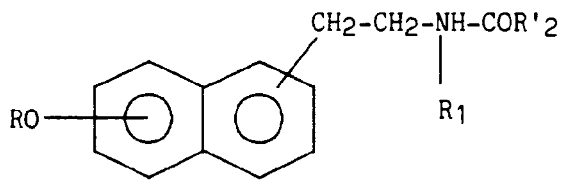

(I/B)

dans laquelle R et R'$_2$ ont la même définition que précédemment,
que l'on purifie si nécessaire par des techniques classiques telles que chromatographie et/ou cristallisation,
que l'on soumet à l'action d'une base forte et préférentiellement un alcoolate de métal alcalin, pour conduire à un dérivé de formule (I/C), que l'on purifie si on le désire par une technique choisie parmi cristallisation ou chromatographie.

**6.** Procédé selon l'une des revendications 1 à 5 de préparation de composés de formule (I) dans laquelle le groupement OR se trouve en position 7, ainsi que, le cas échéant, de leurs isomères, épimères, diastéréoisomères et de leurs sels d'addition à un acide pharmaceutiquement acceptable.

**7.** Procédé selon l'une des revendications 1 à 5 de préparation de composés de formule (I) dans laquelle $R_1$ représente un atome d'hydrogène ou forme avec $R_2$ et le groupement N-CO le système pyrrolidinone-2, ainsi que, le cas échéant, de leurs isomères, épimères, diastéréoisomères et leurs sels d'addition à un acide pharmaceutiquement acceptable.

**8.** Procédé selon la revendication 1 de préparation de dérivés de formule (I) dans laquelle $R_2$ représente :
. un groupement alkyle inférieur linéaire ou ramifié ou cycloalkyle éventuellement substitué par un atome d'halogène,
. un groupement aryle ou hétéroaryle mono ou bicyclique ou arylalkyle inférieur éventuellement substitué par un ou plusieurs atomes d'halogène ou groupements choisis parmi alkyle inférieur, alkoxy inférieur, ou trifluorométhyle,
. un groupement de formule :

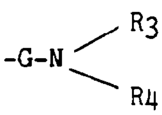

G représentant un groupement alkyle inférieur linéaire ou ramifié,
$R_3$ et $R_4$ forment avec l'atome d'azote auquel ils sont attachés un système hétérocyclique, mono ou bicyclique chaque cycle possédant cinq ou six sommets comprenant éventuellement un autre hétéroatome et éventuellement substitué par un groupement alkyle inférieur ou oxo, aryle ou arylalkyle inférieur, ou aryle substitué ou arylalkyle inférieur substitué étant entendu que le terme substitué affectant les groupements aryle et arylalkyle signifie que ces groupements peuvent être substitués par un ou plusieurs radicaux choisis parmi alkyle inférieur, alkoxy inférieur, trifluorométhyle ou atome d'halogène,
ainsi que, le cas échéant, leurs isomères, épimères, diastéréoisomères et leurs sels d'addition à un acide pharmaceutiquement acceptable.

**9.** Procédé selon l'une des revendications 1 à 6 de préparation de composés de formule (I) dans laquelle le groupement OR se trouve en position 7, $R_1$ représente un atome d'hydrogène et $R_2$ représente un groupement alkyle inférieur linéaire ou ramifié ou cycloalkyle éventuellement substitués par un atome d'halogène ainsi que, le cas échéant, leurs isomères, épimères, diastéréoisomères.

**10.** Procédé selon la revendication 1 de préparation de composés de formule (I) dans laquelle le groupement OR se trouve en position 7, $R_1$ représente un atome d'hydrogène et $R_2$ représente un groupement aryle ou hétéroaryle mono ou bicyclique éventuellement substitué par un ou plusieurs atomes d'halogène ainsi que, le cas échéant, leurs isomères, épimères, diastéréoisomères.

**11.** Procédé de préparation selon la revendication 1 du composé de formule (I) qui est le N-[2-(7-méthoxy napht-1-yl) éthyl] cyclopropylcarboxamide.

**12.** Procédé de préparation selon la revendication 1 du composé de formule (I) qui est le N-[2-(7-méthoxy napht-1-yl) éthyl] (2-oxo pyrrolidin-1-yl) acétamide.

**13.** Procédé de préparation selon la revendication 1 du composé de formule (I) qui est le N-[2-(7-méthoxy napht-1-yl) éthyl] pyrrolidin-2-one.

**14.** Procédé de préparation selon la revendication 1 du composé de formule (I) qui est le N-[2-(7-méthoxy napht-1-yl) éthyl] acétamide.

**15.** Procédé de préparation selon la revendication 1 du composé de formule (I) qui est le N-[2-(7-méthoxy napht-1-yl) éthyl] isobutyramide.

**16.** Procédé de préparation selon la revendication 1 du composé de formule (I) qui est le N-[2-(7-méthoxy napht-1-yl) éthyl] butyramide.

**17.** Procédé de préparation selon la revendication 1 du composé de formule (I) qui est le N-[2-(7-méthoxy napht-1-yl) éthyl] propionamide.

**18.** Procédé de préparation selon la revendication 1 du composé de formule (I) qui est qui est le N-[2-(7-méthoxy napht-1-yl) éthyl] 4-chlorobutyramide.

**19.** Procédé de préparation selon la revendication 1 du composé de formule (I) qui est le N-[2-(7-méthoxy napht-1-yl) éthyl]-2-morpholinoacétamide.

**20.** Procédé de préparation selon la revendication 1 du composé de formule (I) qui est le N-[2-(7-méthoxy napht-1-yl) éthyl]-2-{4-[(2,3,4-triméthoxyphényl)méthyl] pipérazin-1-yl} acétamide ainsi que ses sels d'addition à un acide pharmaceutiquement acceptable.

**21.** Procédé de préparation selon la revendication 1 du composé de formule (I) qui est le N[2-(7-méthoxy napht-1-yl) éthyl] benzamide.

**22.** Procédé de préparation selon la revendication 1 du composé de formule (I) qui est le N[2-(7-méthoxy napht-1-yl) éthyl]-3,5-dichloro-benzamide.

**23.** Procédé de préparation selon la revendication 1 du composé de formule (I) qui est le N[2-(7-méthoxy napht-1-yl) éthyl] (indol-2-yl) carboxamide.

**24.** Procédé de préparation selon la revendication 1 du composé de formule (I) qui est le N[2-(7-méthoxy napht-1-yl) éthyl] pentanamide.

**25.** Procédé de préparation selon la revendication 1 du composé de formule (I) qui est le N[2-(7-méthoxy napht-1-yl) éthyl]cyclobutane carboxamide.

**26.** Procédé de préparation selon la revendication 1 du composé de formule (I) qui est le N[2-(7-méthoxy napht-1-yl) éthyl] 2-iodoacétamide.

**27.** Procédé de préparation selon la revendication 1 du composé de formule (I) qui est le N[2-(7-méthoxy napht-1-yl) éthyl] 2-bromoacétamide.

**28.** Procédé de préparation selon la revendication 1 du composé de formule (I) qui est le N[2-(7-méthoxy napht-1-yl) éthyl]2-chloroacétamide.

**29.** Procédé de préparation de compositions pharmaceutiques contenant comme principe actif au moins un composé selon l'une des revendications 11 à 28, en combinaison avec un ou plusieurs excipients ou véhicules inertes non-toxiques, pharmaceutiquement acceptables.

**30.** Procédé de préparation de composition pharmaceutique selon la revendication 29 utile dans le traitement des troubles du système mélatoninergique et notamment du stress, de l'anxiété, des dépressions saisonnières, des insomnies et fatigues dues aux décalages horaires, schizophrénies, attaque de panique, mélancolie, de la régulation de l'appétit, de l'insomnie, des troubles psychotiques, de l'épilepsie, de la maladie de Parkinson, de la démence sénile, des divers désordres liés au vieillissement normal ou pathologique, pertes de mémoire, maladie d'Alzheimer, ainsi que les troubles de la circulation cérébrale, de certains cancers, du psoriasis, de l'acné, de la séborrhée ou en tant qu'inhibiteur de l'ovulation ou en médecine vétérinaire dans les troubles du pelage.

## Patentansprüche

### Patentansprüche für folgende Vertragsstaaten : AT, BE, CH, DE, DK, FR, GB, IT, LI, LU, NL, SE

1. Verbindungen der allgemeinen Formel (I)

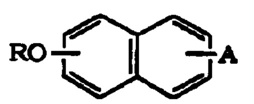

(I)

in der

A eine Gruppe der Formel

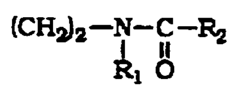

R eine geradkettige oder verzweigte niedrigmolekulare Alkylgruppe,

$R_1$ ein Wasserstoffatom oder eine geradkettige oder verzweigte niedrigmolekulare Alkylgruppe,

$R_2$

. ein Wasserstoffatom,

. eine geradkettige oder verzweigte niedrigmolekulare Alkylgruppe oder Cycloalkylgruppe, die gegebenenfalls durch ein Halogenatom substituiert ist,

. eine Arylgruppe, eine Heteroarylgruppe, eine Arylniedrigalkylgruppe, eine substituierte Arylgruppe, eine substituierte Heteroarylgruppe oder eine substituierte Arylalkylgruppe, wobei unter einer Heteroarylgruppe eine monocyclische oder bicyclische ungesättigte Gruppe mit 1 bis 3 Heteroatomen ausgewählt aus Stickstoffatomen, Sauerstoffatomen oder Schwefelatomen, wobei jeder Ring vier oder fünf Ringglieder aufweist, zu verstehen ist und unter einer Arylgruppe eine Phenylgruppe oder eine Naphthylgruppe zu verstehen ist,

. eine gegebenenfalls reduzierte und/oder durch eine Oxogruppe substituierte Imidazolylgruppe oder

. eine Gruppe der Formel

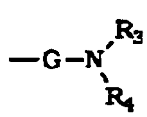

in der G eine geradkettige oder verzweigte niedrigmolekulare Alkylgruppe darstellt,

$R_3$ und $R_4$, die gleichartig oder verschieden sein können, jeweils eine niedrigmolekulare Alkylgruppe, ein Wasserstoffatom, eine Phenylgruppe oder eine Phenylniedrigalkylgruppe oder $R_3$und $R_4$ gemeinsam mit dem Stickstoffatom, an das sie gebunden sind, ein aromatisches oder nichtaromatisches, monocyclisches oder bicyclisches heterocyclisches System, wobei jeder Ring fünf oder sechs Ringglieder aufweist und gegebenenfalls ein weiteres Heteroatom enthält und gegebenenfalls durch eine oder mehrere Niedrigalkylgruppen, Oxogruppen, Arylgruppen oder Arylniedrigalkylgruppen oder substituierte Arylgruppen oder substituierte Arylniedrigalkylgruppen substituiert ist, bedeuten,

wobei der Ausdruck substituiert bezüglich der Arylgruppen, Arylalkylgruppen oder Heteroarylgruppen bei der Definition von $R_2$, $R_3$ und $R_4$ bedeutet, daß diese Gruppen durch

eine oder mehrere aus Niedrigalkylgruppen, Niedrigalkoxygruppen, Trifluormethylgruppen oder Halogenatomen ausgewählte Reste substituiert sind.
oder worin $R_1$ zusammen mit $R_2$ und der Gruppe N-CO ein heterocyclisches System der Formel

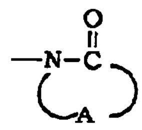

bildet, worin A eine geradkettige oder verzweigte Alkylgruppe mit 2 bis 8 Kohlenstoffatomen darstellt,
sowie gegebenenfalls deren Isomeren, Epimeren, Diastereoisomeren und Additionssalze mit einer pharmazeutisch annehmbaren Säure,
wobei die Begriffe Niedrigalkyl und Niedrigalkoxy bedeuten, daß die Gruppen 1 bis 6 Kohlenstoffatome aufweisen und der Begriff Cycloalkyl bedeutet, daß die Gruppen 3 bis 8 Kohlenstoffatome umfassen.

**2.** Verbindungen nach Anspruch 1, worin die Gruppe OR in der 7-Stellung steht, sowie gegebenenfalls die Isomeren, Epimeren, Diastereoisomeren und die Additionssalze dieser Verbindungen mit einer pharmazeutisch annehmbaren Säure.

**3.** Verbindungen nach Anspruch 1, worin $R_1$ ein Wasserstoffatom bedeutet oder zusammen mit $R_2$ und der Gruppe N-CO das Pyrrolidin-2-on-System bildet, sowie gegebenenfalls die Isomeren, Epimeren, Diastereoisomeren und Additionssalze dieser Verbindungen mit einer pharmazeutisch annehmbaren Säure.

**4.** Verbindungen nach Anspruch 1, worin $R_2$
. eine geradkettige oder verzweigte Niedrigalkylgruppe oder Cycloalkylgruppe, die gegebenenfalls durch ein Halogenatom substituiert ist,
. eine monocyclische oder bicyclische Arylgruppe oder Heteroarylgruppe oder eine Arylniedrigalkylgruppe, die gegebenenfalls durch eines oder mehrere Halogenatome oder Gruppen ausgewählt aus Niedrigalkylgruppen, Niedrigalkoxygruppen oder Trifluormethylgruppen substituiert ist, oder
. eine Gruppe der Formel

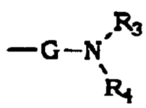

worin G eine geradkettige oder verzweigte niedrigmolekulare Alkylgruppe darstellt und
$R_3$ und $R_4$ gemeinsam mit dem Stickstoffatom, an das sie gebunden sind, ein monocyclisches oder bicyclisches heterocyclisches System, wobei jeder Ring fünf oder sechs Ringglieder aufweist und gegebenenfalls ein weiteres Heteroatom umfaßt sowie gegebenenfalls durch ein niedrigmolekulare Alkylgruppe, eine Oxogruppe, eine Arylgruppe, eine Arylniedrigalkylgruppe, eine substituierte Arylgruppe oder eine substituierte Arylniedrigalkylgruppe substituiert ist, bedeuten, wobei der auf die Arylgruppen und die Arylalkylgruppen bezogene Begriff substituiert bedeutet, daß diese Gruppen durch eine oder mehrere aus niedrigmolekularen Alkylgruppen, niedrigmolekularen Alkoxygruppen, Trifluormethylgruppen oder Halogenatomen ausgewählte Reste substituiert sein können,
sowie gegebenenfalls deren Isomeren, Epimeren, Diastereoisomeren und Additionssalze mit einer pharmazeutisch annehmbaren Säure.

**5.** Verbindungen nach einem der Ansprüche 1 oder 2, worin die Gruppe OR in der 7-Stellung steht, $R_1$ ein Wasserstoffatom und $R_2$ eine geradkettige oder verzweigte niedrigmolekulare Alkylgruppe oder Cycloalkylgruppe, die gegebenenfalls durch ein Halogenatom substituiert ist, bedeuten, sowei gegebenenfalls deren Isomeren, Epimeren und Diastereoisomeren.

**6.** Verbindungen nach Anspruch 1, worin die Gruppe OR in der 7-Stellung steht, $R_1$ ein Wasserstoffatom und $R_2$ eine monocyclische oder bicyclische Arylgruppe oder Heteroarylgruppe, die gegebenenfalls durch eines oder mehrere Halogenatome substituiert ist, bedeuten, sowie gegebenenfalls deren Isomeren, Epimeren und Diastereoisomeren.

7. Verbindung nach Anspruch 1, nämlich N-[2-(7-Methoxy-naphth-1-yl)-ethyl]-cyclopropylcarboxamid.

8. Verbindung nach Anspruch 1, nämlich N-[2-(7-Methoxy-naphth-1-yl)-ethyl]-(2-oxo-pyrrolidin-1-yl)-acetamid.

9. Verbindung nach Anspruch 1, nämlich N-[2-(7-Methoxy-naphth-1-yl)-ethyl]-pyrrolidin-2-on.

10. Verbindung nach Anspruch 1, nämlich N-[2-(7-Methoxy-naphth-1-yl)-ethyl]-acetamid.

11. Verbindung nach Anspruch 1, nämlich N-[2-(7-Methoxy-naphth-1-yl)-ethyl]-isobutyramid.

12. Verbindung nach Anspruch 1, nämlich N-[2-(7-Methoxy-naphth-1-yl)-ethyl]-butyramid.

13. Verbindung nach Anspruch 1, nämlich N-[2-(7-Methoxy-naphth-1-yl)-ethyl]-propionamid.

14. Verbindung nach Anspruch 1, nämlich N-[2-(7-Methoxy-naphth-1-yl)-ethyl]-4-chlorbutyramid.

15. Verbindung nach Anspruch 1, nämlich N-[2-(7-Methoxy-naphth-1-yl)-ethyl]-2-morpholinoacetamid.

16. Verbindung nach Anspruch 1, nämlich N-[2-(7-Methoxy-naphth-1-yl)-ethyl]-2-{4-[2,3,4-trimethoxyphenyl)-methyl]-piperazin-1-yl}-acetamid sowie dessen Additionssalze mit einer pharmazeutisch annehmbaren Säure.

17. Verbindung nach Anspruch 1, nämlich N-[2-(7-Methoxy-naphth-1-yl)-ethyl]-benzamid.

18. Verbindung nach Anspruch 1, nämlich N-[2-(7-Methoxy-naphth-1-yl)-ethyl]-3,5-dichlorbenzamid.

19. Verbindung nach Anspruch 1, nämlich N-[2-(7-Methoxy-naphth-1-yl)-ethyl]-(indol-2-yl)-carboxamid.

20. Verbindung nach Anspruch 1, nämlich N-[2-(7-Methoxy-naphth-1-yl)-ethyl]-pentanamid.

21. Verbindung nach Anspruch 1, nämlich N-[2-(7-Methoxy-naphth-1-yl)-ethyl]-cyclobutan-carboxamid.

22. Verbindung nach Anspruch 1, nämlich N-[2-(7-Methoxy-naphth-1-yl)-ethyl]-2-iodacetamid.

23. Verbindung nach Anspruch 1, nämlich N-[2-(7-Methoxy-naphth-1-yl)-ethyl]-2-bromacetamid.

24. Verbindung nach Anspruch 1, nämlich N-[2-(7-Methoxy-naphth-1-yl)-ethyl]-2-chloracetamid.

25. Verfahren zur Herstellung der Verbindungen der Formel (I) nach Anspruch 1, **dadurch gekennzeichnet**, daß man als Ausgangsmaterial ein Derivat der Formel (II):

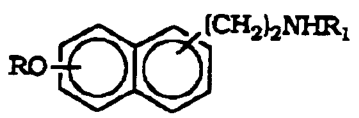

(II)

worin R und $R_1$ die gleichen Bedeutungen besitzen, wie sie für die Formel (I) angegeben sind, verwendet, welches man

- entweder mit einem Derivat der Formel (III):

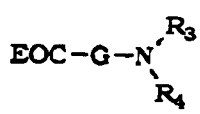

(III)

in der E eine aus Hydroxylgruppen, Niedrigalkoxygruppen oder Halogenatomen ausgewählte austretende Gruppe darstellt und G, $R_3$ und $R_4$ die gleichen Bedeutungen besitzen, wie sie für die Formel (I) angegeben sind.

gegenbenenfalls in Gegenwart eines alkalischen Mittels umsetzt zur Bildung eines Derivats der Formel (I/A), einem Sonderfall der Derivate der Formel (I):

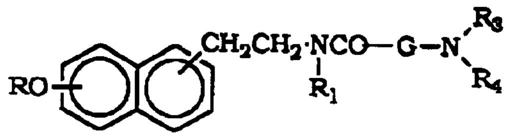

(I/A)

in der R, $R_1$, $R_3$, $R_4$ und G die oben angegebenen Bedeutungen besitzen, und $R_2$ hier eine Gruppe der Formel:

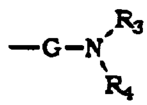

darstellt,
welches man gewünschtenfalls mit Hilfe klassischer Methoden, wie der Chromatographie oder der Kristallisation, reinigt und gewünschtenfalls mit einer pharmazeutisch annehmbaren Säure in ein Salz überführt,
- oder mit einem Säurechlorid der Formel (IV):

$$Cl\text{-}CO\text{-}R'_2 \qquad \textbf{(IV)}$$

oder einem entsprechenden Säureanhydrid, worin
$R'_2$:

- eine geradkettige oder verzweigte niedrigmolekulare Alkylgruppe oder Cycloalkylgruppe, die gegebenenfalls durch ein Halogenatom substituiert ist,
- eine Arylgruppe, Heteroarylgruppe oder Arylniedrigalkylgruppe, die gegebenenfalls durch eines oder mehrere Halogenatome oder aus niedrigmolekularen Alkylgruppen, niedrigmolekularen Alkoxygruppen oder Trifluormethylgruppen ausgewählte Gruppen substituiert ist, oder
- eine gegebenenfalls reduzierte und/oder durch eine Oxogruppe substituierte Imidazolylgruppe bedeutet,

behandelt zur Bildung eines Derivats der Formel (I/B):

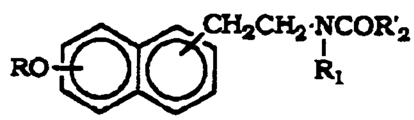

(I/B)

in der R, $R_1$ und $R'_2$ die oben angegebenen Bedeutungen besitzen, welches man erforderlichenfalls mit Hilfe klassischer Methoden, wie der Chromatographie und/oder der Kristallisation, reinigt,
welches Derivat der Formel (I/B) dann, wenn $R'_2$ eine geradkettige oder verzweigte oder durch ein Halogenatom substituierte niedrigmolekulare Alkylgruppe darstellt, gewünschtenfalls der Einwirkung eines Amins der Formel (V)

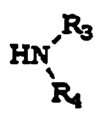

(V)

worin $R_3$ und $R_4$ die oben angegebenen Bedeutungen besitzen, im Überschuß oder in Gegenwart eines tertiären Amins oder eines Alkalimetallsalzes unterworfen werden kann zur Bildung eines Derivats der Formel (I/A), wie sie oben definiert ist, welches man gewünschtenfalls mit Hilfe einer klassischen Methode, wie der Chromatographie und/oder der Kristallisation, reinigen und/oder mit einer pharmazeutisch annehmbaren Säure in ein Salz überführen kann, oder welches Derivat der Formel (I/B) dann, wenn $R'_2$ einen

geradkettigen oder verzweigten Alkylsubstituenten mit mindestens 2 Kohlenstoffatomen darstellt und durch ein Halogenatom substituiert ist, und dann, wenn gleichzeitig $R_1$ ein Wasserstoffatom darstellt, gewünschtenfalls der Einwirkung einer starken Base und vorzugsweise eines Alkalimetallalkoholats unterworfen werden kann zur Bildung eines Derivats der Formel (I/C):

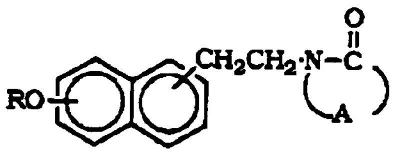

(I/C)

in der R die oben angegebenen Bedeutungen besitzt und A eine geradkettige oder verzweigte Alkylgruppe mit 2 bis 8 Kohlenstoffatomen darstellt, d.h. eines Sonderfalls der Derivate der Formel (I), in der $R_1$ und $R_2$ gemeinsam mit NCO ein durch eine Oxogruppe substituiertes und gegebenenfalls durch eine oder mehrere niedrigmolekulare Alkylgruppen substituiertes monocyclisches System bilden,
welches man gewünschtenfalls mit einer aus der Kristallisation und der Chromatographie ausgewählten Methode reinigt.

**26.** Verfahren nach Anspruch 25 zur Herstellung der Verbindungen der Formel (I/A):

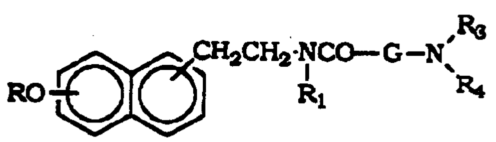

(I/A)

in der R, $R_1$, $R_3$, $R_4$ und G die gleichen Bedeutungen wie bezüglich der Formel (I) angegeben besitzen, **dadurch gekennzeichnet**, daß man ein Derivat der Formel (II):

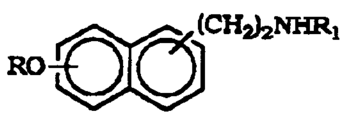

(II)

in der R und $R_1$ die gleichen Bedeutungen wie bezüglich der Formel (I) angegeben besitzen, mit einem Derivat der Formel (III):

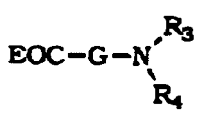

(III)

in der E eine aus Hydroxylgruppen, niedrigmolekularen Alkoxygruppen oder Halogenatomen ausgewählte austretende Gruppe bedeutet und G, $R_3$ und $R_4$ die bezüglich der Formel (I) angegebenen Bedeutungen besitzen,
gegebenenfalls in Gegenwart eines alkalischen Mittels behandelt zur Bildung eines Derivats der Formel (I/A), welches man gewünschtenfalls durch klassische Methoden, wie Chromatographie oder Kristallisation, reinigt und welches man gegebenenfalls in die Isomeren auftrennt und gewünschtenfalls mit einer pharmel (I/A):

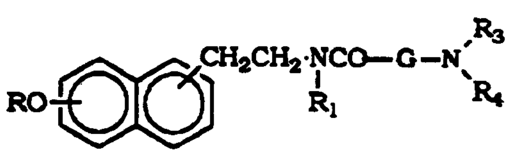

(I/A)

in der R, $R_1$, $R_3$ und $R_4$ die bezüglich der Formel (I) angegebenen Bedeutungen besitzen, **dadurch ge-**

**kennzeichnet**, daß man ein Derivat der Formel (II):

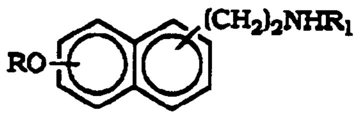

(II)

in der R und $R_1$ die bezüglich der Formel (I) angegebenen Bedeutungen besitzen,

- mit einem Säurechlorid der Formel (IV):

Cl-CO-R'$_2$ **(IV)**

- oder dem entsprechenden Säureanhydrid, worin R'$_2$ eine geradkettige oder verzweigte, mit einem Halogenatom substituierte niedrigmolekulare Alkylgruppe bedeutet,

behandelt zur Bildung eines Derivats der Formel (I/B):

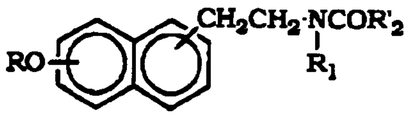

(I/B)

in der R, $R_1$ und R'$_2$ die oben angegebenen Bedeutungen besitzen, welches man erforderlichenfalls mit klassischen Methoden, wie der Chromatographie und/oder der Kristallisation, reinigt,
welches man der Einwirkung eines Amins der Formel (V):

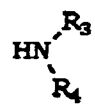

(V)

in der $R_3$ und $R_4$ die oben angegebenen Bedeutungen besitzen,
mazeutisch annehmbaren Säure in ein Salz überführt.

**27.** Verfahren nach Anspruch 25 zur Herstellung der Verbindungen der Formel (I/B):

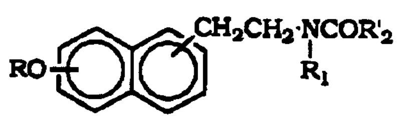

(I/B)

in der R und $R_1$ die bezüglich der Formel (I) angegebenen Bedeutungen besitzen und
$R_2$:

- eine geradkettige oder verzweigte niedrigmolekulare Alkylgruppe oder Cycloalkylgruppe, die gegebenenfalls durch ein Halogenatom substituiert ist, oder
- eine Arylgruppe, Heteroarylgruppe oder Arylniedrigalkylgruppe, die gegebenenfalls durch eines oder mehrere Halogenatome oder aus niedrigmolekularen Alkylgruppen, niedrigmolekularen Alkoxygruppen oder Trifluormethylgruppen ausgewählte Gruppen substituiert ist, bedeutet,

**dadurch gekennzeichnet,** daß man ein Derivat der Formel (II):

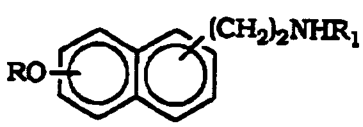

(II)

in der R und $R_1$ die bezüglich der Formel (I) angegebenen Bedeutungen besitzen,

- mit einem Säurechlorid der Formel (IV):

Cl-CO-R'$_2$ **(IV)**

in der $R'_2$ die oben angegebenen Bedeutungen besitzt,

- oder mit dem entsprechenden Säureanhydrid behandelt zur Bildung eines Derivats der Formel (I/B), welches man erforderlichenfalls mit klassischen Methoden, wie die Chromatographie und/oder die Kristallisation, reinigt und gegebenenfalls in die Isomeren auftrennt.

**28.** Verfahren nach Anspruch 25 zur Herstellung der Verbindungen der Forim Überschuß oder in Gegenwart eines tertiären Amins oder eines Alkalimetallsalzes unterwirft zur Bildung eines Derivats der Formel (I/A) der oben definierten Art, welches man gewünschtenfalls mit einer klassischen Methode, wie der Chromatographie und/oder der Kristallisation, reinigt und/oder mit einer pharmazeutisch annehmbaren Säure in ein Salz überführt.

**29.** Verfahren nach Anspruch 25 zur Herstellung der Verbindungen der Formel (I/C):

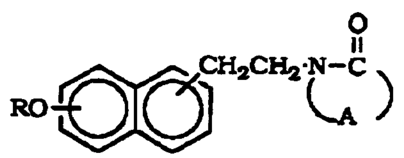

(I/C)

in der R die bezüglich der Formel (I) angegebenen Bedeutungen besitzt und A eine geradkettige oder verzweigte Alkylgruppe mit 2 bis 8 Kohlenstoffatomen darstellt,
**dadurch gekennzeichnet**, daß man ein Derivat der Formel (II):

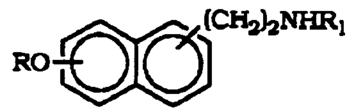

(II)

in der R die bezüglich der Formel (I) angegebenen Bedeutungen besitzt,

- mit einem Säurechlorid der Formel (IV):

$$Cl\text{-}CO\text{-}R'_2 \qquad (IV)$$

- oder dem entsprechenden Säureanhydrid, worin $R'_2$ eine geradkettige oder verzweigte, durch ein Halogenatom substituierte niedrigmolekulare Alkylgruppe darstellt,

behandelt zur Bildung eines Derivats der Formel (I/B):

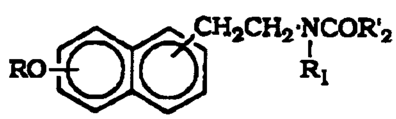

(I/B)

in der R und $R'_2$ die oben angegebenen Bedeutungen besitzen,
welches man erforderlichenfalls mit klassichen Methoden, wie der Chromatographie und/oder der Kristallisation, reinigt und der Einwirkung einer starken Base und vorzugsweise eines Alkalimetallalkoholats unterwirft zur Bildung eines Derivats der Formel (I/C), welches man gewünschtenfalls mit einer aus Kristallisation und Chromatographie ausgewählten Methode reinigt.

**30.** Pharmazeutische Zubereitung enthaltend als Wirkstoff mindestens eine Verbindung nach einem der Ansprüche 1 bis 24 in Kombination mit einem oder mehreren inerten, nicht-toxischen, pharmazeutisch annehmbaren Bindemitteln oder Trägematerialien.

**31.** Pharmazeutische Zubereitung nach Anspruch 30 zur Behandlung von Störungen des melatoninergischen Systems und insbesondere von Streß, Angstzuständen, jahreszeitlichen Depressionen, der Schlaflosigkeit und der Müdigkeit als Folge von Zeitverschiebungen, der Schizophrenie, von Panikanfällen, der Melancholie, zur Steuerung des Appetits, zur Behandlung des Schlaflosigkeit, von psychotischen Störungen, der Epilepsie, der Parkinson-Krankheit, des Altersirrsinns, verschiedener Störungen, die mit dem normalen oder pathologischen Altern verbunden sind, Gedächtnisverlusten, der Alzheimer'schen Krankheit sowie von Störungen der Gehirndurchblutung, bestimmter Krebse, des Psoriasis, der Akne, der Seborrhoe

und als Inhibitor der Ovulation oder in der Veterinärmedizin zur Behandlung von Störungen des Haarkleides.

**Patentansprüche für folgenden Vertragsstaat: ES**

1. Verfahren zur Herstellung von Verbindungen der allgemeinen Formel (I)

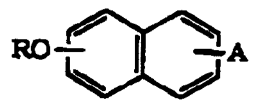

(I)

in der

A eine Gruppe der Formel

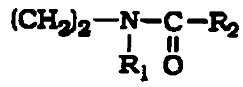

R eine geradkettige oder verzweigte niedrigmolekulare Alkylgruppe,

$R_1$ ein Wasserstoffatom oder eine geradkettige oder verzweigte niedrigmolekulare Alkylgruppe,

$R_2$

. ein Wasserstoffatom,
. eine geradkettige oder verzweigte niedrigmolekulare Alkylgruppe oder Cycloalkylgruppe, die gegebenenfalls durch ein Halogenatom substituiert ist,
. eine Arylgruppe, eine Heteroarylgruppe, eine Arylniedrigalkylgruppe, eine substituierte Arylgruppe, eine substituierte Heteroarylgruppe oder eine substituierte Arylalkylgruppe, wobei unter einer Heteroarylgruppe eine monocyclische oder bicyclische ungesättigte Gruppe mit 1 bis 3 Heteroatomen ausgewählt aus Stickstoffatomen, Sauerstoffatomen oder Schwefelatomen, wobei jeder Ring vier oder fünf Ringglieder aufweist, zu verstehen ist und unter einer Arylgruppe eine Phenylgruppe oder eine Naphthylgruppe zu verstehen ist,
. eine gegebenenfalls reduzierte und/oder durch eine Oxogruppe substituierte Imidazolylgruppe oder
. eine Gruppe der Formel

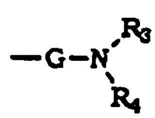

in der G eine geradkettige oder verzweigte niedrigmolekulare Alkylgruppe darstellt,

$R_3$ und $R_4$, die gleichartig oder verschieden sein können, jeweils eine niedrigmolekulare Alkylgruppe, ein Wasserstoffatom, eine Phenylgruppe oder eine Phenylniedrigalkylgruppe oder $R_3$ und $R_4$ gemeinsam mit dem Stickstoffatom, an das sie gebunden sind, ein aromatisches oder nichtaromatisches, monocyclisches oder bicyclisches heterocyclisches System, wobei jeder Ring fünf oder sechs Ringglieder aufweist und gegebenenfalls ein weiteres Heteroatom enthält und gegebenenfalls durch eine oder mehrere Niedrigalkylgruppen, Oxogruppen, Arylgruppen oder Arylniedrigalkylgruppen oder substituierte Arylgruppen oder substituierte Arylniedrigalkylgruppen substituiert ist, bedeuten,

wobei der Ausdruck substituiert bezüglich der Arylgruppen, Arylalkylgruppen oder Heteroarylgruppen bei der Definition von $R_2$, $R_3$ und $R_4$ bedeutet, daß diese Gruppen durch eine oder mehrere aus Niedrigalkylgruppen, Niedrigalkoxygruppen, Trifluormethylgruppen oder Halogenatomen ausgesuchte Reste substituiert sind, oder worin $R_1$ zusammen mit $R_2$ und der Gruppe N-CO ein heterocyclisches System der Formel

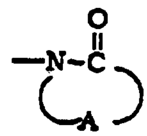

bildet, worin A eine geradkettige oder verzweigte Alkylgruppe mit 2 bis 8 Kohlenstoffatomen darstellt, sowie gegebenenfalls deren Isomeren, Epimeren, Diastereoisomeren und Additionssalze mit einer pharmazeutisch annehmbaren Säure,

wobei die Begriffe Niedrigalkyl und Niedrigalkoxy bedeuten, daß die Gruppen 1 bis 6 Kohlenstoffatome aufweisen und der Begriff Cycloalkyl bedeutet, daß die Gruppen 3 bis 8 Kohlenstoffatome umfassen,
**dadurch gekennzeichnet**, daß man als Ausgangsmaterial ein Derivat der Formel (II):

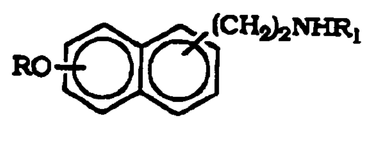
(II)

worin R und $R_1$ die gleichen Bedeutungen besitzen, wie sie für die Formel (I) angegeben sind, verwendet, welches man
- entweder mit einem Derivat der Formel (III):

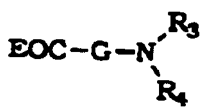
(III)

in der E eine aus Hydroxylgruppen, Niedrigalkoxygruppen oder Halogenatomen ausgewählte austretende Gruppe darstellt und G, $R_3$ und $R_4$ die gleichen Bedeutungen besitzen, wie sie für die Formel (I) angegeben sind,
gegebenenfalls in Gegenwart eines alkalischen Mittels umsetzt zur Bildung eines Derivats der Formel (I/A), einem Sonderfall der Derivate der Formel (I):

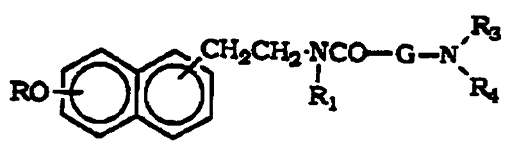
(I/A)

in der R, $R_1$, $R_3$, $R_4$ und G die oben angegebenen Bedeutungen besitzen, und $R_2$ hier eine Gruppe der Formel:

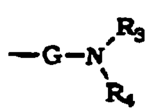

darstellt,
welches man gewünschtenfalls mit Hilfe klassischer Methoden, wie der Chromatographie oder der Kristallisation, reinigt und gewünschtenfalls mit einer pharmazeutisch annehmbaren Säure in ein Salz überführt,
- oder mit einem Säurechlorid der Formel (IV):

$$Cl\text{-}CO\text{-}R'_2 \qquad (IV)$$

oder einem entsprechenden Säureanhydrid, worin
$R'_2$:

- eine geradkettige oder verzweigte niedrigmolekulare Alkylgruppe oder Cycloalkylgruppe, die gegebenenfalls durch ein Halogenatom substituiert ist,
- eine Arylgruppe, Heteroarylgruppe oder Arylniedrigalkylgruppe, die gegebenenfalls durch eines oder mehrere Halogenatome oder aus niedrigmolekularen Alkylgruppen, niedrigmolekularen Alkoxygruppen oder Trifluormethylgruppen ausgewählte Gruppen substituiert ist, oder

- eine gegebenenfalls reduzierte und/oder durch eine Oxogruppe substituierte Imidazolylgruppe bedeutet,

behandelt zur Bildung eines Derivats der Formel (I/B):

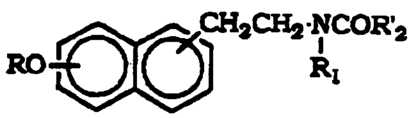

(I/B)

in der R, $R_1$ und $R'_2$ die oben angegebenen Bedeutungen besitzen, welches man erforderlichenfalls mit Hilfe klassischer Methoden, wie der Chromatographie und/oder der Kristallisation, reinigt, welches Derivat der Formel (I/B) dann, wenn $R'_2$ eine geradkettige oder verzweigte oder durch ein Halogenatom substituierte niedrigmolekulare Alkylgruppe darstellt, gewünschtenfalls der Einwirkung eines Amins der Formel (V)

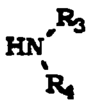

(V)

worin $R_3$ und $R_4$ die oben angegebenen Bedeutungen besitzen, im Überschuß oder in Gegenwart eines tertiären Amins oder eines Alkalimetallsalzes unterworfen werden kann zur Bildung eines Derivats der Formel (I/A), wie sie oben definiert ist, welches man gewünschtenfalls mit Hilfe einer klassischen Methode, wie der Chromatographie und/oder der Kristallisation, reinigen und/oder mit einer pharmazeutisch annehmbaren Säure in ein Salz überführen kann, oder welches Derivat der Formel (I/B) dann, wenn $R'_2$ einen geradkettigen oder verzweigten Alkylsubstituenten mit mindestens 2 Kohlenstoffatomen darstellt und durch ein Halogenatom substituiert ist, und dann, wenn gleichzeitig $R_1$ ein Wasserstoffatom darstellt, gewünschtenfalls der Einwirkung einer starken Base und vorzugsweise eines Alkalimetallalkoholats unterworfen werden kann zur Bildung eines Derivats der Formel (I/C):

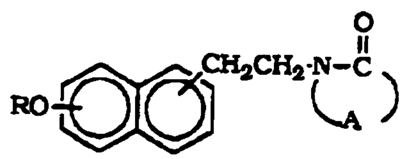

(I/C)

in der R die oben angegebenen Bedeutungen besitzt und A eine geradkettige oder verzweigte Alkylgruppe mit 2 bis 8 Kohlenstoffatomen darstellt, d.h. eines Sonderfalls der Derivate der Formel (I), in der $R_1$ und $R_2$ gemeinsam mit NCO ein durch eine Oxogruppe substituiertes und gegebenenfalls durch eine oder mehrere niedrigmolekulare Alkylgruppen substituiertes monocyclisches System bilden,
welches man gewünschtenfalls mit einer aus der Kristallisation und der Chromatographie ausgewählten Methode reinigt.

**2.** Verfahren nach Anspruch 1 zur Herstellung der Verbindungen der Formel (I/A):

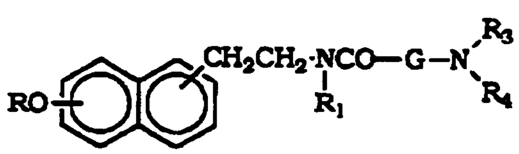

(I/A)

in der R, $R_1$, $R_3$, $R_4$ und G die gleichen Bedeutungen wie bezüglich der Formel (I) angegeben besitzen, **dadurch gekennzeichnet**, daß man ein Derivat der Formel (II):

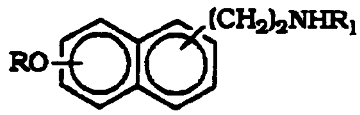

(II)

in der R und $R_1$ die gleichen Bedeutungen wie bezüglich der Formel (I) angegeben besitzen, mit einem Derivat der Formel (III):

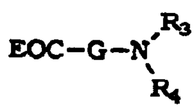

(III)

in der E eine aus Hydroxylgruppen, niedrigmolekularen Alkoxygruppen oder Halogenatomen ausgewählte austretende Gruppe bedeutet und G, $R_3$ und $R_4$ die bezüglich der Formel (I) angegebenen Bedeutungen besitzen,
gegebenenfalls in Gegenwart eines alkalischen Mittels behandelt zur Bildung eines Derivats der Formel (I/A), welches man gewünschtenfalls durch klassische Methoden, wie Chromatographie oder Kristallisation, reinigt und welches man gegebenenfalls in die Isomeren auftrennt und gewünschtenfalls mit einer pharmazeutisch annehmbaren Säure in ein Salz überführt.

**3.** Verfahren nach Anspruch 1 zur Herstellung der Verbindungen der Formel (I/B):

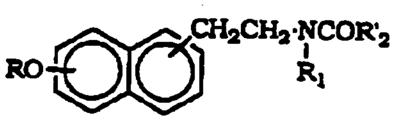

(I/B)

in der R und $R_1$ die bezüglich der Formel (I) angegebenen Bedeutungen besitzen
und
$R_2$:
- eine geradkettige oder verzweigte niedrigmolekulare Alkylgruppe oder Cycloalkylgruppe, die gegebenenfalls durch ein Halogenatom substituiert ist, oder
- eine Arylgruppe, Heteroarylgruppe oder Arylniedrigalkylgruppe, die gegebenenfalls durch eines oder mehrere Halogenatome oder aus niedrigmolekularen Alkylgruppen, niedrigmolekularen Alkoxygruppen oder Trifluormethylgruppen ausgewählte Gruppen substituiert ist, bedeutet,

**dadurch gekennzeichnet**, daß man ein Derivat der Formel (II):

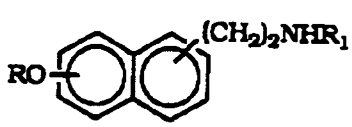

(II)

in der R und $R_1$ die bezüglich der Formel (I) angegebenen Bedeutungen besitzen,
- mit einem Säurechlorid der Formel (IV):

  $Cl\text{-}CO\text{-}R'_2$ **(IV)**

  in der $R'_2$ die oben angegebenen Bedeutungen besitzt,

- oder mit dem entsprechenden Säureanhydrid behandelt zur Bildung eines Derivats der Formel (I/B), welches man erforderlichenfalls mit klassischen Methoden, wie die Chromatographie und/oder die Kristallisation, reinigt und gegebenenfalls in die Isomeren auftrennt.

**4.** Verfahren nach Anspruch 1 zur Herstellung der Verbindungen der Formel (I/A):

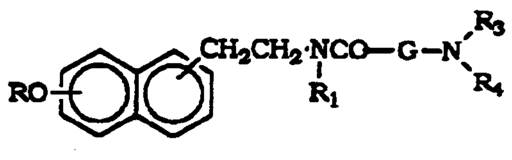

**(I/A)**

in der R, $R_1$, $R_3$ und $R_4$ die bezüglich der Formel (I) angegebenen Bedeutungen besitzen, **dadurch gekennzeichnet**, daß man ein Derivat der Formel (II):

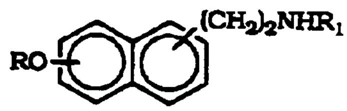

**(II)**

in der R und $R_1$ die bezüglich der Formel (I) angegebenen Bedeutungen besitzen,

- mit einem Säurechlorid der Formel (IV):

  Cl-CO-$'_2$ **(IV)**

- oder dem entsprechenden Säureanhydrid, worin $R'_2$ eine geradkettige oder verzweigte, mit einem Halogenatom substituierte niedrigmolekulare Alkylgruppe bedeutet,

behandelt zur Bildung eines Derivats der Formel (I/B):

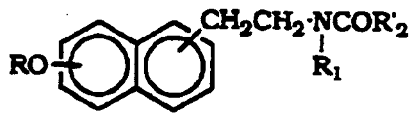

**(I/B)**

in der R, $R_1$ und $R'_2$ die oben angegebenen Bedeutungen besitzen, welches man erforderlichenfalls mit klassischen Methoden, wie der Chromatographie und/oder der Kristallisation, reinigt,
welches man der Einwirkung eines Amins der Formel (V):

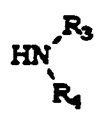

**(V)**

in der $R_3$ und $R_4$ die oben angegebenen Bedeutungen besitzen,
im Überschuß oder in Gegenwart eines tertiären Amins oder eines Alkalimetallsalzes unterwirft zur Bildung eines Derivats der Formel (I/A) der oben definierten Art, welches man gewünschtenfalls mit einer klassischen Methode, wie der Chromatographie und/oder der Kristallisation, reinigt und/oder mit einer pharmazeutisch annehmbaren Säure in ein Salz überführt.

**5.** Verfahren nach Anspruch 1 zur Herstellung der Verbindungen der Formel (I/C):

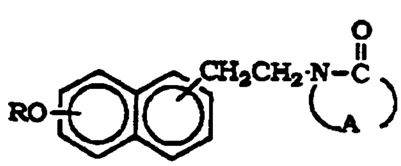

**(I/C)**

in der R die bezüglich der Formel (I) angegebenen Bedeutungen besitzt und A eine geradkettige oder verzweigte Alkylgruppe mit 2 bis 8 Kohlenstoffatomen darstellt,
**dadurch gekennzeichnet**, daß man ein Derivat der Formel (II):

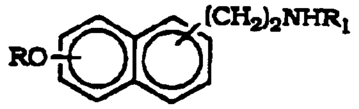

(II)

in der R die bezüglich der Formel (I) angegebenen Bedeutungen besitzt,

- mit einem Säurechlorid der Formel (IV):

$$Cl\text{-}CO\text{-}R'_2 \quad \textbf{(IV)}$$

- oder dem entsprechenden Säureanhydrid, worin $R'_2$ eine geradkettige oder verzweigte, durch ein Halogenatom substituierte niedrigmolekulare Alkylgruppe darstellt,

behandelt zur Bildung eines Derivats der Formel (I/B):

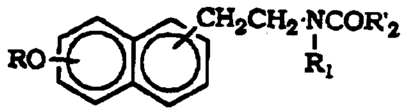

(I/B)

in der R und $R'_2$ die oben angegebenen Bedeutungen besitzen,
welches man erforderlichenfalls mit klassichen Methoden, wie der Chromatographie und/oder der Kristallisation, reinigt und der Einwirkung einer starken Base und vorzugsweise eines Alkalimetallalkoholats unterwirft zur Bildung eines Derivats der Formel (I/C), welches man gewünschtenfalls mit einer aus Kristallisation und Chromatographie ausgewählten Methode reinigt.

**6.** Verfahren nach einem der Ansprüche 1 bis 5 zur Herstellung der Verbindungen der Formel (I), in der die Gruppe OR in der 7-Stellung steht, sowie gegebenenfalls ihrer Isomeren, Epimeren, Diastereoisomeren und deren Additionssalze mit einer pharmazeutisch annehmbaren Säure.

**7.** Verfahren nach einem der Ansprüche 1 bis 5 zur Herstellung der Verbindungen der Formel (I), in der $R_1$ ein Wasserstoffatom darstellt oder zusammen mit $R_2$ und der Gruppe N-CO das Pyrrolidin-2-on-System bildet, sowie gegebenenfalls ihrer Isomeren, Epimeren, Diastereoisomeren und Additionssalze mit einer pharmazeutisch annehmbaren Säure.

**8.** Verfahren nach Anspruch 1 zur Herstellung von Derivaten der Formel (I), in der $R_2$:

. eine geradkettige oder verzweigte Niedrigalkylgruppe oder Cycloalkylgruppe, die gegebenenfalls durch ein Halogenatom substituiert ist,

. eine monocyclische oder bicyclische Arylgruppe oder Heteroarylgruppe oder eine Arylniedrigalkylgruppe, die gegebenenfalls durch eines oder mehrere Halogenatome oder Gruppen ausgewählt aus Niedrigalkylgruppen, Niedrigalkoxygruppen oder Trifluormethylgruppen substituiert ist, oder

. eine Gruppe der Formel

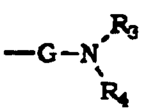

worin G eine geradkettige oder verzweigte niedrigmolekulare Alkylgruppe darstellt und $R_3$ und $R_4$ gemeinsam mit dem Stickstoffatom, an das sie gebunden sind, ein monocyclisches oder bicyclisches heterocyclisches System, wobei jeder Ring fünf oder sechs Ringglieder aufweist und gegebenenfalls ein weiteres Heteroatom umfaßt sowie gegebenenfalls durch eine niedrigmolekulare Alkylgruppe, eine Oxogruppe, eine Arylgruppe, eine Arylniedrigalkylgruppe, eine substituierte Arylgruppe oder eine substituierte Arylniedrigalkylgruppe substituiert ist, wobei der auf die Arylgruppen und die Arylalkylgruppen bezogene Begriff substituiert bedeutet, daß diese Gruppen durch eine oder mehrere aus niedrigmolekularen Alkylgruppen, niedrigmolekularen Alkoxygruppen, Trifluormethylgruppen oder Halogenatomen ausgewählte Reste substituiert sein können, sowie gegebenenfalls deren Isomeren, Epimeren, Diastereoisomeren und Additionssalze mit einer pharmazeutisch annehmbaren Säure.

9. Verfahren nach einem der Ansprüche 1 bis 6 zur Herstellung von Verbindungen der Formel (I), in der die Gruppe OR in der 7-Stellung steht und $R_1$ ein Wasserstoffatom und $R_2$ eine geradkettige oder verzweigte niedrigmolekulare Alkylgruppe oder Cycloalkylgruppe, die gegebenenfalls durch ein Halogenatom substituiert ist, bedeuten, sowie gegebenenfalls ihrer Isomeren, Epimeren und Diastereoisomeren.

10. Verfahren nach Anspruch 1 zur Herstellung der Verbindungen der Formel (I), in der die Gruppe OR in der 7-Stellung steht und $R_1$ ein Wasserstoffatom und $R_2$ eine monocyclische oder bicyclische, gegebenenfalls durch eines oder mehrere Halogenatome substituierte Arylgruppe oder Heteroarylgruppe bedeuten, sowie gegebenenfalls ihrer Isomeren, Epimeren und Diastereoisomeren.

11. Verfahren nach Anspruch 1 zur Herstellung einer Verbindung der Formel (I), nämlich N-[2-(7-Methoxy-naphth-1-yl)-ethyl]-cyclopropylcarboxamid.

12. Verfahren nach Anspruch 1 zur Herstellung einer Verbindung der Formel (I), nämlich N-[2-(7-Methoxy-naphth-1-yl)-ethyl]-(2-oxo-pyrrolidin-1-yl)-acetamid.

13. Verfahren nach Anspruch 1 zur Herstellung einer Verbindung der Formel (I), nämlich N-[2(7-Methoxy-naphth-1-yl)-ethyl]-pyrrolidin-2-on.

14. Verfahren nach Anspruch 1 zur Herstellung einer Verbindung der Formel (I), nämlich N-[2-(7-Methoxy-naphth-1-yl)-ethyl]-acetamid.

15. Verfahren nach Anspruch 1 zur Herstellung einer Verbindung der Formel (I), nämlich N-[2(7-Methoxy-naphth-1-yl)-ethyl]-isobutyramid.

16. Verfahren nach Anspruch 1 zur Herstellung einer Verbindung der Formel (I), nämlich N-[2-(7-Methoxy-naphth-1-yl)-ethyl]-butyramid.

17. Verfahren nach Anspruch 1 zur Herstellung einer Verbindung der Formel (I), nämlich N-[2-(7-Methoxy-naphth-1-yl)-ethyl]-propionamid.

18. Verfahren nach Anspruch 1 zur Herstellung einer Verbindung der Formel (I), nämlich N-[2-(7-Methoxy-naphth-1-yl)-ethyl]-4-chlorbutyramid.

19. Verfahren nach Anspruch 1 zur Herstellung einer Verbindung der Formel (I), nämlich N-[2-(7-Methoxy-naphth-1-yl)-ethyl)-2-morpholinoacetamid.

20. Verfahren nach Anspruch 1 zur Herstellung einer Verbindung der Formel (I), nämlich N-[2-(7-Methoxy-naphth-1-yl)-ethyl]-2-{4-[2,3,4-trimethoxyphenyl)-methyl]-piperazin-1-yl}-acetamid sowie dessen Additionssalze mit einer pharmazeutisch annehmbaren Säure.

21. Verfahren nach Anspruch 1 zur Herstellung einer Verbindung der Formel (I), nämlich N-[2-(7-Methoxy-naphth-1-yl)-ethyl]-benzamid.

22. Verfahren nach Anspruch 1 zur Herstellung einer Verbindung der Formel (I), nämlich N-[2-(7-Methoxy-naphth-1-yl)-ethyl]-3,5-dichlorbenzamid.

23. Verfahren nach Anspruch 1 zur Herstellung einer Verbindung der Formel (I), nämlich N-[2-(7-Methoxy-naphth-1-yl)-ethyl]-(indol-2-yl)-carboxamid.

24. Verfahren nach Anspruch 1 zur Herstellung einer Verbindung der Formel (I), nämlich N-[2-(7-Methoxy-naphth-1-yl)-ethyl]-pentanamid.

25. Verfahren nach Anspruch 1 zur Herstellung einer Verbindung der Formel (I), nämlich N-[2-(7-Methoxy-naphth-1-yl)-ethyl]-cyclobutan-carboxamid.

26. Verfahren nach Anspruch 1 zur Herstellung einer Verbindung der Formel (I), nämlich N-[2-(7-Methoxy-naphth-1-yl)-ethyl]-2-iodacetamid.

27. Verfahren nach Anspruch 1 zur Herstellung einer Verbindung der Formel (I), nämlich N-[2-(7-Methoxy-naphth-1-yl)-ethyl]-2-bromacetamid.

**28.** Verfahren nach Anspruch 1 zur Herstellung einer Verbindung der Formel (I), nämlich N-[2-(7-Methoxy-naphth-1-yl)-ethyl]-2-chloracetamid.

**29.** Verfahren zur Herstellung von pharmazeutischen Zubereitungen enthaltend als Wirkstoff mindestens eine Verbindung nach einem der Ansprüche 11 bis 28 in Kombination mit einem oder mehreren inerten, nicht-toxischen, pharmazeutisch annehmbaren Trägermaterialien oder Bindemitteln.

**30.** Verfahren zur Herstellung von pharmazeutischen Zubereitungen nach Anspruch 29, die zur Behandlung von Störungen des melatoninergischen Systems und insbesondere von Streß, Angstzuständen, jahreszeitlichen Depressionen, der Schlaflosigkeit und der Müdigkeit als Folge von Zeiterschiebungen, der Schizophrenie, von Panikanfällen, der Melancholie, zur Steuerung des Appetits, zur Behandlung der Schlaflosigkeit, von psychotischen Störungen, der Epilepsie, der Parkinson-Krankheit, des Altersirrsinns, verschiedener Störungen, die mit dem normalen oder pathologischen Altern verbunden sind, Gedächtnisverlusten, der Alzheimer'schen Kranheit sowie von Störungen der Gehirndurchblutung, bestimmter Krebse, der Psoriasis, der Akne, der Seborrhoe und als Inhibitor der Ovulation oder in der Veterinärmedizin zur Behandlung von Störungen des Haarkleides, geeignet ist.

**Patentansprüche für folgenden Vertragsstaat: GR**

**1.** Verfahren zur Herstellung von Verbindungen der allgemeinen Formel (I)

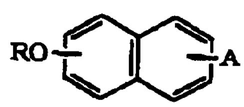

(I)

in der

A eine Gruppe der Formel

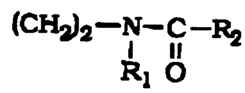

R eine geradkettige oder verzweigte niedrigmolekulare Alkylgruppe,

$R_1$ ein Wasserstoffatom oder eine geradkettige oder verzweigte niedrigmolekulare Alkylgruppe,

$R_2$ ein Wasserstoffatom,

. eine geradkettige oder verzweigte niedrigmolekulare Alkylgruppe oder Cycloalkylgruppe, die gegebenenfalls durch ein Halogenatom substituiert ist,

. eine Arylgruppe, eine Heteroarylgruppe, eine Arylniedrigalkylgruppe, eine substituierte Arylgruppe, eine substituierte Heteroarylgruppe oder eine substituierte Arylalkylgruppe, wobei unter einer Heteroarylgruppe eine monocyclische oder bicyclische ungesättigte Gruppe mit 1 bis 3 Heteroatomen ausgewählt aus Stickstoffatomen, Sauerstoffatomen oder Schwefelatomen, wobei jeder Ring vier oder fünf Ringglieder aufweist, zu verstehen ist und unter einer Arylgruppe eine Phenylgruppe oder eine Naphthylgruppe zu verstehen ist,

. eine gegebenenfalls reduzierte und/oder durch eine Oxogruppe substituierte Imidazolylgruppe oder

. eine Gruppe der Formel

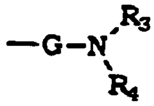

in der G eine geradkettige oder verzweigte niedrigmolekulare Alkylgruppe darstellt,

$R_3$ und $R_4$, die gleichartig oder verschieden sein können, jeweils eine niedrigmolekulare Alkylgruppe, ein Wasserstoffatom, eine Phenylgruppe oder eine Phenylniedrigalkylgruppe oder $R_3$ und $R_4$ gemeinsam mit dem Stickstoffatom, an das sie gebunden sind, ein aromatisches oder

nichtaromatisches, monocyclisches oder bicyclisches heterocyclisches System, wobei jeder Ring fünf oder sechs Ringglieder aufweist und gegebenenfalls ein weiteres Heteroatom enthält und gegebenenfalls durch eine oder mehrere Niedrigalkylgruppen, Oxogruppen, Arylgruppen oder Arylniedrigalkylgruppen oder substituierte Arylgruppen oder substituierte Arylniedrigalkylgruppen substituiert ist, bedeuten,
wobei des Ausdruck substituiert bezüglich der Arylgruppen, Arylalkylgruppen oder Heteroarylgruppen bei der Definition von $R_2$, $R_3$ und $R_4$ bedeutet, daß diese Gruppen durch eine oder mehrere aus Niedrigalkylgruppen, Niedrigalkoxygruppen, Trifluormethylgruppen oder Halogenatomen ausgesuchte Reste substituiert sind,

oder worin $R_1$ zusammen mit $R_2$ und der Gruppe N-CO ein heterocyclisches System der Formel

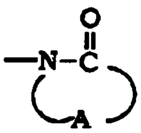

bildet, worin A eine geradkettige oder verzweigte Alkylgruppe mit 2 bis 8 Kohlenstoffatomen darstellt,

sowie gegebenenfalls deren Isomeren, Epimeren, Diastereoisomeren und Additionssalze mit einer pharmazeutisch annehmbaren Säure,
wobei die Begriffe Niedrigalkyl und Niedrigalkoxy bedeuten, daß die Gruppen 1 bis 6 Kohlenstoffatome aufweisen und der Begriff Cycloalkyl bedeutet, daß die Gruppen 3 bis 8 Kohlenstoffatome umfassen,
**dadurch gekennzeichnet**, daß man als Ausgangsmaterial ein Derivat der Formel (II):

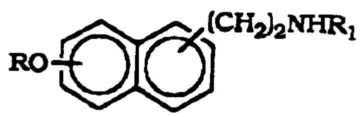

(II)

worin R und $R_1$ die gleichen Bedeutungen besitzen, wie sie für die Formel (I) angegeben sind, verwendet, welches man
- entweder mit einem Derivat der Formel (III):

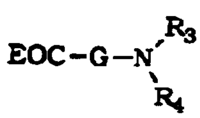

(III)

in der E eine aus Hydroxylgruppen, Niedrigalkoxygruppen oder Halogenatomen ausgewählte austretende Gruppe darstellt und G, $R_3$ und $R_4$ die gleichen Bedeutungen besitzen, wie sie für die Formel (I) angegeben sind,
gegebenenfalls in Gegenwart eines alkalischen Mittels umsetzt zur Bildung eines Derivats der Formel (I/A), einem Sonderfall der Derivate der Formel (I):

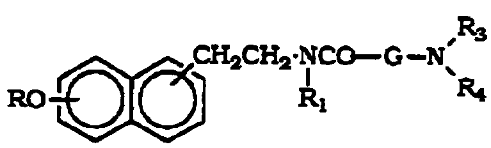

(I/A)

in der R, $R_1$, $R_3$, $R_4$ und G die oben angegebenen Bedeutungen besitzen, und $R_2$ hier eine Gruppe der Formel:

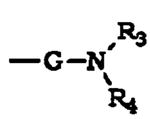

darstellt,
welches man gewünschtenfalls mit Hilfe klassischer Methoden, wie der Chromatographie oder der Kristallisation, reinigt und gewünschtenfalls mit einer pharmazeutisch annehmbaren Säure in ein Salz überführt,
- oder mit einem Säurechlorid der Formel (IV):

$$Cl\text{-}CO\text{-}R'_2 \qquad (IV)$$

oder einem entsprechenden Säureanhydrid, worin
$R'_2$:

- eine geradkettige oder verzweigte niedrigmolekulare Alkylgruppe oder Cycloalkylgruppe, die gegebenenfalls durch ein Halogenatom substituiert ist,
- eine Arylgruppe, Heteroarylgruppe oder Arylniedrigalkylgruppe, die gegebenenfalls durch eines oder mehrere Halogenatome oder aus niedrigmolekularen Alkylgruppen, niedrigmolekularen Alkoxygruppen oder Trifluormethylgruppen ausgewählte Gruppen substituiert ist, oder
- eine gegebenenfalls reduzierte und/oder durch eine Oxogruppe substituierte Imidazolylgruppe bedeutet,

behandelt zur Bildung eines Derivats der Formel (I/B):

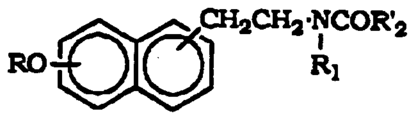 (I/B)

in der R, $R_1$ und $R'_2$ die oben angegebenen Bedeutungen besitzen, welches man erforderlichenfalls mit Hilfe klassischer Methoden, wie der Chromatographie und/oder der Kristallisation, reinigt,
welches Derivat der Formel (I/B) dann, wenn $R'_2$ eine geradkettige oder verzweigte oder durch ein Halogenatom substituierte niedrigmolekulare Alkylgruppe darstellt, gewünschtenfalls der Einwirkung eines Amins der Formel (V)

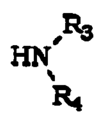 (V)

worin $R_3$ und $R_4$ die oben angegebenen Bedeutungen besitzen, im Überschuß oder in Gegenwart eines tertiären Amins oder eines Alkalimetallsalzes unterworfen werden kann zur Bildung eines Derivats der Formel (I/A), wie sie oben definiert ist, welches man gewünschtenfalls mit Hilfe einer klassischen Methode, wie der Chromatographie und/oder der Kristallisation, reinigen und/oder mit einer pharmazeutisch annehmbaren Säure in ein Salz überführen kann, oder welches Derivat der Formel (I/B) dann, wenn $R'_2$ einen geradkettigen oder verzweigten Alkylsubstituenten mit mindestens 2 Kohlenstoffatomen darstellt und durch ein Halogenatom substituiert ist, und dann, wenn gleichzeitig $R_1$ ein Wasserstoffatom darstellt, gewünschtenfalls der Einwirkung einer starken Base und vorzugsweise eines Alkalimetallalkoholats unterworfen werden kann zur Bildung eines Derivats der Formel (I/C):

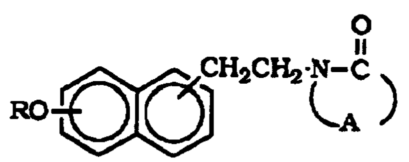 (I/C)

in der R die oben angegebenen Bedeutungen besitzt und A eine geradkettige oder verzweigte Alkylgruppe mit 2 bis 8 Kohlenstoffatomen darstellt, d.h. eines Sonderfalls der Derivate der Formel (I), in der $R_1$ und $R_2$ gemeinsam mit NCO ein durch eine Oxogruppe substituiertes und gegebenenfalls durch eine oder mehrere niedrigmolekulare Alkylgruppen substituiertes monocyclisches System bilden,
welches man gewünschtenfalls mit einer aus der Kristallisation und der Chromatographie ausgewählten Methode reinigt.

2. Verfahren nach Anspruch 1 zur Herstellung der Verbindungen der Formel (I/A):

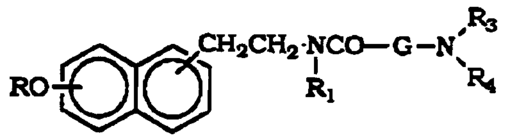

(I/A)

in der R, $R_1$, $R_3$, $R_4$ und G die gleichen Bedeutungen wie bezüglich der Formel (I) angegebenen besitzen, **dadurch gekennzeichnet**, daß man ein Derivat der Formel (II):

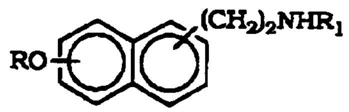

(II)

in der R und $R_1$ die gleichen Bedeutungen wie bezüglich der Formel (I) angegeben besitzen, mit einem Derivat der Formel (III):

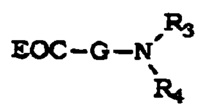

(III)

in der E eine aus Hydroxylgruppen, niedrigmolekularen Alkoxygruppen oder Halogenatomen ausgewählte austretende Gruppe bedeutet und G, $R_3$ und $R_4$ die bezüglich der Formel (I) angegebenen Bedeutungen besitzen,
gegebenenfalls in Gegenwart eines alkalischen Mittels behandelt zur Bildung eines Derivats der Formel (I/A), welches man gewünschtenfalls durch klassische Methoden, wie Chromatographie oder Kristallisation, reinigt und welches man gegebenenfalls in die Isomeren auftrennt und gewünschtenfalls mit einer pharmazeutisch annehmbaren Säure in ein Salz überführt.

3. Verfahren nach Anspruch 1 zur Herstellung der Verbindungen der Formel (I/B):

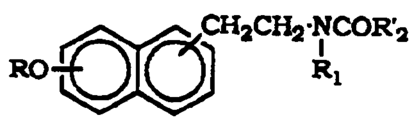

(I/B)

in der R und $R_1$ die bezüglich der Formel (I) angegebenen Bedeutungen besitzen und
$R_2$:

- eine geradkettige oder verzweigte niedrigmolekulare Alkylgruppe oder Cycloalkylgruppe, die gegebenenfalls durch ein Halogenatom substituiert ist, oder
- eine Arylgruppe, Heteroarylgruppe oder Arylniedrigalkylgruppe, die gegebenenfalls durch eines oder mehrere Halogenatome oder aus niedrigmolekularen Alkylgruppen, niedrigmolekularen Alkoxygruppen oder Trifluormethylgruppen ausgewählte Gruppen substituiert ist, bedeutet,

**dadurch gekennzeichnet**, daß man ein Derivat der Formel (II):

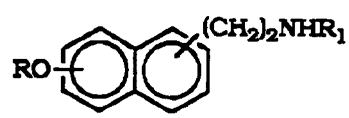

(II)

in der R und $R_1$ die bezüglich der Formel (I) angegebenen Bedeutungen besitzen,

- mit einem Säurechlorid der Formel (IV):

  $$Cl\text{-}CO\text{-}R'_2 \qquad (IV)$$

  in der $R'_2$ die oben angegebenen Bedeutungen besitzt,
- oder mit dem entsprechenden Säureanhydrid behandelt zur Bildung eines Derivats der Formel (I/B),

welches man erforderlichenfalls mit klassischen Methoden, wie die Chromatographie und/oder die Kristallisation, reinigt und gegebenenfalls in die Isomeren auftrennt.

**4.** Verfahren nach Anspruch 1 zur Herstellung der Verbindungen der Formel (I/A):

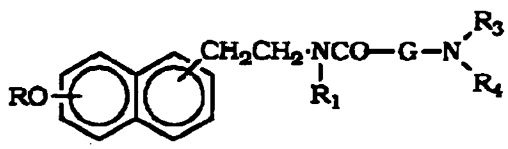

(I/A)

in der R, $R_1$, $R_3$ und $R_4$ die bezüglich der Formel (I) angegebenen Bedeutungen besitzen, **dadurch gekennzeichnet**, daß man ein Derivat der Formel (II):

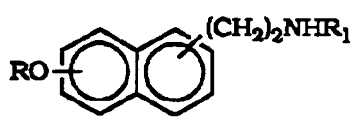

(II)

in der R und $R_1$ die bezüglich der Formel (I) angegebenen Bedeutungen besitzen,

- mit einem Säurechlorid der Formel (IV):

$$Cl\text{-}CO\text{-}R'_2 \quad \textbf{(IV)}$$

- oder dem entsprechenden Säureanhydrid, worin $R'_2$ eine geradkettige oder verzweigte, mit einem Halogenatom substituierte niedrigmolekulare Alkylgruppe bedeutet,

behandelt zur Bildung eines Derivats der Formel (I/B):

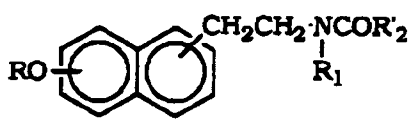

(I/B)

in der R, $R_1$ und $R'_2$ die oben angegebenen Bedeutungen besitzen, welches man erforderlichenfalls mit klassischen Methoden, wie der Chromatographie und/oder der Kristallisation, reinigt,
welches man der Einwirkung eines Amins der Formel (V):

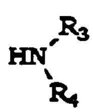

(V)

in der $R_3$ und $R_4$ die oben angegebenen Bedeutungen besitzen,
im Überschuß oder in Gegenwart eines tertiären Amins oder eines Alkalimetallsalzes unterwirft zur Bildung eines Derivats der Formel (I/A) der oben definierten Art, welches man gewünschtenfalls mit einer klassischen Methode, wie der Chromatographie und/oder der Kristallisation, reinigt und/oder mit einer pharmazeutisch annehmbaren Säure in ein Salz überführt.

**5.** Verfahren nach Anspruch 1 zur Herstellung der Verbindungen der Formel (I/C):

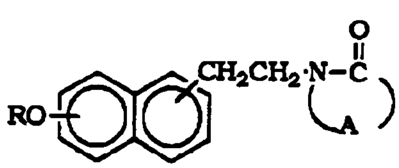

(I/C)

in der R die bezüglich der Formel (I) angegebenen Bedeutungen besitzt und A eine geradkettige oder verzweigte Alkylgruppe mit 2 bis 8 Kohlenstoffatomen darstellt,

**dadurch gekennzeichnet**, daß man ein Derivat der Formel (II):

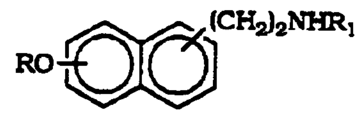

(II)

in der R die bezüglich der Formel (I) angegebenen Bedeutungen besitzt,

- mit einem Säurechlorid der Formel (IV):

  $Cl\text{-}CO\text{-}R'_2$ **(IV)**

- oder dem entsprechenden Säureanhydrid, worin $R'_2$ eine geradkettige oder verzweigte, durch ein Halogenatom substituierte niedrigmolekulare Alkylgruppe darstellt,

behandelt zur Bildung eines Derivats der Formel (I/B):

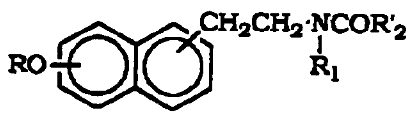

(I/B)

in der R und $R'_2$ die oben angegebenen Bedeutungen besitzen,
welches man erforderlichenfalls mit klassichen Methoden, wie der Chromatographie und/oder der Kristallisation, reinigt und der Einwirkung einer starken Base und vorzugsweise eines Alkalimetallalkoholats unterwirft zur Bildung eines Derivats der Formel (I/C), welches man gewünschtenfalls mit einer aus Kristallisation und Chromatographie ausgewählten Methoden reinigt.

**6.** Verfahren nach einem der Ansprüche 1 bis 5 zur Herstellung der Verbindungen der Formel (I), in der die Gruppe OR in der 7-Stellung steht, sowie gegebenenfalls ihrer Isomeren, Epimeren, Diastereoisomeren und deren Additionssalze mit einer pharmazeutisch annehmbaren Säure.

**7.** Verfahren nach einem der Ansprüche 1 bis 5 zur Herstellung der Verbindungen der Formel (I), in der $R_1$ ein Wasserstoffatom darstellt oder zusammen mit $R_2$ und der Gruppe N-CO das Pyrrolidin-2-on-System bildet, sowie gegebenenfalls ihrer Isomeren, Epimeren, Diastereoisomeren und Additionssalze mit einer pharmazeutisch annehmbaren Säure.

**8.** Verfahren nach Anspruch 1 zur Herstellung von Derivaten der Formel (I), in der $R_2$:

. eine geradkettige oder verzweigte Niedrigalkylgruppe oder Cycloalkylgruppe, die gegebenenfalls durch ein Halogenatom substituiert ist,

. eine monocyclische oder bicyclische Arylgruppe oder Heteroarylgruppe oder eine Arylniedrigalkylgruppe, die gegebenenfalls durch eines oder mehrere Halogenatome oder Gruppen ausgewählt aus Niedrigalkylgruppen, Niedrigalkoxygruppen oder Trifluormethylgruppen substituiert ist, oder

. eine Gruppe der Formel

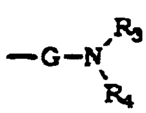

worin G eine geradkettige oder verzweigte niedrigmolekulare Alkylgruppe darstellt und

$R_3$ und $R_4$ gemeinsam mit dem Stickstoffatom, an das sie gebunden sind, ein monocyclisches oder bicyclisches heterocyclisches System, wobei jeder Ring fünf oder sechs Ringglieder aufweist und gegebenenfalls ein weiteres Heteroatom umfaßt sowie gegebenenfalls durch eine niedrigmolekulare Alkylgruppe, eine Oxogruppe, eine Arylgruppe, eine Arylniedrigalkylgruppe, eine substituierte Arylgruppe oder eine substituierte Arylniedrigalkylgruppe substituiert ist, wobei der auf die Arylgruppen und die Arylalkylgruppen bezogene Begriff substituiert bedeutet, daß diese Gruppen durch eine oder mehrere aus niedrigmolekularen Alkylgruppen, niedrigmolekularen Alkoxygruppen, Trifluormethylgruppen oder Halogenatomen ausgewählte Reste substituiert sein können,
sowie gegebenenfalls deren Isomeren, Epimeren, Diastereoisomeren und Additionssalze mit einer pharmazeutisch annehmbaren Säure.

**9.** Verfahren nach einem der Ansprüche 1 bis 6 zur Herstellung von Verbindungen der Formel (I), in der die Gruppe OR in der 7-Stellung steht und $R_1$ ein Wasserstoffatom und $R_2$ eine geradkettige oder verzweigte niedrigmolekulare Alkylgruppe oder Cycloalkylgruppe, die gegebenenfalls durch ein Halogenatom substituiert ist, bedeuten, sowie gegebenenfalls ihrer Isomeren, Epimeren und Diastereoisomeren.

**10.** Verfahren nach Anspruch 1 zur Herstellung der Verbindungen der Formel (I), in der die Gruppe OR in der 7-Stellung steht und $R_1$ ein Wasserstoffatom und $R_2$ eine monocyclische oder bicyclische, gegebenenfalls durch eines oder mehrere Halogenatome substituierte Arylgruppe oder Heteroarylgruppe bedeuten, sowie gegebenenfalls ihrer Isomeren, Epimeren und Diastereoisomeren.

**11.** Verfahren nach Anspruch 1 zur Herstellung einer Verbindung der Formel (I), nämlich N-[2-(7-Methoxy-naphth-1-yl)-ethyl]-cyclopropylcarboxamid.

**12.** Verfahren nach Anspruch 1 zur Herstellung einer Verbindung der Formel (I), nämlich N-[2-(7-Methoxy-naphth-1-yl)-ethyl]-(2-oxo-pyrrolidin-1-yl)-acetamid.

**13.** Verfahren nach Anspruch 1 zur Herstellung einer Verbindung der Formel (I), nämlich N-[2-(7-Methoxy-naphth-1-yl)-ethyl]-pyrrolidin-2-on.

**14.** Verfahren nach Anspruch 1 zur Herstellung einer Verbindung der Formel (I), nämlich N-[2-(7-Methoxy-naphth-1-yl)-ethyl]-acetamid.

**15.** Verfahren nach Anspruch 1 zur Herstellung einer Verbindung der Formel (I), nämlich N-[2-(7-Methoxy-naphth-1-yl)-ethyl]-isobutyramid.

**16.** Verfahren nach Anspruch 1 zur Herstellung einer Verbindung der Formel (I), nämlich N-[2-(7-Methoxy-naphth-1-yl)-ethyl]-butyramid.

**17.** Verfahren nach Anspruch 1 zur Herstellung einer Verbindung der Formel (I), nämlich N-[2-(7-Methoxy-naphth-1-yl)-ethyl]-propionamid.

**18.** Verfahren nach Anspruch 1 zur Herstellung einer Verbindung der Formel (I), nämlich N-[2-(7-Methoxy-naphth-1-yl)-ethyl]-4-chlorbutyramid.

**19.** Verfahren nach Anspruch 1 zur Herstellung einer Verbindung der Formel (I), nämlich N-[2-(7-Methoxy-naphth-1-yl)-ethyl)-2-morpholinoacetamid.

**20.** Verfahren nach Anspruch 1 zur Herstellung einer Verbindung der Formel (I), nämlich N-[2-(7-Methoxy-naphth-1-yl)-ethyl]-2-{4-[2,3,4-trimethoxyphenyl)-methyl]-piperazin-1-yl}-acetamid sowie dessen Additionssalze mit einer pharmazeutisch annehmbaren Säure.

**21.** Verfahren nach Anspruch 1 zur Herstellung einer Verbindung der Formel (I), nämlich N-[2-(7-Methoxy-naphth-1-yl)-ethyl]-benzamid.

**22.** Verfahren nach Anspruch 1 zur Herstellung einer Verbindung der Formel (I), nämlich N-[2-(7-Methoxy-naphth-1-yl)-ethyl]-3,5-dichlorbenzamid.

**23.** Verfahren nach Anspruch 1 zur Herstellung eine Verbindung der Formel (I), nämlich N-[2-(7-Methoxy-naphth-1-yl)-ethyl]-(indol-2-yl)-carboxamid.

**24.** Verfahren nach Anspruch 1 zur Herstellung einer Verbindung der Formel (I), nämlich N-[2-(7-Methoxy-naphth-1-yl)-ethyl]-pentanamid.

**25.** Verfahren nach Anspruch 1 zur Herstellung einer Verbindung der Formel (I), nämlich N-[2-(7-Methoxy-naphth-1-yl)-ethyl]-cyclobutan-carboxamid.

**26.** Verfahren nach Anspruch 1 zur Herstellung einer Verbindung der Formel (I), nämlich N-[2-(7-Methoxy-naphth-1-yl)-ethyl]-2-iodacetamid.

**27.** Verfahren nach Anspruch 1 zur Herstellung einer Verbindung der Formel (I), nämlich N-[2-(7-Methoxy-naphth-1-yl)-ethyl]-2-bromacetamid.

**28.** Verfahren nach Anspruch 1 zur Herstellung einer Verbindung der Formel (I), nämlich N-[2-(7-Methoxy-naphth-1-yl)-ethyl-2-chloracetamid.

**29.** Verfahren zur Herstellung von pharmazeutischen Zubereitungen enthaltend als Wirkstoff mindestens eine Verbindung nach einem der Ansprüche 11 bis 28 in Kombination mit einem oder mehreren inerten, nicht-toxischen, pharmazeutisch annehmbaren Trägermaterialien oder Bindemitteln.

**30.** Verfahren zur Herstellung von pharmazeutischen Zubereitungen nach Anspruch 29, die zur Behandlung von Störungen des melatoninergischen Systems und insbesondere von Streß, Angstzuständen, jahreszeitlichen Depressionen, der Schlaflosigkeit und der Müdigkeit als Folge von Zeiterschiebungen, der Schizophrenie, von Panikanfällen, der Melancholie, zur Steuerung des Appetits, zur Behandlung der Schlaflosigkeit, von psychotischen Störungen, der Epilepsie, der Parkinson-Krankheit, des Altersirrsinns, verschiedener Störungen, die mit dem normalen oder pathologischen Altern verbunden sind, Gedächtnisverlusten, der Alzheimer'schen Krankheit sowie von Störungen der Gehirndurchblutung, bestimmter Krebse, der Psoriasis, der Akne, der Seborrhoe und als Inhibitor der Ovulation oder in der Veterinärmedizin zur Behandlung von Störungen des Haarkleides, geeignet ist.

## Claims

### Claims for the following Contracting States: AT, BE, CH, DE, DK, FR, GB, IT, LI, LU, NL, SE

**1.** Compounds of the general formula (I) :

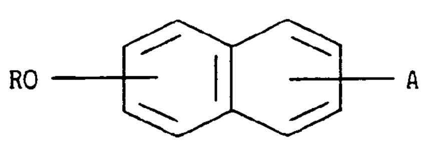

(I)

wherein :

A represents a group

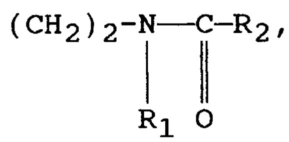

R represents a straight-chain or branched lower alkyl group,

$R_1$ represents a hydrogen atom or a straight-chain or branched lower alkyl group,

$R_2$ represents

- . a hydrogen atom,
- . a straight-chain or branched lower alkyl group or a cycloalkyl group, each of which is optionally substituted by a halogen atom,
- . an aryl or heteroaryl or aryl-lower alkyl group or substituted aryl or substituted heteroaryl or substituted arylalkyl group, there being understood by heteroaryl group an unsaturated mono- or bi-cyclic group comprising from 1 to 3 hetero atoms selected from nitrogen, oxygen and sulphur, each ring comprising 4 or 5 apices, and there being understood by aryl group phenyl or naphthyl,
- . an imidazolyl group that is optionally reduced and/or substituted by an oxo group,
- . a group of formula :

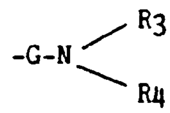

G representing a straight-chain or branched lower alkyl group, and
$R_3$ and $R_4$, which are the same or different, each representing a lower alkyl group or a hydrogen atom or a phenyl or phenyl-lower alkyl group, or $R_3$ and $R_4$, together with the nitrogen atom to which they are attached, forming an aromatic or non-aromatic, mono- or bi-cyclic, heterocyclic system, each ring having five or six apices optionally comprising an additional hetero atom and optionally being substituted by one or more lower alkyl or oxo, aryl or aryl-lower alkyl, or substituted aryl or substituted aryl-lower alkyl groups, the term "substituted" used in relation to aryl and arylalkyl or heteroaryl groups in the definition of $R_2$, $R_3$, $R_4$ indicating that those groups are substituted by one or more radicals selected from lower alkyl, lower alkoxy, trifluoromethyl and halogen,

or $R_1$ forms, with $R_2$ and the group N-CO, a heterocyclic system of the formula :

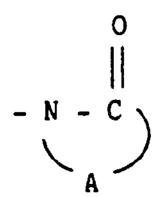

A representing a straight-chain or branched alkyl radical having from 2 to 8 carbon atoms,
and also, where appropriate, their isomers, epimers and diastereoisomers and their addition salts with a pharmaceutically acceptable acid,
the terms "lower alkyl" and "lower alkoxy" indicating groups comprising from 1 to 6 carbon atoms and "cycloalkyl" indicating groups comprising from 3 to 8 carbon atoms.

2. Compounds according to claim 1 wherein the OR group is in the 7-position, and also, where appropriate, their isomers, epimers and diastereoisomers and their addition salts with a pharmaceutically acceptable acid.

3. Compounds according to claim 1 wherein $R_1$ represents a hydrogen atom or forms, with $R_2$ and the group N-CO, the 2-pyrrolidinone system, and also, where appropriate, their isomers, epimers and diastereoisomers and their addition salts with a pharmaceutically acceptable acid.

4. Compounds according to claim 1 wherein $R_2$ represents:
   . a straight-chain or branched lower alkyl group or a cycloalkyl group, each of which is optionally substituted by a halogen atom,
   . a mono- or bi-cyclic aryl or heteroaryl group or an aryl-lower alkyl group, each of which is optionally substituted by one or more halogen atoms or groups selected from lower alkyl, lower alkoxy and trifluoromethyl,
   . a group of formula :

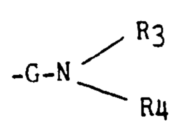

G representing a straight-chain or branched lower alkyl group, and
$R_3$ and $R_4$, together with the nitrogen atom to which they are attached, forming a mono- or bi-cyclic, heterocyclic system, each ring having five or six apices optionally comprising an additional hetero atom and optionally being substituted by a lower alkyl or oxo, aryl or aryl-lower alkyl, or substituted aryl or substituted aryl-lower alkyl group, the term "substituted" used in relation to aryl and arylalkyl groups indicating that those groups may be substituted by one or more radicals selected from lower alkyl, lower alkoxy, trifluoromethyl and halogen, and also, where appropriate, their isomers, epimers and diastereoisomers and

their addition salts with a pharmaceutically acceptable acid.

5. Compounds according to either claim 1 or 2 wherein the group OR is in the 7-position, $R_1$ represents a hydrogen atom and $R_2$ represents a straight-chain or branched lower alkyl group or a cycloalkyl group, each of which is optionally substituted by a halogen atom, and also, where appropriate, their isomers, epimers and diastereoisomers.

6. Compounds according to claim 1 wherein the group OR is in the 7-position, $R_1$ represents a hydrogen atom and $R_2$ represents a mono- or bi-cyclic aryl or heteroaryl group optionally substituted by one or more halogen atoms, and also, where appropriate, their isomers, epimers and diastereoisomers.

7. Compound according to claim 1, which is N-[2-(7-methoxynaphth-1-yl)ethyl]cyclopropylcarboxamide.

8. Compound according to claim 1, which is N-[2-(7-methoxynaphth-1-yl)ethyl](2-oxopyrrolidin-1-yl)acetamide.

9. Compound according to claim 1, which is N-[2-(7-methoxynaphth-1-yl)ethyl]pyrrolidin-2-one.

10. Compound according to claim 1, which is N-[2-(7-methoxynaphth-1-yl)ethyl]acetamide.

11. Compound according to claim 1, which is N-[2-(7-methoxynaphth-1-yl)ethyl]isobutyramide.

12. Compound according to claim 1, which is N-[2-(7-methoxynaphth-1-yl)ethyl]butyramide.

13. Compound according to claim 1, which is N-[2-(7-methoxynaphth-1-yl)ethyl]propionamide.

14. Compound according to claim 1, which is N-[2-(7-methoxynaphth-1-yl)ethyl]-4-chlorobutyramide.

15. Compound according to claim 1, which is N-[2-(7-methoxynaphth-1-yl)ethyl]-2-morpholinoacetamide.

16. Compound according to claim 1, which is N-[2-(7-methoxynaphth-1-yl)ethyl]-2-{4-[(2,3,4-trimethoxyphenyl)methyl]piperazin-1-yl}acetamide, and also the addition salts thereof with a pharmaceutically acceptable acid.

17. Compound according to claim 1, which is N-[2-(7-methoxynaphth-1-yl)ethyl]benzamide.

18. Compound according to claim 1, which is N-[2-(7-methoxynaphth-1-yl)ethyl]-3,5-dichlorobenzamide.

19. Compound according to claim 1, which is N-[2-(7-methoxynaphth-1-yl)ethyl]-(indol-2-yl)carboxamide.

20. Compound according to claim 1, which is N-[2-(7-methoxynaphth-1-yl)ethyl]pentanamide.

21. Compound according to claim 1, which is N-[2-(7-methoxynaphth-1-yl)ethyl]cyclobutane carboxamide.

22. Compound according to claim 1, which is N-[2-(7-methoxynaphth-1-yl)ethyl]-2-iodoacetamide.

23. Compound according to claim 1, which is N-[2-(7-methoxynaphth-1-yl)ethyl]-2-bromoacetamide.

24. Compound according to claim 1, which is N-[2-(7-methoxynaphth-1-yl)ethyl]-2-chloroacetamide.

25. Process for the preparation of compounds of formula (I) according to claim 1, characterised in that there is used as starting material a compound of formula (II) :

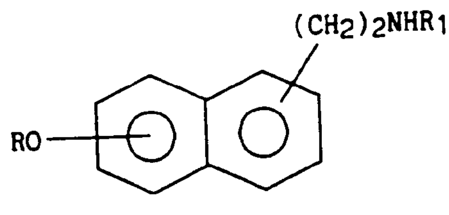

(II)

wherein R and $R_1$ are as defined for formula (I), which is treated :

- either with a compound of formula (III) :

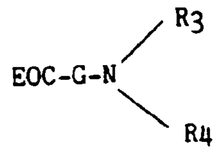

(III)

wherein E denotes a leaving group selected from hydroxy, lower alkoxy and halogen, and G, $R_3$ and $R_4$ are as defined for formula (I),
optionally in the presence of an alkaline agent, to yield a compound of formula (I/A), a particular case of compounds of formula (I) :

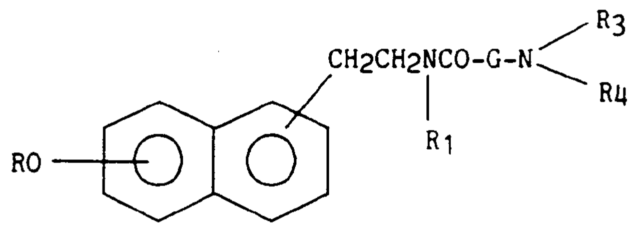

(I/A)

wherein R, $R_1$, $R_3$, $R_4$ and G are as defined hereinbefore, $R_2$ in this case indicating a group :

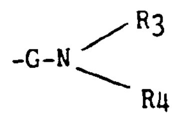

which is purified, if desired, by conventional methods such as chromatography and crystallisation, and which if desired is converted into a salt with a pharmaceutically acceptable acid,
- or with an acid chloride of formula (IV) :

$$Cl\text{-}CO\text{-}R'_2 \qquad (IV)$$

or with the corresponding acid anhydride,
$R'_2$ representing :
- a straight-chain or branched lower alkyl group or a cycloalkyl group, each of which is optionally substituted by a halogen atom,
- an aryl or heteroaryl or aryl-lower alkyl group, each of which is optionally substituted by one or more halogen atoms or groups selected from lower alkyl, lower alkoxy and trifluoromethyl,
- an imidazolyl group that is optionally reduced and/or substituted by an oxo group,

to yield a compound of formula (I/B) :

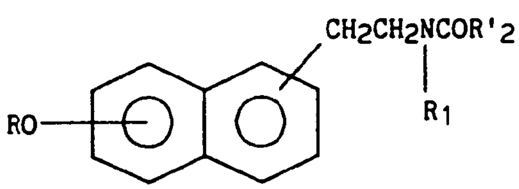

(I/B)

wherein R, $R_1$ and $R'_2$ are as defined hereinbefore, which, if necessary, is purified by conventional methods, such as chromatography and/or crystallisation,
which compound of formula (I/B), when $R'_2$ represents a straight-chain or branched lower alkyl group that is substituted by a halogen atom, may, if desired, be subjected to the action of an amine of formula (V) :

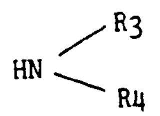

(V)

wherein $R_3$ and $R_4$ are as defined hereinbefore,
in excess or in the presence of a tertiary amine or an alkali metal salt, to yield a compound of formula (I/A) as defined hereinbefore,
which, if desired, is purified by a conventional method, such as chromatography and/or crystallisation, and/or converted into a salt with a pharmaceutically acceptable acid,
which compound of formula (I/B), when $R'_2$ represents a straight-chain or branched alkyl substituent that comprises at least two carbon atoms and is substituted by a halogen atom and when, simultaneously, $R_1$ represents a hydrogen atom, may, if desired, be subjected to the action of a strong base and preferably an alkali metal alcoholate to yield a compound of formula (I/C) :

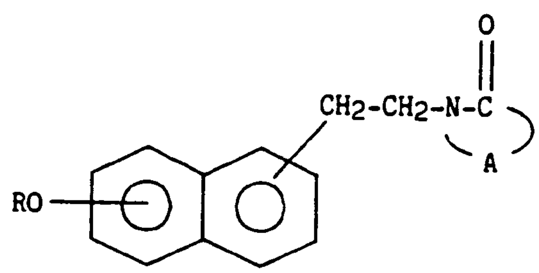

(I/C)

wherein R is as defined hereinbefore and A represents a straight-chain or branched alkyl group comprising from 2 to 8 carbon atoms, a particular case of compounds of formula (I) in which $R_1$ and $R_2$ form with NCO a monocyclic system that is substituted by an oxo group and is optionally substituted by one or more lower alkyl groups, which, if desired, is purified by a method selected from crystallisation and chromatography.

**26.** Process according to claim 25 for the preparation of compounds of formula (I/A) :

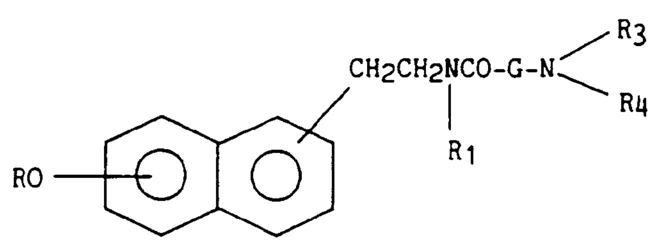

(I/A)

wherein R, $R_1$, $R_3$, $R_4$ and G are as defined for formula (I), characterised in that a compound of formula (II) :

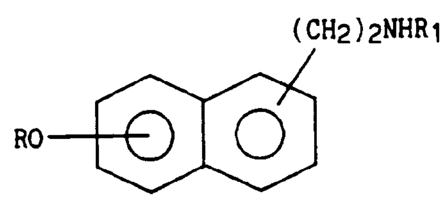

(II)

wherein R and $R_1$ are as defined for formula (I) is treated with a compound of formula (III) :

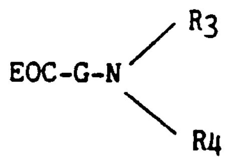

(III)

wherein E represents a leaving group selected from hydroxy, lower alkoxy and halogen, and G, $R_3$ and $R_4$ are as defined for formula (I),
optionally in the presence of an alkaline agent, to yield a compound of formula (I/A) which, if desired, is purified by conventional methods, such as chromatography and crystallisation, of which, where appropriate, the isomers are separated, and which, if desired, is converted into a salt with a pharmaceutically acceptable acid.

**27.** Process according to claim 25 for the preparation of compounds of formula (I/B) :

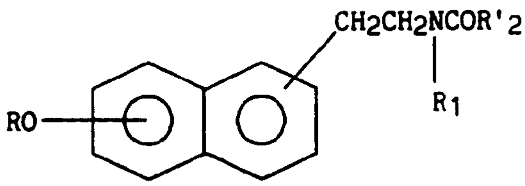

(I/B)

wherein R and $R_1$ are as defined for formula (I), and $R_2$ represents :

- a straight-chain or branched lower alkyl group or a cycloalkyl group, each of which is optionally substituted by a halogen atom,
- an aryl or heteroaryl or aryl-lower alkyl group, each of which is optionally substituted by one or more halogen atoms or groups selected from lower alkyl, lower alkoxy and trifluoromethyl,

characterised in that a compound of formula (II) :

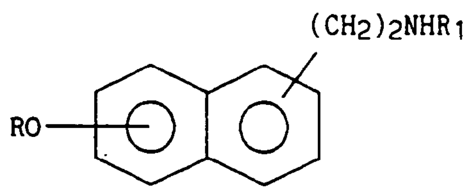

(II)

wherein R and $R_1$ are as defined for formula (I) is treated

- with an acid chloride of formula (IV) :

  $$Cl\text{-}CO\text{-}R'_2 \qquad (IV)$$

  wherein $R'_2$ is as defined above,
- or with the corresponding acid anhydride to yield a compound of formula (I/B) which, if necessary, is purified by conventional methods, such as chromatography and/or crystallisation, and of which, where appropriate, the isomers are separated.

**28.** Process according to claim 25 for the preparation of compounds of formula (I/A) :

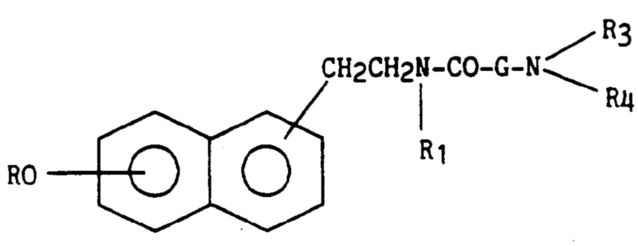

(I/A)

wherein R, $R_1$, $R_3$ and $R_4$ are as defined for formula (I), characterised in that a compound of formula (II) :

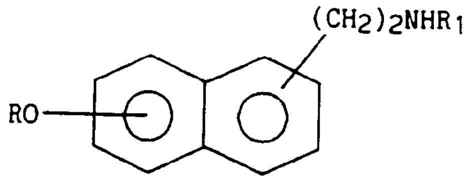

(II)

wherein R and $R_1$ are as defined for formula (I) is treated

- with an acid chloride of formula (IV) :

$$Cl\text{-}CO\text{-}R'_2 \qquad (IV)$$

- or with the corresponding acid anhydride, wherein $R'_2$ represents a straight-chain or branched lower alkyl group that is substituted by a halogen atom,

to yield a compound of formula (I/B) :

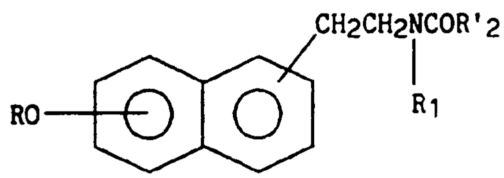

(I/B)

wherein R, $R_1$ and $R'_2$ are as defined hereinbefore, which, if necessary, is purified by conventional methods, such as chromatography and/or crystallisation,
which is subjected to the action of an amine of formula (V) :

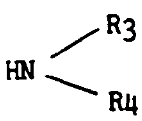

(V)

wherein $R_3$ and $R_4$ are as defined hereinbefore,
in excess or in the presence of a tertiary amine or an alkali metal salt to yield a compound of formula (I/A) as defined hereinbefore,
which, if desired, is purified by a conventional method, such as chromatography and/or crystallisation, and/or converted into a salt with a pharmaceutically acceptable acid.

**29.** Process according to claim 25 for the preparation of compounds of formula (I/C) :

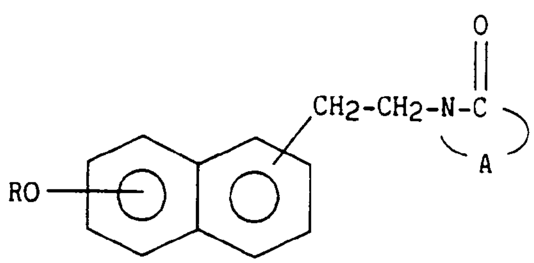

(I/C)

wherein R is as defined for formula (I) and A represents a straight-chain or branched alkyl group comprising from 2 to 8 carbon atoms,
characterised in that a compound of formula (II) :

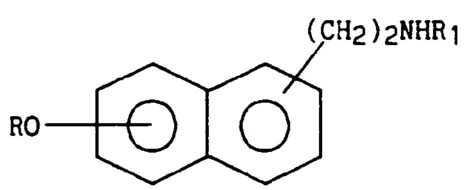

(II)

wherein R is as defined for formula (I), is treated

- with an acid chloride of formula (IV) :

$$Cl\text{-}CO\text{-}R'_2 \qquad (IV)$$

- or with the corresponding acid anhydride, wherein $R'_2$ represents a straight-chain or branched lower alkyl group that is substituted by a halogen atom,

to yield a compound of formula (I/B) :

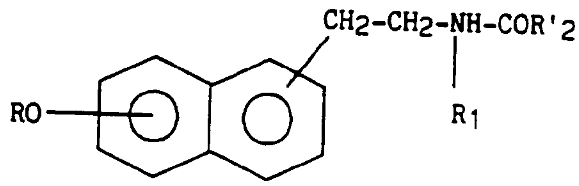

(I/B)

wherein R and $R'_2$ are as defined hereinbefore,
which, if necessary, is purified by conventional methods, such as chromatography and/or crystallisation, which is subjected to the action of a strong base and preferably an alkali metal alcoholate to yield a compound of formula (I/C),
which, if desired, is purified by a method selected from crystallisation and chromatography.

**30.** Pharmaceutical composition comprising as active ingredient at least one compound according to any one of claims 1 to 24, in combination with one or more pharmaceutically acceptable, inert, non-toxic excipients or carriers.

**31.** Pharmaceutical composition according to claim 30 useful in the treatment of disorders of the melatoninergic system and especially stress, anxiety, seasonal depression, insomnia and fatigue due to shiftwork, schizophrenias, panic attack, melancholy, regulation of the appetite, insomnia, psychotic disorders, epilepsy, Parkinson's disease, senile dementia, various disorders associated with normal or pathological ageing, loss of memory, Alzheimer's disease, and also cerebral circulation disorders, certain cancers, psoriasis, acne, seborrhoea, and also as an ovulation inhibitor or in veterinary medicine in fur disorders.

**Claims for the following Contracting State : ES**

**1.** Process for the preparation of compounds of the general formula (I) :

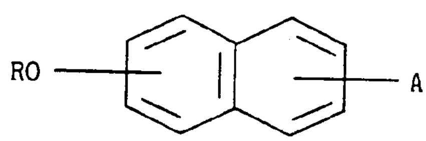

(I)

wherein :

A represents a group

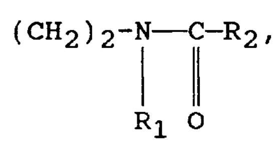

R represents a straight-chain or branched lower alkyl group,
$R_1$ represents a hydrogen atom or a straight-chain or branched lower alkyl group,
$R_2$ represents

. a hydrogen atom,
. a straight-chain or branched lower alkyl group or a cycloalkyl group, each of which is optionally substituted by a halogen atom,
. an aryl or heteroaryl or aryl-lower alkyl group or substituted aryl or substituted heteroaryl or substituted arylalkyl group, there being understood by heteroaryl group an unsaturated mono- or bi-cyclic group comprising from 1 to 3 hetero atoms selected from nitrogen, oxygen and sulphur, each ring comprising 4 or 5 apices, and there being understood by aryl group phenyl or naphthyl,

. an imidazolyl group that is optionally reduced and/or substituted by an oxo group,

. a group of formula :

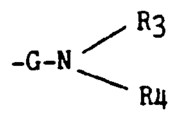

G representing a straight-chain or branched lower alkyl group, and $R_3$ and $R_4$, which are the same or different, each representing a lower alkyl group or a hydrogen atom or a phenyl or phenyl-lower alkyl group, or $R_3$ and $R_4$, together with the nitrogen atom to which they are attached, forming an aromatic or non-aromatic, mono- or bi-cyclic, heterocyclic system, each ring having five or six apices optionally comprising an additional hetero atom and optionally being substituted by one or more lower alkyl or oxo, aryl or aryl-lower alkyl, or substituted aryl or substituted aryl-lower alkyl groups, the term "substituted" used in relation to aryl and arylalkyl or heteroaryl groups in the definition of $R_2$, $R_3$, $R_4$ indicating that those groups are substituted by one or more radicals selected from lower alkyl, lower alkoxy, trifluoromethyl and halogen,

or $R_1$ forms, with $R_2$ and the group N-CO, a heterocyclic system of the formula :

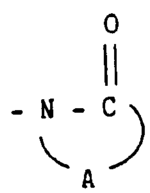

A representing a straight-chain or branched alkyl radical having from 2 to 8 carbon atoms, and also, where appropriate, their isomers, epimers and diastereoisomers and their addition salts with a pharmaceutically acceptable acid, the terms "lower alkyl" and "lower alkoxy" indicating groups comprising from 1 to 6 carbon atoms and "cycloalkyl" indicating groups comprising from 3 to 8 carbon atoms, characterised in that there is used as starting material a compound of formula (II) :

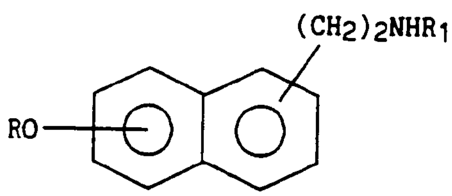

(II)

wherein R and $R_1$ are as defined for formula (I), which is treated :

- either with a compound of formula (III) :

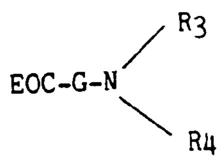

(III)

wherein E denotes a leaving group selected from hydroxy, lower alkoxy and halogen, and G, $R_3$ and $R_4$ are as defined for formula (I), optionally in the presence of an alkaline agent, to yield a compound of formula (I/A), a particular case of compounds of formula (I) :

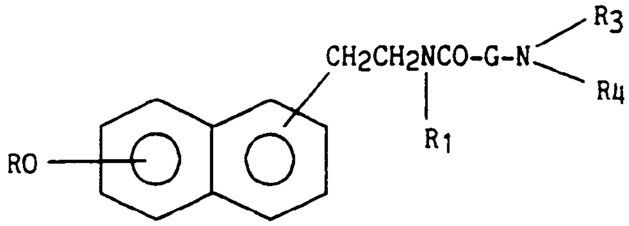

(I/A)

wherein R, $R_1$, $R_3$, $R_4$ and G are as defined hereinbefore, $R_2$ in this case indicating a group :

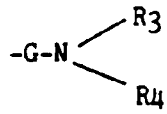

which is purified, if desired, by conventional methods, such as chromatography and crystallisation, and which if desired is converted into a salt with a pharmaceutically acceptable acid,

- or with an acid chloride of formula (IV) :

$$Cl\text{-}CO\text{-}R'_2 \qquad (IV)$$

or with the corresponding acid anhydride,

$R'_2$ representing :

- a straight-chain or branched lower alkyl group or a cycloalkyl group, each of which is optionally substituted by a halogen atom,
- an aryl or heteroaryl or aryl-lower alkyl group, each of which is optionally substituted by one or more halogen atoms or groups selected from lower alkyl, lower alkoxy and trifluoromethyl,
- an imidazolyl group that is optionally reduced and/or substituted by an oxo group,

to yield a compound of formula (I/B) :

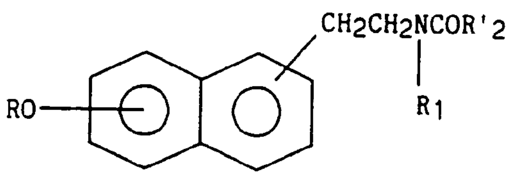

(I/B)

wherein R, $R_1$ and $R'_2$ are as defined hereinbefore, which, if necessary, is purified by conventional methods, such as chromatography and/or crystallisation,

which compound of formula (I/B), when $R'_2$ represents a straight-chain or branched lower alkyl group that is substituted by a halogen atom, may, if desired, be subjected to the action of an amine of formula (V) :

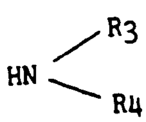

(V)

wherein $R_3$ and $R_4$ are as defined hereinbefore,

in excess or in the presence of a tertiary amine or an alkali metal salt, to yield a compound of formula (I/A) as defined hereinbefore,

which, if desired, is purified by a conventional method, such as chromatography and/or crystallisation, and/or converted into a salt with a pharmaceutically acceptable acid,

which compound of formula (I/B), when $R'_2$ represents a straight-chain or branched alkyl substituent that comprises at least two carbon atoms and is substituted by a halogen atom and when, simultaneously, $R_1$ represents a hydrogen atom, may, if desired, be subjected to the action of a strong base and preferably an alkali metal alcoholate to yield a compound of formula (I/C) :

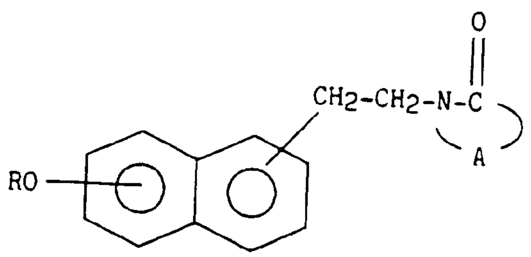

(I/C)

wherein R is as defined hereinbefore and A represents a straight-chain or branched alkyl group comprising from 2 to 8 carbon atoms, a particular case of compounds of formula (I) in which $R_1$ and $R_2$ form with NCO a monocyclic system that is substituted by an oxo group and is optionally substituted by one or more lower alkyl groups, which, if desired, is purified by a method selected from crystallisation and chromatography.

2. Process according to claim 1 for the preparation of compounds of formula (I/A) :

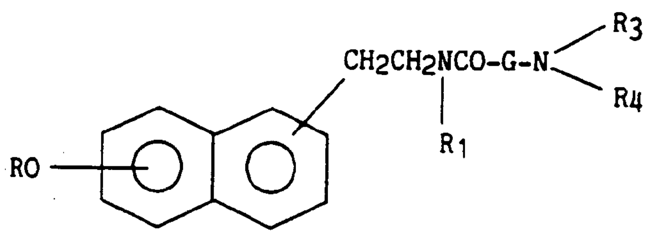

(I/A)

wherein R, $R_1$, $R_3$, $R_4$ and G are as defined for formula (I), characterised in that a compound of formula (II) :

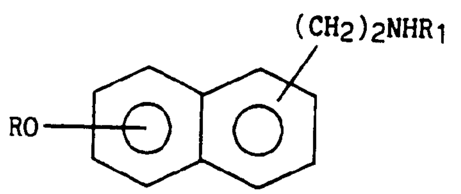

(II)

wherein R and $R_1$ are as defined for formula (I) is treated with a compound of formula (III) :

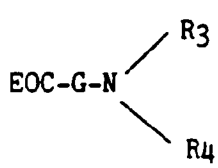

(III)

wherein E represents a leaving group selected from hydroxy, lower alkoxy and halogen, and G, $R_3$ and $R_4$ are as defined for formula (I),
optionally in the presence of an alkaline agent, to yield a compound of formula (I/A) which, if desired, is purified by conventional methods, such as chromatography and crystallisation, of which, where appropriate, the isomers are separated, and which, if desired, is converted into a salt with a pharmaceutically acceptable acid.

3. Process according to claim 1 for the preparation of compounds of formula (I/B) :

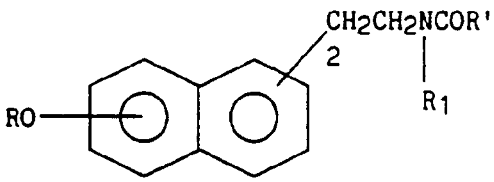

(I/B)

wherein R and $R_1$ are as defined for formula (I), and $R_2$ represents :

- a straight-chain or branched lower alkyl group or a cycloalkyl group, each of which is optionally substituted by a halogen atom,
- an aryl or heteroaryl or aryl-lower alkyl group, each of which is optionally substituted by one or more halogen atoms or groups selected from lower alkyl, lower alkoxy and trifluoromethyl,

characterised in that a compound of formula (II) :

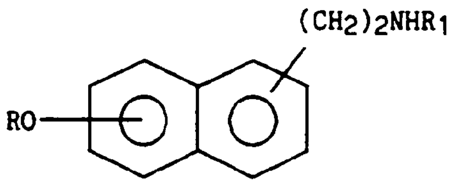

(II)

wherein R and $R_1$ are as defined for formula (I) is treated

- with an acid chloride of formula (IV) :

  $Cl\text{-}CO\text{-}R'_2$ (IV)

  wherein $R'_2$ is as defined above,
- or with the corresponding acid anhydride to yield a compound of formula (I/B) which, if necessary, is purified by conventional methods, such as chromatography and/or crystallisation, and of which, where appropriate, the isomers are separated.

4. Process according to claim 1 for the preparation of compounds of formula (I/A) :

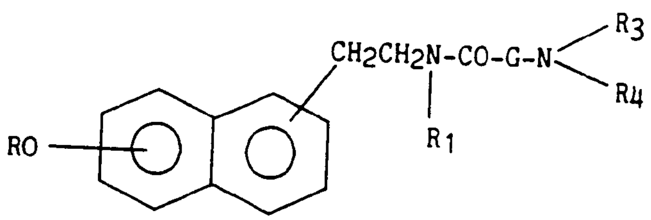

(I/A)

wherein R, $R_1$, $R_3$ and $R_4$ are as defined for formula (I), characterised in that a compound of formula (II) :

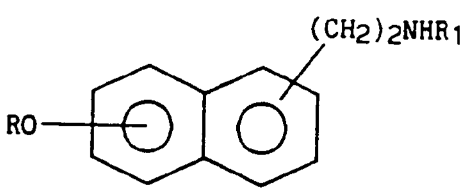

(II)

wherein R and $R_1$ are as defined for formula (I) is treated

- with an acid chloride of formula (IV) :

  $Cl\text{-}CO\text{-}R'_2$ (IV)
- or with the corresponding acid anhydride, wherein $R'_2$ represents a straight-chain or branched lower alkyl group that is substituted by a halogen atom,

to yield a compound of formula (I/B) :

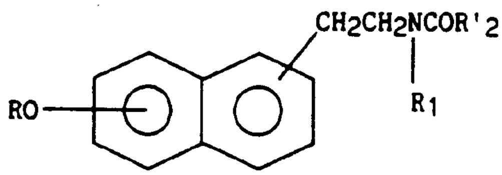

(I/B)

wherein R, $R_1$ and $R'_2$ are as defined hereinbefore, which, if necessary, is purified by conventional methods, such as chromatography and/or crystallisation, which is subjected to the action of an amine of formula (V) :

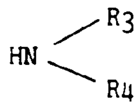

(V)

wherein $R_3$ and $R_4$ are as defined hereinbefore,
in excess or in the presence of a tertiary amine or an alkali metal salt to yield a compound of formula (I/A) as defined hereinbefore,
which, if desired, is purified by a conventional method, such as chromatography and/or crystallisation, and/or converted into a salt with a pharmaceutically acceptable acid.

**5.** Process according to claim 1 for the preparation of compounds of formula (I/C) :

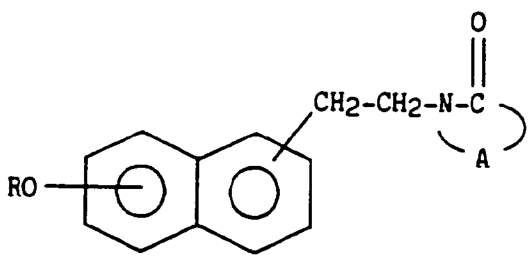

(I/C)

wherein R is as defined for formula (I) and A represents a straight-chain or branched alkyl group comprising from 2 to 8 carbon atoms,
characterised in that a compound of formula (II) :

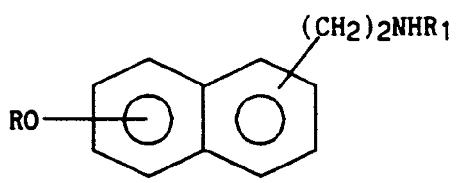

(II)

wherein R is as defined for formula (I), is treated
- with an acid chloride of formula (IV) :

$$Cl\text{-}CO\text{-}R'_2 \qquad (IV)$$

- or with the corresponding acid anhydride, wherein $R'_2$ represents a straight-chain or branched lower alkyl group that is substituted by a halogen atom,

to yield a compound of formula (I/B) :

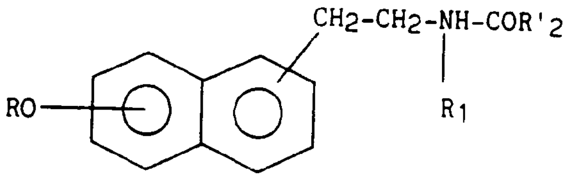

(I/B)

wherein R and $R'_2$ are as defined hereinbefore,
which, if necessary, is purified by conventional methods, such as chromatography and/or crystallisation,
which is subjected to the action of a strong base and preferably an alkali metal alcoholate to yield a compound of formula (I/C),
which, if desired, is purified by a method selected from crystallisation and chromatography.

6. Process according to any one of claims 1 to 5 for the preparation of compounds of formula (I) wherein the OR group is in the 7-position, and also, where appropriate, their isomers, epimers and diastereoisomers and their addition salts with a pharmaceutically acceptable acid.

7. Process according to any one of claims 1 to 5 for the preparation of compounds of formula (I) wherein $R_1$ represents a hydrogen atom or forms, with $R_2$ and the group N-CO, the 2-pyrrolidinone system, and also, where appropriate, their isomers, epimers and diastereoisomers and their addition salts with a pharmaceutically acceptable acid.

8. Process according to claim 1 for the preparation of compounds of formula (I) wherein $R_2$ represents:
   . a straight-chain or branched lower alkyl group or a cycloalkyl group, each of which is optionally substituted by a halogen atom,
   . a mono- or bi-cyclic aryl or heteroaryl group or an aryl-lower alkyl group, each of which is optionally substituted by one or more halogen atoms or groups selected from lower alkyl, lower alkoxy and trifluoromethyl,
   . a group of formula :

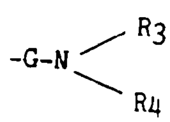

G representing a straight-chain or branched lower alkyl group, and
$R_3$ and $R_4$, together with the nitrogen atom to which they are attached, forming a mono- or bi-cyclic, heterocyclic system, each ring having five or six apices optionally comprising an additional hetero atom and optionally being substituted by a lower alkyl or oxo, aryl or aryl-lower alkyl, or substituted aryl or substituted aryl-lower alkyl group, the term "substituted" used in relation to aryl and arylalkyl groups indicating that those groups may be substituted by one or more radicals selected from lower alkyl, lower alkoxy, trifluoromethyl and halogen, and also, where appropriate, their isomers, epimers and diastereoisomers and their addition salts with a pharmaceutically acceptable acid.

9. Process according to any one of claims 1 to 6 for the preparation of compounds of formula (I) wherein the group OR is in the 7-position, $R_1$ represents a hydrogen atom and $R_2$ represents a straight-chain or branched lower alkyl group or a cycloalkyl group, each of which is optionally substituted by a halogen atom, and also, where appropriate, their isomers, epimers and diastereoisomers.

10. Process according to claim 1 for the preparation of compounds of formula (I) wherein the group OR is in the 7-position, $R_1$ represents a hydrogen atom and $R_2$ represents a mono- or bi-cyclic aryl or heteroaryl group optionally substituted by one or more halogen atoms, and also, where appropriate, their isomers, epimers and diastereoisomers.

11. Process according to claim 1 for the preparation of the compound of formula (I) which is N-[2-(7-methoxynaphth-1-yl)ethyl]cyclopropylcarboxamide.

12. Process according to claim 1 for the preparation of the compound of formula (I) which is N-[2-(7-methoxynaphth-1-yl)ethyl](2-oxopyrrolidin-1-yl)acetamide.

**13.** Process according to claim 1 for the preparation of the compound of formula (I) which is N-[2-(7-methoxy-naphth-1-yl)ethyl]pyrrolidin-2-one.

**14.** Process according to claim 1 for the preparation of the compound of formula (I) which is N-[2-(7-methoxy-naphth-1-yl)ethyl]acetamide.

**15.** Process according to claim 1 for the preparation of the compound of formula (I) which is N-[2-(7-methoxy-naphth-1-yl)ethyl]isobutyramide.

**16.** Process according to claim 1 for the preparation of the compound of formula (I) which is N-[2-(7-methoxy-naphth-1-yl)ethyl]butyramide.

**17.** Process according to claim 1 for the preparation of the compound of formula (I) which is N-[2-(7-methoxy-naphth-1-yl)ethyl]propionamide.

**18.** Process according to claim 1 for the preparation of the compound of formula (I) which is N-[2-(7-methoxy-naphth-1-yl)ethyl]-4-chlorobutyramide.

**19.** Process according to claim 1 for the preparation of the compound of formula (I) which is N-[2-(7-methoxy-naphth-1-yl)ethyl]-2-morpholinoacetamide.

**20.** Process according to claim 1 for the preparation of the compound of formula (I) which is N-[2-(7-methoxy-naphth-1-yl)ethyl]-2-{4-[(2,3,4-trimethoxyphenyl)-methyl]piperazin-1-yl}acetamide, and also the addition salts thereof with a pharmaceutically acceptable acid.

**21.** Process according to claim 1 for the preparation of the compound of formula (I) which is N-[2-(7-methoxy-naphth-1-yl)ethyl]benzamide.

**22.** Process according to claim 1 for the preparation of the compound of formula (I) which is N-[2-(7-methoxy-naphth-1-yl)ethyl]-3,5-dichlorobenzamide.

**23.** Process according to claim 1 for the preparation of the compound of formula (I) which is N-[2-(7-methoxy-naphth-1-yl)ethyl]-(indol-2-yl)carboxamide.

**24.** Process according to claim 1 for the preparation of the compound of formula (I) which is N-[2-(7-methoxy-naphth-1-yl)ethyl]pentanamide.

**25.** Process according to claim 1 for the preparation of the compound of formula (I) which is N-[2-(7-methoxy-naphth-1-yl)ethyl]cyclobutane carboxamide.

**26.** Process according to claim 1 for the preparation of the compound of formula (I) which is N-[2-(7-methoxy-naphth-1-yl)ethyl]-2-iodoacetamide.

**27.** Process according to claim 1 for the preparation of the compound of formula (I) which is N-[2-(7-methoxy-naphth-1-yl)ethyl]-2-bromoacetamide.

**28.** Process according to claim 1 for the preparation of the compound of formula (I) which is N-[2-(7-methoxy-naphth-1-yl)ethyl]-2-chloroacetamide.

**29.** Process for the preparation of pharmaceutical compositions comprising as active ingredient at least one compound according to any one of claims 11 to 28, in combination with one or more pharmaceutically acceptable, inert, non-toxic excipients or carriers.

**30.** Process for the preparation of a pharmaceutical composition according to claim 29 useful in the treatment of disorders of the melatoninergic system and especially stress, anxiety, seasonal depression, insomnia and fatigue due to shiftwork, schizophrenias, panic attack, melancholy, regulation of the appetite, insomnia, psychotic disorders, epilepsy, Parkinson's disease, senile dementia, various disorders associated with normal or pathological ageing, loss of memory, Alzheimer's disease, and also cerebral circulation disorders, certain cancers, psoriasis, acne, seborrhoea, and also as an ovulation inhibitor or in veterinary medicine in fur disorders.

**Claims for the following Contracting State : GR**

1. Process for the preparation of compounds of the general formula (I) :

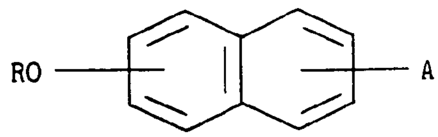

(I)

wherein :

A represents a group

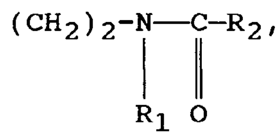

R represents a straight-chain or branched lower alkyl group,

$R_1$ represents a hydrogen atom or a straight-chain or branched lower alkyl group,

$R_2$ represents

. a hydrogen atom,

. a straight-chain or branched lower alkyl group or a cycloalkyl group, each of which is optionally substituted by a halogen atom,

. an aryl or heteroaryl or aryl-lower alkyl group or substituted aryl or substituted heteroaryl or substituted arylalkyl group, there being understood by heteroaryl group an unsaturated mono- or bi-cyclic group comprising from 1 to 3 hetero atoms selected from nitrogen, oxygen and sulphur, each ring comprising 4 or 5 apices, and there being understood by aryl group phenyl or naphthyl,

. an imidazolyl group that is optionally reduced and/or substituted by an oxo group,

. a group of formula :

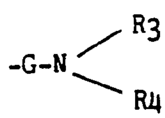

G representing a straight-chain or branched lower alkyl group, and

$R_3$ and $R_4$, which are the same or different, each representing a lower alkyl group or a hydrogen atom or a phenyl or phenyl-lower alkyl group, or $R_3$ and $R_4$, together with the nitrogen atom to which they are attached, forming an aromatic or non-aromatic, mono- or bi-cyclic, heterocyclic system, each ring having five or six apices optionally comprising an additional hetero atom and optionally being substituted by one or more lower alkyl or oxo, aryl or aryl-lower alkyl, or substituted aryl or substituted aryl-lower alkyl groups, the term "substituted" used in relation to aryl and arylalkyl or heteroaryl groups in the definition of $R_2$, $R_3$, $R_4$ indicating that those groups are substituted by one or more radicals selected from lower alkyl, lower alkoxy, trifluoromethyl and halogen,

or $R_1$ forms, with $R_2$ and the group N-CO, a heterocyclic system of the formula :

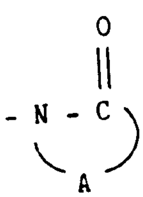

A representing a straight-chain or branched alkyl radical having from 2 to 8 carbon atoms,

and also, where appropriate, their isomers, epimers and diastereoisomers and their addition salts with a

pharmaceutically acceptable acid,
the terms "lower alkyl" and "lower alkoxy" indicating groups comprising from 1 to 6 carbon atoms and "cycloalkyl" indicating groups comprising from 3 to 8 carbon atoms, characterised in that there is used as starting material a compound of formula (II) :

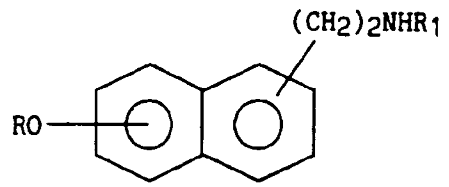

(II)

wherein R and $R_1$ are as defined for formula (I), which is treated :

- either with a compound of formula (III) :

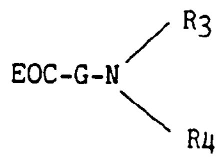

(III)

wherein E denotes a leaving group selected from hydroxy, lower alkoxy and halogen, and G, $R_3$ and $R_4$ are as defined for formula (I),
optionally in the presence of an alkaline agent, to yield a compound of formula (I/A), a particular case of compounds of formula (I) :

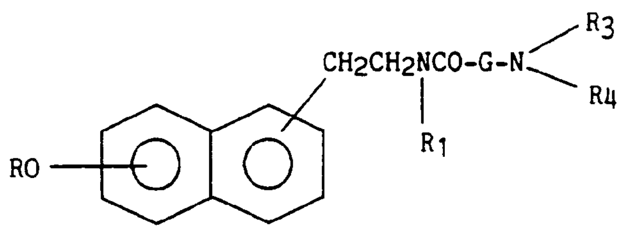

(I/A)

wherein R, $R_1$, $R_3$, $R_4$ and G are as defined hereinbefore, $R_2$ in this case indicating a group :

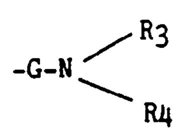

which is purified, if desired, by conventional methods, such as chromatography and crystallisation, and which if desired is converted into a salt with a pharmaceutically acceptable acid,

- or with an acid chloride of formula (IV) :

$$Cl\text{-}CO\text{-}R'_2 \qquad (IV)$$

or with the corresponding acid anhydride,
$R'_2$ representing :

- a straight-chain or branched lower alkyl group or a cycloalkyl group, each of which is optionally substituted by a halogen atom,
- an aryl or heteroaryl or aryl-lower alkyl group, each of which is optionally substituted by one or more halogen atoms or groups selected from lower alkyl, lower alkoxy and trifluoromethyl,
- an imidazolyl group that is optionally reduced and/or substituted by an oxo group,

to yield a compound of formula (I/B) :

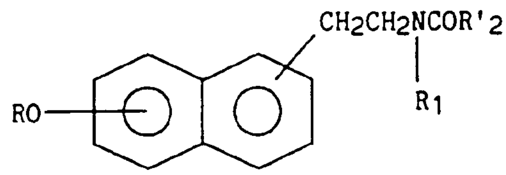

(I/B)

wherein R, $R_1$ and $R'_2$ are as defined hereinbefore, which, if necessary, is purified by conventional methods, such as chromatography and/or crystallisation,
which compound of formula (I/B), when $R'_2$ represents a straight-chain or branched lower alkyl group that is substituted by a halogen atom, may, if desired, be subjected to the action of an amine of formula (V) :

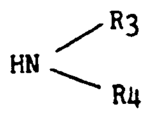

(V)

wherein $R_3$ and $R_4$ are as defined hereinbefore,
in excess or in the presence of a tertiary amine or an alkali metal salt, to yield a compound of formula (I/A) as defined hereinbefore,
which, if desired, is purified by a conventional method, such as chromatography and/or crystallisation, and/or converted into a salt with a pharmaceutically acceptable acid,
which compound of formula (I/B), when $R'_2$ represents a straight-chain or branched alkyl substituent that comprises at least two carbon atoms and is substituted by a halogen atom and when, simultaneously, $R_1$ represents a hydrogen atom, may, if desired, be subjected to the action of a strong base and preferably an alkali metal alcoholate to yield a compound of formula (I/C) :

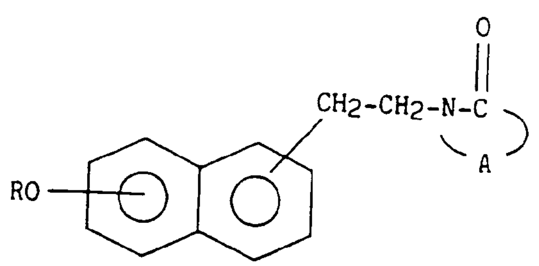

(I/C)

wherein R is as defined hereinbefore and A represents a straight-chain or branched alkyl group comprising from 2 to 8 carbon atoms, a particular case of compounds of formula (I) in which $R_1$ and $R_2$ form with NCO a monocyclic system that is substituted by an oxo group and is optionally substituted by one or more lower alkyl groups, which, if desired, is purified by a method selected from crystallisation and chromatography.

2. Process according to claim 1 for the preparation of compounds of formula (I/A) :

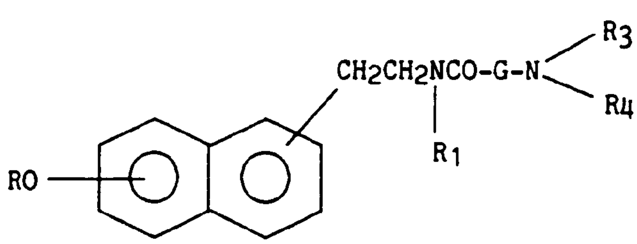

(I/A)

wherein R, $R_1$, $R_3$, $R_4$ and G are as defined for formula (I), characterised in that a compound of formula (II) :

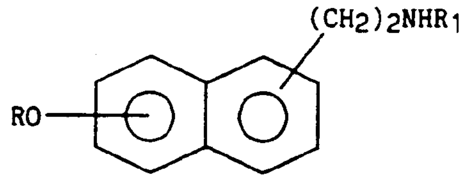

(II)

wherein R and $R_1$ are as defined for formula (I) is treated with a compound of formula (III) :

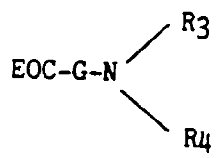

(III)

wherein E represents a leaving group selected from hydroxy, lower alkoxy and halogen, and G, $R_3$ and $R_4$ are as defined for formula (I),
optionally in the presence of an alkaline agent, to yield a compound of formula (I/A) which, if desired, is purified by conventional methods, such as chromatography and crystallisation, of which, where appropriate, the isomers are separated, and which, if desired, is converted into a salt with a pharmaceutically acceptable acid.

**3.** Process according to claim 1 for the preparation of compounds of formula (I/B) :

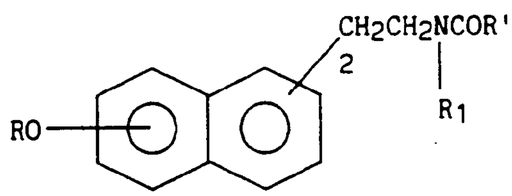

(I/B)

wherein R and $R_1$ are as defined for formula (I), and $R_2$ represents :

- a straight-chain or branched lower alkyl group or a cycloalkyl group, each of which is optionally substituted by a halogen atom,
- an aryl or heteroaryl or aryl-lower alkyl group, each of which is optionally substituted by one or more halogen atoms or groups selected from lower alkyl, lower alkoxy and trifluoromethyl,
  characterised in that a compound of formula (II) :

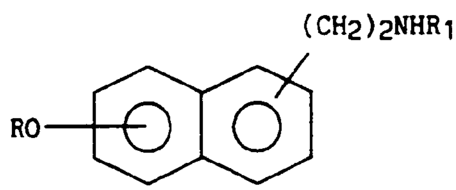

(II)

wherein R and $R_1$ are as defined for formula (I) is treated

- with an acid chloride of formula (IV) :

  $$Cl\text{-}CO\text{-}R'_2 \qquad (IV)$$

  wherein $R'_2$ is as defined above,
- or with the corresponding acid anhydride to yield a compound of formula (I/B) which, if necessary, is purified by conventional methods, such as chromatography and/or crystallisation, and of which, where appropriate, the isomers are separated.

**4.** Process according to claim 1 for the preparation of compounds of formula (I/A) :

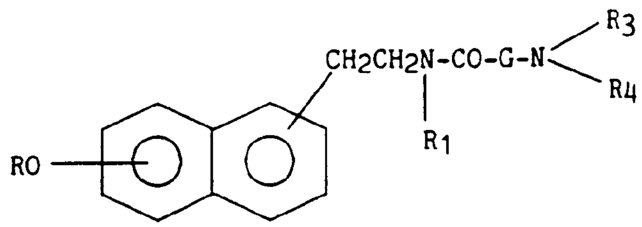

(I/A)

wherein R, $R_1$, $R_3$ and $R_4$ are as defined for formula (I), characterised in that a compound of formula (II) :

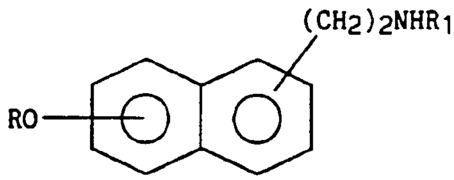

(II)

wherein R and $R_1$ are as defined for formula (I) is treated

- with an acid chloride of formula (IV) :

  $Cl\text{-}CO\text{-}R'_2$ (IV)

- or with the corresponding acid anhydride, wherein $R'_2$ represents a straight-chain or branched lower alkyl group that is substituted by a halogen atom,

to yield a compound of formula (I/B) :

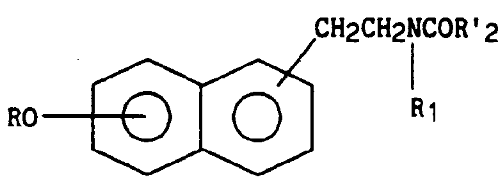

(I/B)

wherein R, $R_1$ and $R'_2$ are as defined hereinbefore, which, if necessary, is purified by conventional methods, such as chromatography and/or crystallisation,
which is subjected to the action of an amine of formula (V) :

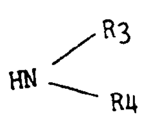

(V)

wherein $R_3$ and $R_4$ are as defined hereinbefore,
in excess or in the presence of a tertiary amine or an alkali metal salt to yield a compound of formula (I/A) as defined hereinbefore,
which, if desired, is purified by a conventional method, such as chromatography and/or crystallisation, and/or converted into a salt with a pharmaceutically acceptable acid.

5. Process according to claim 1 for the preparation of compounds of formula (I/C) :

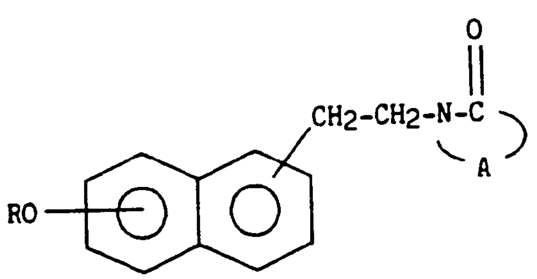

(I/C)

wherein R is as defined for formula (I) and A represents a straight-chain or branched alkyl group comprising from 2 to 8 carbon atoms,
characterised in that a compound of formula (II) :

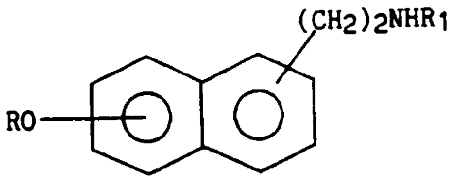

(II)

wherein R is as defined for formula (I), is treated
- with an acid chloride of formula (IV) :

$Cl\text{-}CO\text{-}R'_2$ (IV)

- or with the corresponding acid anhydride, wherein $R'_2$ represents a straight-chain or branched lower alkyl group that is substituted by a halogen atom,

to yield a compound of formula (I/B) :

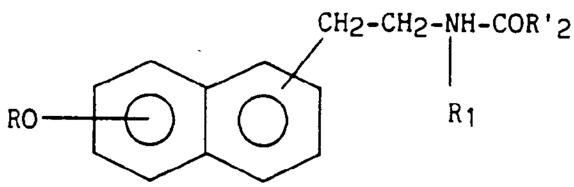

(I/B)

wherein R and $R'_2$ are as defined hereinbefore,
which, if necessary, is purified by conventional methods, such as chromatography and/or crystallisation, which is subjected to the action of a strong base and preferably an alkali metal alcoholate to yield a compound of formula (I/C),
which, if desired, is purified by a method selected from crystallisation and chromatography.

6. Process according to any one of claims 1 to 5 for the preparation of compounds of formula (I) wherein the OR group is in the 7-position, and also, where appropriate, their isomers, epimers and diastereoisomers and their addition salts with a pharmaceutically acceptable acid.

7. Process according to any one of claims 1 to 5 for the preparation of compounds of formula (I) wherein $R_1$ represents a hydrogen atom or forms, with $R_2$ and the group N-CO, the 2-pyrrolidinone system, and also, where appropriate, their isomers, epimers and diastereoisomers and their addition salts with a pharmaceutically acceptable acid.

8. Process according to claim 1 for the preparation of compounds of formula (I) wherein $R_2$ represents:
   . a straight-chain or branched lower alkyl group or a cycloalkyl group, each of which is optionally substituted by a halogen atom,
   . a mono- or bi-cyclic aryl or heteroaryl group or an aryl-lower alkyl group, each of which is optionally substituted by one or more halogen atoms or groups selected from lower alkyl, lower alkoxy and trifluoromethyl,
   . a group of formula :

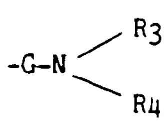

G representing a straight-chain or branched lower alkyl group, and
$R_3$ and $R_4$, together with the nitrogen atom to which they are attached, forming a mono- or bi-cyclic, heterocyclic system, each ring having five or six apices optionally comprising an additional hetero atom and optionally being substituted by a lower alkyl or oxo, aryl or aryl-lower alkyl, or substituted aryl or substituted aryl-lower alkyl group, the term "substituted" used in relation to aryl and arylalkyl groups indicating that those groups may be substituted by one or more radicals selected from lower alkyl, lower alkoxy, trifluoromethyl and halogen, and also, where appropriate, their isomers, epimers and diastereoisomers and their addition salts with a pharmaceutically acceptable acid.

**9.** Process according to any one of claims 1 to 6 for the preparation of compounds of formula (I) wherein the group OR is in the 7-position, $R_1$ represents a hydrogen atom and $R_2$ represents a straight-chain or branched lower alkyl group or a cycloalkyl group, each of which is optionally substituted by a halogen atom, and also, where appropriate, their isomers, epimers and diastereoisomers.

**10.** Process according to claim 1 for the preparation of compounds of formula (I) wherein the group OR is in the 7-position, $R_1$ represents a hydrogen atom and $R_2$ represents a mono- or bi-cyclic aryl or heteroaryl group optionally substituted by one or more halogen atoms, and also, where appropriate, their isomers, epimers and diastereoisomers.

**11.** Process according to claim 1 for the preparation of the compound of formula (I) which is N-[2-(7-methoxynaphth-1-yl)ethyl]cyclopropylcarboxamide.

**12.** Process according to claim 1 for the preparation of the compound of formula (I) which is N-[2-(7-methoxynaphth-1-yl)ethyl](2-oxopyrrolidin-1-yl)acetamide.

**13.** Process according to claim 1 for the preparation of the compound of formula (I) which is N-[2-(7-methoxynaphth-1-yl)ethyl]pyrrolidin-2-one.

**14.** Process according to claim 1 for the preparation of the compound of formula (I) which is N-[2-(7-methoxynaphth-1-yl)ethyl]acetamide.

**15.** Process according to claim 1 for the preparation of the compound of formula (I) which is N-[2-(7-methoxynaphth-1-yl)ethyl]isobutyramide.

**16.** Process according to claim 1 for the preparation of the compound of formula (I) which is N-[2-(7-methoxynaphth-1-yl)ethyl]butyramide.

**17.** Process according to claim 1 for the preparation of the compound of formula (I) which is N-[2-(7-methoxynaphth-1-yl)ethyl]propionamide.

**18.** Process according to claim 1 for the preparation of the compound of formula (I) which is N-[2-(7-methoxynaphth-1-yl)ethyl]-4-chlorobutyramide.

**19.** Process according to claim 1 for the preparation of the compound of formula (I) which is N-[2-(7-methoxynaphth-1-yl)ethyl]-2-morpholinoacetamide.

**20.** Process according to claim 1 for the preparation of the compound of formula (I) which is N-[2-(7-methoxynaphth-1-yl)ethyl]-2-{4-[(2,3,4-trimethoxyphenyl)-methyl]piperazin-1-yl}acetamide, and also the addition salts thereof with a pharmaceutically acceptable acid.

**21.** Process according to claim 1 for the preparation of the compound of formula (I) which is N-[2-(7-methoxynaphth-1-yl)ethyl]benzamide.

**22.** Process according to claim 1 for the preparation of the compound of formula (I) which is N-[2-(7-methoxynaphth-1-yl)ethyl]-3,5-dichlorobenzamide.

**23.** Process according to claim 1 for the preparation of the compound of formula (I) which is N-[2-(7-methoxynaphth-1-yl)ethyl]-(indol-2-yl)carboxamide.

**24.** Process according to claim 1 for the preparation of the compound of formula (I) which is N-[2-(7-methoxynaphth-1-yl)ethyl]pentanamide.

**25.** Process according to claim 1 for the preparation of the compound of formula (I) which is N-[2-(7-methoxynaphth-1-yl)ethyl]cyclobutane carboxamide.

**26.** Process according to claim 1 for the preparation of the compound of formula (I) which is N-[2-(7-methoxy-naphth-1-yl)ethyl]-2-iodoacetamide.

**27.** Process according to claim 1 for the preparation of the compound of formula (I) which is N-[2-(7-methoxy-naphth-1-yl)ethyl]-2-bromoacetamide.

**28.** Process according to claim 1 for the preparation of the compound of formula (I) which is N-[2-(7-methoxy-naphth-1-yl)ethyl]-2-chloroacetamide.

**29.** Process for the preparation of pharmaceutical compositions comprising as active ingredient at least one compound according to any one of claims 11 to 28, in combination with one or more pharmaceutically acceptable, inert, non-toxic excipients or carriers.

**30.** Process for the preparation of a pharmaceutical composition according to claim 29 useful in the treatment of disorders of the melatoninergic system and especially stress, anxiety, seasonal depression, insomnia and fatigue due to shiftwork, schizophrenias, panic attack, melancholy, regulation of the appetite, insomnia, psychotic disorders, epilepsy, Parkinson's disease, senile dementia, various disorders associated with normal or pathological ageing, loss of memory, Alzheimer's disease, and also cerebral circulation disorders, certain cancers, psoriasis, acne, seborrhoea, and also as an ovulation inhibitor or in veterinary medicine in fur disorders.